# EUROPEAN PATENT APPLICATION

(11) **EP 1 661 879 A1**
(43) Date of publication of application: **31.05.2006**
(21) Application number: 04771411.8
(22) Date of filing: 03.08.2004
(51) Int. Cl.: C07C 59/68, C07C 62/34, C07C 65/24, C07C 65/28, C07C 217/90, C07C 233/29, C07D 213/30, C07D 263/32, C07D 277/20, C07D 317/46

(54) **DIPHENYL ETHER COMPOUND, PROCESS FOR PRODUCING THE SAME, AND USE**

(30) Priority: 04.08.2003 JP 2003286199
(71) Applicant: ONO PHARMACEUTICAL CO., LTD., Osaka-shi, Osaka 541-8526 (JP)
(72) Inventor: KUSUDA, Shinya c/o Ono Pharmaceutical Co., Ltd., Mishima-gun, Osaka 618-8585 (JP); NAKAYAMA, Yoshisuke c/o Ono Pharmaceutical Co.Ltd, 1-1, Sakurai 3-chome, Shimamoto-cho (JP); IMA, Masaki c/o Ono Pharmaceutical Co., Ltd., Mishima-gun, Osaka 618-8585 (JP); TAJIMA, Hisao c/o Ono Pharmaceutical Co., Ltd., Mishima-gun, Osaka 618-8585 (JP); KATO, Sachiko c/o Ono Pharmaceutical Co., Ltd., Mishima-gun, Osaka 618-8585 (JP)
(74) Representative: Henkel, Feiler & Hänzel
(86) International application number: PCT/JP2004/011424
(87) International publication number: WO 2005/012221

(57) **Abstract**

The present invention relates to a compound represented by formula (I): 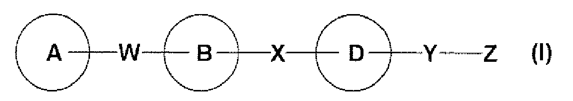 wherein ring A, ring B and ring D each independently represents a cyclic group which may have a substituent(s); W is a spacer having 1 to 8 atom(s) in its main chain; X is a spacer having 1 to 2 atom(s) in its main chain; Y is a binding bond or a spacer having 1 to 8 atom(s) in its main chain; and Z is an acidic group, or a salt thereof, a solvate thereof or a prodrug thereof. The compound represented by formula (I) has a PPAR δ agonistic action and is useful as a preventive and/or treating agent for diseases where sugar and lipid metabolisms are abnormal (diabetes, hyperlipemia, arteriosclerosis, cardiovascular diseases, obesity and metabolic syndrome or the like), hypertension, circulatory diseases and skin inflammation diseases.

SEQUENCE LISTING

<110> ONO Pharmaceutical Co.,Ltd.

<120> Diphenyl ether compounds, production method and use thereof

<130> ONF-5116PCT

<150> JP 2003-286199
<151> 2003-08-04

<160> 3
<170> PatentIn Ver. 3.1

<210> 1
<211> 85
<212> DNA
<213> Artificial sequence
<220>
<223> Enhancer sequence including 4 times repeated Gal4 protein response
sequences
<400> 1
tcgacggagt actgtcctcc gcgacggagt actgtcctcc gcgacggagt actgtcctcc 60
gcgacggagt actgtcctcc gagct 85

<210> 2

<211> 9

<212> PRT

<213> Unknown

<220>

<223> Nuclear localozation signal derived from SV-40 T-antigen

<400> 2

Ala Pro Lys Lys Lys Arg Lys Val Gly

1               5

<210> 3

<211> 9

<212> PRT

<213> Influenza virus

<220>

<223> hemagglutinin epitope

<400> 3

Tyr Pro Tyr Asp Val Pro Asp Tyr Ala

1               5

## Description

### Technical Field

The present invention relates to a diphenyl ether compound which is useful for the treatment of hyperlipidemia, a process for producing the same and use thereof.

### Background Art

Recently in the study of transcription factors associated with marker genes expression induction in adipocytes differentiation, peroxisome proliferator activated receptor (abbreviated as PPAR hereinafter), which is one of intranuclear receptors, has got attention. The cDNAs of PPAR were cloned from various kinds of animals, and plural isoform genes were found, particularly in mammals three types of isoforms (α, δ, γ) are known (see *J. Steroid Biochem. Molec. Biol.,* 51, 157 (1994); *Gene Expression.,* 4, 281 (1995); *Biochem Biophys. Res. Commun.,* 224, 431 (1996); *Mol. Endocrinology.,* 6, 1634 (1992)). Further, it is known that PPAR γ isoform predominantly expresses in adipose tissues, immune cells, adrenal gland, spleen, small intestine, PPAR α isoform mainly expresses in adipose tissue, liver, retina, and PPAR δ isoform universally expresses without specificity for tissue (see *Endocrinology.,* 137, 354 (1996)).

By the way, thiazolidine derivatives such as pioglitazone, ciglitazone, rosiglitazone, troglitazone *etc*. are known as agents for the treatment of non-insulin dependent diabetes mellitus (NIDDM) and are hypoglycemic agents which are used for the improvement of hyperglycemia in the patients suffering from diabetes. They are also effective for the correction or improvement of hyperinsulinemia, improvement of glucose tolerance and decrease of serum lipid and therefore they are thought to be considerably hopeful as agents for the improvement of insulin resistance.

In addition, one of the intracellular target proteins of these thiazolidine derivatives is exactly PPAR γ and it is resolved that they enhance the transcription activity of PPAR γ (see Endocrinology., 137, 4189 (1996); Cell., 83, 803 (1995); Cell., 83, 813 (1995); J. Biol. Chem., 270, 12953 (1995)). Therefore, a PPAR γ activator (agonist) which enhances its transcription activity is thought to be hopeful as a hypoglycemic agent and/or a hypolipidemic agent. Furthermore, since a PPAR γ agonist is known to promote the expression of PPAR γ protein itself (Genes & Development., 10, 974 (1996)), an agent which increases the expression of PPAR γ protein itself as well as PPAR γ activating agent is also thought to be clinically useful.

Intracellular receptor, PPAR γ is related to adipocytes differentiation (see J. Biol. Chem., 272, 5637 (1997) and Cell., 83, 803 (1995)). It is known that thiazolidine derivatives which activate this receptor promote adipocytes differentiation. Recently it was reported that thiazolidine derivatives increase body fat and cause man to gain weight and to become obese (see Lancet., 349, 952 (1997)). From these, since PPAR γ activators (agonists) and PPAR γ promotors for its expression that can increase the expression of the protein itself have hypoglycemic effect, hypolipidemic effect, they are expected to be useful as agents for prevention and/or treatment of diseases associated with metabolic disorders (e.g. diabetes, hyperlipidemia, (hypercholesterolemia, hypo-HDL (high-density lipoprotein)-cholesterolemia, hyper-LDL (low-density lipoprotein)-cholesterolemia, hypertriglyceridemia *etc*.), atherosclerosis, cardiovascular disease, adiposity, metabolic syndrome *etc.),* hypertension, circulatory diseases *etc.*

Additionally, the fibrate compound (e.g., chlofibrate) is known as a hypolipidemic agent. It is also resolved that one of the intracellular target proteins of fbrate compounds is PPAR α (see *Nature*., 347, 645 (1990); *J. Steroid Biochem. Molec. Biol*., 51, 157 (1994); *Biochemistry*., 32, 5598 (1993)). From these facts, PPAR α activators are thought to have a hypolipidemic effect, and so they are expected to be useful as agents for prevention and/or treatment of hyperlipidemia *etc*.

Besides, it has been recently reported that PPAR α possesses anti-obese activity (see WO97/36579). In addition, it was reported that the metabolic stimulation effect of lipid (cholesterol, HDL, LDL and triglyceride *etc.)* were induced by PPAR α agonists (see *J*. *Lipid Res*., 39, 17 (1998)). That is, it was reported that they had the elevating effect of high-density lipoprotein (HDL) cholesterol and the lowering effect of low-density lipoprotein (LDL) cholesterol, very low-density lipoprotein (VLDL) cholesterol and triglyceride. It was also reported that composition of fatty acids in blood, hypertension and insulin resistance were improved by administration of bezafibrate which is one of fibrate compounds (see *Diabetes*., 46, 348 (1997)). Therefore, since agonists that activate PPAR α and PPAR α regulators that promote expression of PPAR α protein itself have hypolipidemic effect, they are hopeful agents for the treatment and/or prevention of lipid metabolic disorder (e.g. hyperlipidemia (hypercholesterolemia, hypo-HDL-emia, hyper-LDL-emia, hypertriglyceridemia *etc.),* atherosclerosis, cardiovascular disease, adiposity, metabolic syndrome *etc.),* hypertension, circulatory diseases *etc.*

On the other hand, PPAR δ is sometimes called PPAR β, or it is also called NUC1 in human. Until now, as for activity of PPAR δ, it is disclosed in the specification of WO 9601430 that hNUClB (PPAR subtype whose structure is different from that of human NUCl in one amino acid) inhibited the transcription activities of human PPAR α and thyroid hormone receptor. Recently, it was reported that the compounds, which possessed high affinity to PPAR δ protein and which could activate PPAR δ significantly (agonists) were found out and that they had HDL (high density lipoprotein) cholesterol level-elevating effect and non-HDL cholesterol level-lowering effect(see WO97/28149, WO01/00603, *Proc. Natl. Acad. Sci. U.S.A*. 98, 5306 (2001)). It has been known that macrophage takes up oxidized LDL resulting in foaming and is sedimented in angioendothelium whereby lipid metabolic diseases are induced. Accordingly, it is expected that agonists which are able to activate PPAR δ decrease foam cells due to, for example, an elevating effect for HDL cholesterol and a lowering effect for LDL and are useful, for example, as preventive and/or treating agents for diseases caused by abnormal lipid metabolism (such as hyperlipemia (*e*.*g*., hypercholesterolemia, hypo-HDL-emia, hyper-LDL-emia and hypertryglyceridemia), arteriosclerosis, cardiovascular disease, obesity and metabolic syndrome), hypertension, circulatory diseases, *etc*. Recently, it has been reported that activation of PPARδ promotes oxidation of fatty acid especially in skeletal muscles (see *Proc. Natl. Acad Sci. U.S.A*., 100, 15924 (2003); *Cell,* 113, 159 (2003)). They also show that PPARδ agonists are useful for the improvement of abnormal lipid metabolism and for the treatment of obesity.

The activation of PPARδ not only have the effect on lipid metabolic disorder, but also promote the cell differentiation of keratinocytes and is involved in sustain of skin structure as barrier function of organisms. It is observed that the over-proliferative changes of skins occur in PPAR δ-deficient mice treated with TPA (12-*O-*tetradecanoylphorbol-13-acetate) (see *Mol. Cell Biol.,* 20, 5119 (2000)). In addition, it is shown that it has the anti-inflammatory activity on dermal inflammation treated with TPA (see *J. Invest. Dermatol.,* 122, 971 (2004)). Therefore, PPAR δ agonists are useful for the preventive and/or therapeutic agent of dermal inflammatory disease (e.g. dermatitis (atopic dermatitis *etc.),* erythralgia, pruritus *etc*.) and are expected to have the effect as therapeutic facilitated drug of wound (*e*.*g*. burn wound, external wound *etc*.). Additionally, it is observed that callosal myelin coating disorder occurs in PPAR δ-deficient mice (see *Mol. Cell Biol*., 20, 5119 (2000)) and PPAR δ agonists have possibility of having utility as preventive and/or therapeutic agent of a certain nerve disease.

It is known that, for example, a carboxylic acid derivative represented by formula (A): 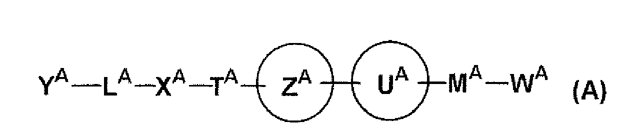 wherein L^{A}, M^{A}, T^{A} and X^{A} are single bond, *etc.;* W^{A} is carboxyl; Y^{A} is an aromatic group or an alicyclic hydrocarbon group; and Z^{A} and U^{A} are each an aromatic group,
has a PPAR agonistic action and is useful as an improving agent for insulin resistance (cf. WO 02/98840).

### Disclosure of the Invention

Accordingly, an object of the present invention is to provide a PPAR regulator which is useful for preventing and/or treatment agent for hyperlipidemia *etc*., has superior oral absorption and is safe.

The present inventors have conducted intensive studies for finding compounds having a PPARδ agonistic action and, as a result, they have found that the compounds of the present invention represented by formula (I) achieve the object and further that those compounds have an elevating action for HDL, a raising action for LDL clearance, a promoting action for carrying out of lipid or, particularly, cholesterol, a suppressive action for making macrophage into foams and an inhibitory action for biosynthesis of cholesterol whereupon the present invention has been achieved. The present inventors have further found that, as a result of a PPARδ agonistic action, the compounds have a promoting action for reverse transfer system where lipid (particularly cholesterol) in periphery (such as muscle tissue and angioendothelium) is transported to liver and then metabolized and discharged and found the possibility that a PPAR δ agonist acts as a promoter for reverse transfer. Thus, the present invention relates to the followings:
1. A compound represented by formula (I): 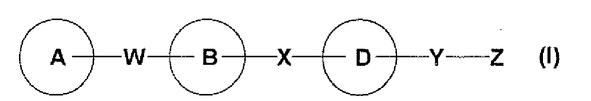 wherein ring A, ring B and ring D each independently represents a cyclic group which may have a substituent(s); W represents a spacer having 1 to 8 atom(s) in its main chain; X represents a spacer having 1 to 2 atom(s) in its main chain; Y represents a binding bond or a spacer having 1 to 8 atom(s) in its main chain; and Z represents an acidic group, or a salt thereof, a solvate thereof or a prodrug thereof,
2. The compound according to the above 1, which is represented by formula (IC): 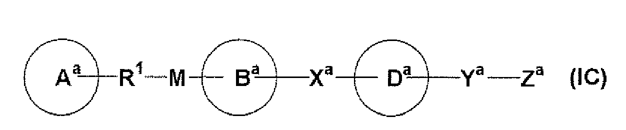 wherein ring A^{a}, ring B^{a} and ring D^{a} each independently represents a C5-10 monocyclic or bicyclic carbon ring which may have a substituent(s), or a 5- to 10-membered monocyclic or bicyclic heterocyclic ring containing 1 to 3 hetero atoms selected from an oxygen atom, a nitrogen atom and/or a sulfur atom; X^{a} represents -O-, -S-, -CO- or -CONR²-, in which R² represents a hydrogen atom, a hydrocarbon group which may have a substituent(s) or a cyclic group which may have a substituent(s); Y^{a} is a binding bond or methylene which may have a substituent(s); Z^{a} represents carboxyl which may be esterified; R¹ represents C1-6 alkylene, C2-6 alkenylene or C2-6 alkynylene; and M represents a spacer having 1 or 2 atoms in its main chain selected from an oxygen atom, carbonyl and a nitrogen atom which may have a substituent(s), or a salt thereof, a solvate thereof or a prodrug thereof,
3. The compound according to the above 1, wherein ring A is a benzene, a pyridine, oxazole or thiazole ring which may have a substituent(s); ring B is a benzene or pyridine ring which may have a substituent(s); ring D is a benzene or pyridine ring which may have a substituent(s); W is -R^{1a}-M^{a}-, in which R^{1a} is propylene, propenylene or propynylene, and M^{a} is -O-, -NH-, -NHCO- or -CONH-; X represents -O- or -CONR²-, in which R² represents a hydrogen atom, a hydrocarbon group which may have a substituent(s) or a cyclic group which may have a substituent(s); Y is a binding bond or methylene; and Z is carboxyl which may be esterified, or a salt thereof, a solvate thereof or a prodrug thereof,
4. The compound according to the above 2, which is represented by formula (ID): 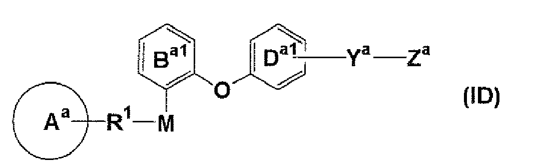 wherein ring B^{a1} and ring D^{a1} each independently represents a benzene ring which may have a substituent(s); and other symbols have the same meanings as described in the above 2, or a salt thereof, a solvate thereof or a prodrug thereof,
5. The compound according to the above 2, which is represented by formula (IE): 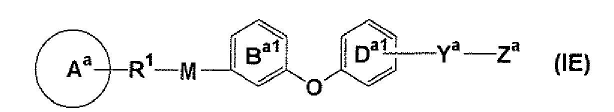 wherein all symbols have the same meanings as described in the above 2, or a salt thereof, a solvate thereof or a prodrug thereof,
6. The compound according to the above 2, which is represented by formula (IF): 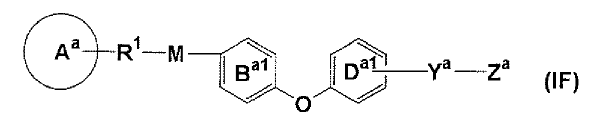 wherein all symbols have the same meanings as described in the above 2, or a salt thereof, a solvate thereof or a prodrug thereof,
7. The compound according to the above 1, which is selected from the group consisting of:
   (1) {4-methoxy-3-[3-({(2E)-3-[4-(trifluoromethyl)phenyl]-2-propen-1-yl}oxy)phenoxy]phenyl}acetic acid,
   (2) {2-chloro-4-methyl-3-[3-({(2E)-3-[4-(trifluoromethyl)phenyl]-2-propen-1-yl}oxy)phenoxy]phenyl}acetic acid,
   (3) {2-methyl-3-[2-({(2E)-3-[4-(trifluoromethyl)phenyl]-2-propenyl}oxy)phenoxy]phenyl}acetic acid,
   (4) 2-chloro-4-methyl-3-[3-({(2E)-3-[4-(trifluoromethyl)phenyl]-2-propenyl}oxy)phenoxy]benzoic acid,
   (5) 3-methyl-5-[2-({(2E)-3-[4-(trifluoromethyl)phenyl]-2-propenyl}oxy)phenoxy]benzoic acid,
   (6) {3-methyl-5-[2-({(2E)-3-[4-(trifluoromethyl)phenyl]-2-propenyl }oxy)phenoxy]phenyl}acetic acid,
   (7) {3-[4-methyl-2-({(2E)-3-[4-(trifluoromethyl)phenyl]-2-propenyl}oxy)phenoxy]phenyl}acetic acid,
   (8) {3-[2-({3-[4-(tifluoromethyl)phenyl]-2-propynyl}oxy)phenoxy]phenyl}acetic acid,
   (9) 3-methyl-5-[4-methyl-2-({3-[4-(trifluoromethyl)phenyl]-2-propynyl}oxy)phenoxy]benzoic acid,
   (10) {3-methyl-5-[2-({3-[4-(trifluoromethyl)phenyl]-2-propynyl }oxy)phenoxy]phenyl}acetic acid,
   (11) {3-methyl-5-[4-methyl-2-({3-[4-(trifluoromethyl)phenyl]-2-propynyl }oxy)phenoxy]phenyl}acetic acid,
   (12) {3-methyl-5-[4-methyl-2-({(2E)-3-[4-(trifluoromethyl)phenyl]-2-propenyl }oxy)phenoxy]phenyl }acetic acid,
   (13) [3-(2-{[3-(4-chlorophenyl)-2-propynyl]oxy}phenoxy)phenyl]acetic acid,
   (14) {2-chloro-5-[2-({3-[4-(trifluoromethyl)phenyl]-2-propynyl}oxy)phenoxy]phenyl}acetic acid,
   (15) {3-[2-chloro-6-({3-[4-(trifluoromethyl)phenyl]-2-propynyl}oxy)phenoxy]phenyl}acetic acid,
   (16) 3-methyl-5-[4-methyl-2-({3-[6-(trifluoromethyl)-3-pyridinyl]-2-propynyl}oxy)phenoxy]benzoic acid,
   (17) {3-[2-({(2E)-3-[4-(trifluoromethyl)phenyl]-2-propenyl}amino)phenoxy]phenyl}acetic acid,
   (18) 3-(2-{[3-(4-chlorophenyl)-2-propynyl]oxy}phenoxy)-5-methylbenzoic acid,
   (19) [3-(4-methyl-2-{[3-(4-methylphenyl)-2-propynyl]oxy}phenoxy)phenyl]acetic acid,
   (20) [3-(2-{[3-(4-chlorophenyl)-2-propynyl]oxy}phenoxy)-5-methylphenyl]acetic acid,
   (21) [3-methyl-5-(4-methyl-2-{[3-(4-methylphenyl)-2-propynyl]oxy}phenoxy)phenyl]acetic acid, and
   (22) {3-methyl-5-[4-methyl-2-({3-[6-(trifluoromethyl)-3-pyridinyl]-2-propynyl}oxy)phenoxy]phenyl}acetic acid,
      or a salt thereof, a solvate thereof or a prodrug thereof,
8. A pharmaceutical composition comprising the compound according to the above 1, or a salt thereof, a solvate thereof or a prodrug thereof,
9. The pharmaceutical composition according to the above 8, which is an agent for accelerating evacuation of lipid, an agent for reverse transport of lipid, an agent for inhibiting foam of macrophage, an agent for increasing HDL, an agent for decreasing LDL or an inhibitor of cholesterol biosynthesis,
10. The pharmaceutical composition according to the above 8, which is an agent for preventing and/or treating PPAR-mediated diseases,
11. The pharmaceutical composition according to the above 10, wherein PPAR is PPAR δ,
12. The pharmaceutical composition according to the above 11, wherein PPAR δ-mediated disease is hyperlipidemia or adiposity,
13. A medicament comprising the compound represented by formula (I) according to the above 1, a salt thereof, a solvate thereof or a prodrug thereof and one kind or more kinds selected from a MTP inhibitor, a HMG-CoA reductase inhibitor, a squalene synthase inhibitor, a fibrate drug, an ACAT inhibitor, a 5-lipoxygenase inhibitor, a cholesterol absorption inhibitor, a bile acid absorption inhibitor, an ileal Na⁺/bile acid transporter inhibitor, LDL receptor activator, LDL receptor expression enhancer, a pancreatic lipase inhibitor, a probucol formulation, a nicotine acid formulation and a cholesterol ester transporter protein inhibitor,
14. A method for accelerating evacuation of lipid in a mammal, which comprises administering to a mammal an effective amount of the compound represented by formula (I) according to the above 1, a salt thereof, a solvate thereof or a prodrug thereof,
15. Use of the compound represented by formula (I) according to the above, a salt thereof, a solvate thereof or a prodrug thereof for the manufacture of a medicament for accelerating evacuation of lipid,
16. A method for preventing and/or treating PPAR δ-mediated diseases in a mammal, which comprises administering to a mammal an effective amount of the compound represented by formula (I) according to the above 1, a salt thereof, a solvate thereof or a prodrug thereof, and
17. Use of the compound represented by formula (I) according to the above, a salt thereof, a solvate thereof or a prodrug thereof for the manufacture of a medicament for preventing and/or treating PPAR δ-mediated diseases.

In the present specification, a cyclic group in the cyclic group which may have a substituent(s) represented by ring A, ring B or ring D includes, for example, a carbon ring and a hetero ring. The carbon ring includes, for example, mono-, bi-, tri- or tetra-carbocyclic ring and examples include a C3-20 mono-, bi-, tri- or tetra-cyclic aromatic carbocyclic ring, a carbon ring in which a part or all thereof is saturated, a spire-linked bicyclic, tricyclic or tetracyclic carbon ring and a bridged bicyclic, tricyclic or tetracyclic carbon ring. The C3-20 mono-, bi-, tri- or tetra-cyclic aromatic carbon ring and carbon ring in which a part or all thereof is saturated include, for example, benzene, azulene, naphthalene, phenanthlene, anthracene, triphenylene, chrysene, naphthacene, pleiadene, cyclopropane, cyclobutane, cyclopentane, cyclohexane, cycloheptane, cyclooctane, cyclononane, cyclodecane, cycloundecane, cyclododecane, cyclotridodecane, cyclotetradecane, cyclopentadecane, cyclopentene, cyclohexene, cycloheptene, cyclooctene, cyclopentadiene, cyclohexadiene, cycloheptadiene, cyclooctadiene, pentalene, perhydropentalene, perhydroazulene, indene, perhydroindene, indane, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, fluoranthene, acephenanthrylene, aceanthrylene, pyrene rings and the like. The spiro-linked bicyclic, tricyclic or tetracyclic carbon ring and a bridged bicyclic, tricyclic or tetracyclic carbon ring include, for example, spiro[4.4]nonane, spiro[4.5]decane, spiro[5.5]undecane, bicyclo[2.2.1]heptane, bicycla[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, noradamantane rings, and the like.

The hetero ring includes a mono-, bi-, tri- or tetra-cyclic hetero ring containing 1 to 5 hetero atoms selected from an oxygen atom, a nitrogen atom and/or a sulfur atom is listed and examples include a 3- to 20-membered mono-, bi-, tri- or tetra-cyclic aromatic hetero ring containing 1 to 5 hetero atoms selected from an oxygen atom, a nitrogen atom and/or a sulfur atom which may be partially or fully saturated. The 3- to 20-membered mono-, bi-, tri- or tetra-cyclic aromatic hetero ring containing 1 to 5 hetero atoms selected from an oxygen atom, a nitrogen atom and/or a sulfur atom which may be partially or fully saturated includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, pyrrolopyridine, benzoxazole, benzothiazole, benzoimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzafuran, xanthene, dibenzothiophenene, phenothiazine, phenoxazine, phenoxathiine, thianthrene, phenanthridine, phenanthroline, perimidine, pyridonaphthyridine, pyrazoloisoquinoline, pyrazolonaphthyridine, pyrimidoindole, indolizinoindole, benzodioxol, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, benzooxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzoimidazole, perhydrobenzoimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, tetrapyridonaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydropyrazolopyridoazepine, hexahydropyrazolopyridoazepine, tetrahydropyrimidoindole, dihydrothiazinoindole, tetrahydrothiazinoindole, dihydrooxazinoindole, tetrahydrooxazinoindole, hexahydroindolizinoindole, dihydroindolobenzodiazepine, octahydroindoloquinolidine, hexahydroimidazopyridoindole, hexahydropyrrolothiazepinoindole, dioxolane, dioxane, dithiolane, dithiane, dioxaindane, benzodioxane, chromane, benzodithiolane, benzodithiane, azaspiro[4.4]nonane, oxazaspiro[4.4]nonane, oxaazaspiro[2.5]octane, dioxaspiro[4.4]nonane, azaspiro[4.5]decane, thiaspiro[4.5]decane, dithiaspira[4.5]decane, dioxaspiro[4.5]decane, oxazaspiro[4.5]decane, azaspiro[5.5]undecane, oxaspiro[5.5]undecane, dioxaspiro[5.5]undecane, 2,3,4,9-tetrahydrospiro[β-carboline-1,1'-cyclopentane], azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane rings, and the like.

The substituent(s) in the cyclic group which may have a substituent(s) include, for example, (1) alkyl which may have a substituent(s), (2) alkenyl which may have a substituent(s), (3) alkynyl which may have a substituent(s), (4) a carbocyclic group which may have a substituent(s), (5) a heterocyclic group which may have a substituent(s), (6) hydroxyl group which may have a substituent(s), (7) thiol which may have a substituent(s), (8) amino which may have a substituent(s), (9) carbamoyl which may have a substituent(s), (10) sulfamoyl which may have a substituent(s), (11) carboxyl, (12) alkoxycarbonyl (for example, a C1-6 alkoxycarbonyl such as methoxycarbonyl, ethoxycarbonyl and tert-butoxycarbonyl), (13) sulfo (-SO₃H), (14) sulfino, (15) phosphono, (16) nitro, (17) cyano, (18) amidino, (19) imino, (20) -B(OH)₂, (21) halogen atom (such as fluorine, chlorine, bromine and iodine), (22) alkylsulfinyl (for example, C1-4 alkylsulfinyl such as methylsulfinyl and ethylsulfinyl), (23) aromatic ring sulfinyl (for example, C6-10 aromatic ring sulfinyl such as phenylsulfinyl), (24) alkylsulfonyl (for example, C1-4 alkylsulfonyl such as methylsulfonyl and ethylsulfonyl), (25) aromatic ring sulfonyl (for example, C6-10 aromatic ring sulfonyl such as phenylsulfonyl), (26) acyl, (27) oxo, (28) thioxo, (29) (C1-6 alkoxyimino)methyl (such as (methoxyimino)methyl), (30) alkoxy which may have a substituent(s) (for example, a Cl-6 alkoxy which may be substituted with halogen atom(s), *etc.* such as methoxy, ethoxy, propoxy, hexyloxy and trifluoromethoxy) and (31) alkylthio which may have a substituent(s) (for example, a C1-6 alkylthio which may be substituted such as methylthio, ethylthio, propylthio, hexylthio and trifluoromethylthio) and one to five of any of these substituents may be substituted at the position(s) where substitution is possible. The carbocyclic group in (4) carbocyclic group which may have a substituent(s) as the substituent has the same meaning as the carbon ring in the cyclic group in the above-described cyclic group which may have a substituent(s) represented by the ring A. The heterocyclic group in (5) heterocyclic group which may have a substituent(s) as the substituent has the same meaning as the hetero ring in the cyclic group in the above-described cyclic group which may have a substituent(s) represented by the ring A. Examples of the substituent in (4) carbocyclic group which may have a substituent(s) and (5) cyclic group which may have a substituent(s) include C1-8 alkyl (such as methyl, ethyl, n-propyl, isopropyl, n-butyl, isobutyl, sec-butyl, tert-butyl, pentyl, hexyl, heptyl and octyl), C2-8 alkenyl (such as ethenyl, propenyl, butenyl, pentenyl, hexenyl, heptenyl and octenyl), C2-8 alkynyl (such as ethynyl, propynyl, butynyl, pentynyl, hexynyl, heptynyl and octynyl), hydroxyl group, amino, carboxyl, nitro, mono- or di-Cl-6 alkylamino (such as methylamino, ethylamino, propylamino, dimethylamino and diethylamino), C1-6 alkoxy (such as methoxy, ethoxy, propoxy and hexyloxy), C1-6 alkoxycarbonyl (such as methoxycarbonyl, ethoxycarbonyl and tert-butoxycarbonyl), C1-6 alkylcarbonyloxy (such as acetoxy and ethylcarbonyloxy), C1-4 alkylthio (such as methylthio, ethylthio, propylthio and butylthio), halogen atom (such as fluorine, chlorine, bromine and iodine), trihalomethyl (such as trifluoromethyl), trihalomethoxy (such as trifluoromethoxy) and trihalomethylthio (such as trifluoromethylthio) and 1 to 4 of any of these substituent may be substituted at the position(s) which can be substituted. Examples of the alkyl as a substituent in (1) alkyl which may have a substituent(s) include straight-chain or branched-chain C1-20 alkyl such as methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and icosyl. Examples of the substituent for the alkyl described above include hydroxyl, amino, carboxyl, nitro, mono-or di-C1-6 alkylamino (such as methylamino, ethylamino, propylamino, dimethylamino and diethylamino), N-aromatic ring amino (such as N-phenylamino), N-aromatic ring-N-(C1-6 alkyl)amino (such as N-phenyl-N-methylamino, N-phenyl-N-ethylamino, N-phenyl-N-propylamino, N-phenyl-N-butylamino, N-phenyl-N-pentylamino and N-phenyl-N-hexylamino), acylamino, N-acyl-N-(C1-6 alkyl)amino (examples of the C1-6 alkyl in that group are methyl, ethyl, propyl, butyl, pentyl and hexyl), C1-6 alkoxy (such as methoxy, ethoxy, propoxy, isopropoxy and hexyloxy), C3-7 cycloalkyl-C1-6 alkoxy (such as cyclohexylmethyloxy and cyclopentylethyloxy), C3-7 cycloalkyloxy (such as cyclohexyloxy), C7-15 aralkyloxy (such as benzyloxy, phenethyloxy, phenylpropyloxy, naphthylmethyloxy and naphthylethyloxy), phenoxy, C1-6 alkoxycarbonyl (such as methoxycarbonyl, ethoxycarbonyl and tert-butoxycarbonyl), C1-6 alkylcarbonyloxy (such as acetoxy and ethylcarbonyloxy), C1-4 alkylthio (such as methylthio, ethylthio, propylthio and butylthio), halogen atom (such as fluorine, chlorine, bromine and iodine), C1-4 alkylsulfonyl (such as methylsulfonyl, ethylsulfonyl, propylsulfonyl and butylsulfonyl), C6-10 aromatic ring sulfonyl (such as phenylsulfonyl), acyl, carbocyclic group which may have a substituent(s) (having the same meaning as the above-described (4) carbocyclic group which may have a substituent(s) as a substituent) and heterocyclic group which may have a substituent(s) (having the same meaning as the above-described (5) cyclic group which may have a substituent(s) as a substituent) and 1 to 4 of any of these substituents may be substituted at the position(s) which can be substituted. The acyl as a substituent for the alkyl or the acyl in acylamino and N-acyl-N-(C1-6 alkyl)amino has the same meaning as the acyl as a substituent in (6) hydroxyl group which may have a substituent(s), (7) thiol which may have a substituent(s) and (8) amino which may have a substituent(s) as a substituent which will be mentioned later. Examples of the alkenyl in (2) alkenyl which may have a substituent(s) as a substituent include straight-chain or branched-chain C2-20 alkyl such as ethenyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl and icosenyl. The substituent for alkenyl hereinabove has the same meaning as the substituent in the above-described (I) alkyl which may have a substituent(s) as a substituent. Examples of the alkynyl in (3) alkynyl which may have a substituent(s) as a substituent include straight-chain or branched-chain C2-20 alkynyl such as ethynyl, propynyl, butynyl, pentynyl, hexynyl, heptynyl, octynyl, nonynyl, decynyl, undecynyl, dodecynyl, tridecynyl, tetradecynyl, pentadecynyl, hexadecynyl, heptadecynyl, octadecynyl, nonadecynyl and icosynyl. Here, a substituent for the alkynyl has the same meaning as a substituent in the above-described (1) alkyl which may have a substituent(s) as a substituent. Examples of the substituent in (6) hydroxyl group which may have a substituent(s), (7) thiol which may have a substituent(s) and (8) amino which may have a substituent(s) as a substituent are alkyl which may have a substituent(s) (having the same meaning as the above-described (1) alkyl which may have a substituent(s)), carbocyclic group which may have a substituent(s) (having the same meaning as the above-described (4) a carbocyclic group which may have a substituent(s)), heterocyclic group which may have a substituent(s) (having the same meaning as the above-described (5) a heterocyclic group which may have a substituent(s)), alkylsulfonyl (for example, C1-4 alkylsulfonyl such as methylsulfonyl and ethylsulfonyl), aromatic ring sulfonyl (for example, C6-10 aromatic ring sulfonyl such as phenylsulfonyl) and acyl. Examples of the acyl mentioned here are alkylcarbonyl which may have a substituent(s), alkenylcarbonyl which may have a substituent(s), alkynylcarbonyl which may have a substituent(s), carbocyclic carbonyl which may have a substituent(s) and heterocyclic carbonyl which may have a substituent(s). The alkyl which may have a substituent(s) in alkylcarbonyl which may have a substituent(s) has the same meaning as the above-described (1) alkyl which may have a substituent(s) as a substituent. The alkenyl which may have a substituent(s) in alkenylcarbonyl which may have a substituent(s) has the same meaning as the above-described (2) alkenyl which may have a substituent(s) as a substituent. The alkynyl which may have a substituent(s) in alkynylcarbonyl which may have a substituent(s) has the same meaning as the above-described (3) alkynyl which may have a substituent(s) as a substituent. The carbocyclic which may have a substituent(s) in carbocyclic carbonyl which may have a substituent(s) has the same meaning as the above-described (4) carbocyclic group which may have a substituent(s) as a substituent. The heterocyclic which may have a substituent(s) in heterocyclic carbonyl which may have a substituent(s) has the same meaning as the above-described (5) heterocyclic group which may have a substituent(s) as a substituent. Examples of (9) carbamoyl which may have a substituent(s) as a substituent include unsubstituted carbamoyl, N-mono-C1-4 alkylcarbamoyl (such as N-methylcarbamoyl, N-ethylcarbamoyl, N-propylcarbamoyl, N-isopropylcarbamoyl and N-butylcarbamoyl), N,N-di-C1-4 alkylcarbamoyl (such as N,N-dimethylcarbamoyl, N,N-diethylcarbamoyl, N,N-dipropylcarbamoyl and N,N-dibutylcarbamoyl) and 1-piperidylcarbonyl. Examples of (10) sulfamoyl which may have a substituent(s) as a substituent include unsubstituted sulfamoyl, N-mono-C1-4 alkylsulfamoyl (such as N-methylsulfamoyl, N-ethylsulfamoyl, N-propylsulfamoyl, N-isopropylsulfamoyl and N-butylsulfamoyl), N,N-di-C1-4 alkylsulfamoyl (such as N,N-dimethylsulfamoyl, N,N-diethylsulfamoyl, N,N-diisopropylsulfamoyl and N,N-dibutylsulfamoyl). Also, (26) acyl as a substituent has the same meaning as the acyl in the above-described (6) hydroxyl group which may have a substituent(s) as a substituent.

The spacer having 1 to 8 atoms in its main chain represented by W means a space which is formed by connection of 1 to 8 atom(s). Here, numbers of atoms in the main chain are counted so as to make the atom in the main chain minimum. Examples of the spacer having 1 to 8 atoms in its main chain represented by W include C1-8 alkylene which may have a substituent(s) (such as methylene, ethylene, propylene, butylene, pentylene, hexylene, heptylene and octylene), C2-8 alkenylene which may have a substituent(s) (such as ethenylene, propenylene, butenylene, butadienylene, pentenylene, pentadienylene, hexenylene, hexadienylene, heptenylene, heptadienylene, octenylene and octadienylene) and C2-8 alkynylene which may have a substituent(s) (such as ethynylene, propynylene, butynylene, butadiynylene, pentynylene, pentadiynylene, hexynylene, hexadiynylene, heptynylene, heptadiynylene, octynylene and octadiynylene). Here, carbon atom in C1-8 alkylene, C2-8 alkenylene and C2-8 alkynylene may be substituted with one to three group(s) selected from an oxygen atom (-O-), a sulfur atom which may be oxidized (such as -S-, -SO- and -SO₂-), carbonyl (-CO-) and a nitrogen atom (-NH-) which may have a substituent(s) [examples of the substituent are (i) alkyl which may have a substituent(s) (having the same meaning as the above-described (I) alkyl which may have a substituent(s)), (ii) carbocyclic group which may have a substituent(s) (having the same meaning as the above-described (4) carbocyclic group which may have a substituent(s), (iii) heterocyclic group which may have a substituent(s) (having the same meaning as the above-described (5) heterocyclic group which may have a substituent(s) and (iv) acyl (having the same meaning as in the above-described (26) acyl)]. Examples of the substituent in C1-8 alkylene which may have a substituent(s), C2-8 alkenylene which may have a substituent(s) and C2-8 alkynylene which may have a substituent(s) include alkyl which may have a substituent(s) (having the same meaning as in the above-described (1) alkyl which may have a substituent(s)), halogen atom (such as fluorine, chlorine, bromine and iodine), hydroxyl group which may have a substituent(s) (having the same meaning as in the above-described (6) hydroxyl group which may have a substituent(s)), amino which may have a substituent(s) (having the same meaning as in the above-described (8) amino which may have a substituent(s)), oxo and imino which may have a substituent(s) (such as C1-6 alkylimino, hydroxyimino, C1-6 alkoxyimino and cyanoimino) and one to three of any of these substituents may be substituted at the position(s) which can be substituted. It is also possible that a ring (for example, C3-7 cycloalkyl such as cyclopropyl, cyclobutyl and cyclohexyl) is formed together with the atom in the main chain to which two substituents are bonded.

The spacer having 1 or 2 atoms in its main chain represented by X means a space where 1 to 2 atom(s) is/are connected. Here, numbers of atoms in the main chain are counted so as to make the atom in the main chain minimum. Examples of the spacer having 1 to 2 atoms in its main chain represented by X include -O-, -CO-, -CHOH-, -S-, -SO-, -SO₂-, -NR²-, -CONR²-, -NR²CO-, -SO₂NR²-, -NR²SO₂- [R² in the groups is a hydrogen atom, a hydrocarbon group which may have a substituent(s) or a cyclic group which may have a substituent(s)], C1-2 alkylene which may have a substituent(s), ethylene which may have a substituent(s) and acetylene. Examples of the hydrocarbon group which may have a substituent(s) include alkyl (for example, C1-15 alkyl such as methyl, ethyl, propyl, isopropyl, butyl, isobutyl, sec-butyl, tert-butyl, pentyl, hexyl, heptyl, octyl, nonyl, decyl, undecyl, dodecyl, tridecyl, tetradecyl and pentadecyl), alkenyl (for example, C2-10 alkenyl such as vinyl, allyl, 2-methylallyl, 2-butenyl, 3-butenyl and 3-octenyl) and alkynyl (for example, C2-10 alkynyl such as ethynyl, 2-propynyl and 3-hexynyl). The substituent in the hydrocarbon group which may have a substituent(s) has the same meaning as the substituent in the above-described (1) alkyl which may have a substituent(s). One to three of these substituent(s) may be substituted at any position(s) where substitution is possible. C1-2 alkylene in the C1-2 alkylene which may have a substituent(s) is methylene or ethylene. The substituent in the C1-2 alkylene which may have a substituent(s) has the same meaning as the substituent in the above-described (1) alkyl which may have a substituent(s). One to three of these substituent(s) may be substituted at any position(s) where substitution is possible.

The substituent in the ethylene which may have a substituent(s) has the same meaning as in the substituent in the above-described (1) alkyl which may have a substituent(s). One to two of these substituent(s) as such may be substituted at any position(s) where substitution is possible. The cyclic group which may have a substituent(s) represented by R² has the same meaning as in the above-described cyclic group which may have a substituent(s) represented by ring A.

The spacer having 1 to 8 atoms in its main chain represented by Y has the same meaning as the above-described spacer having 1 to 8 atoms in its main chain represented by W.

Acidic group represented by Z means, for example, carboxyl which may be esterified, sulfo (-SO₃H), -SO₂NHR² (R² has the same meaning as described above.), -NHSO₂R²- (R² has the same meaning as described above.), phosphono (-PO(OH)₂), phenol (-C₆H₄OH) or various Broensted acid such as residue of nitrogen ring having deprotonatable hydrogen atom *etc.* Broensted acid indicates a substance which gives hydrogen atom to other substance. Residue of nitrogen ring having deprotonatable hydrogen atom means, for example, 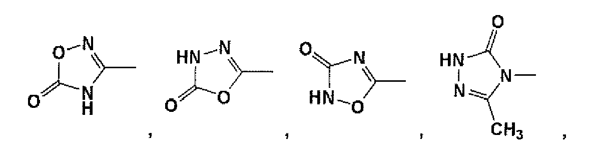 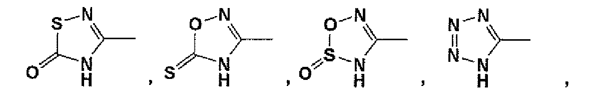 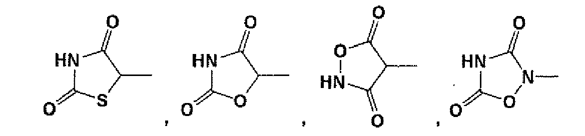 and the like. The carboxyl which may be esterified includes, for example, free carboxyl, alkoxycarbonyl (for example, C1-6 alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl or neopentyloxycarbonyl), aryloxycarbonyl (for example, C6-10 aryloxycarbonyl such as phenoxycarbonyl or 2-naphtyloxycarbonyl), aralkyloxycarbonyl (for example, C6-10 aryl-C1-4 alkoxycarbonyl such as benzyloxycarbonyl or phenethyloxycarbonyl), and the like.

Ring A, ring B or ring D is preferably a C5-10 monocyclic or bicyclic carbon ring which may have a substituent(s), or a 5- to 10-membered monocyclic or bicyclic heterocyclic ring containing 1 to 3 hetero atoms selected from an oxygen atom, a nitrogen atom and/or a sulfur atom. Herein, the C5-10 monocyclic or bicyclic carbon ring includes a C5-10 monocyclic or bicyclic aromatic carbon ring, a carbon ring in which a part or all thereof is saturated, a spiro-linked bicyclic carbon ring, and a bridged bicyclic carbon ring and the like. Examples include benzene, azulene, naphthalene, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, pentalene, perhydropentalene, perhydroazulene, indene, perhydroindene, indane, dihydronaphthalene, tetrahydronaphthalene, perhydronaphthalene, spiro[4.4]nonane, spiro[4.5]decane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-en rings. Also, the 5- to 10-membered monocyclic or bicyclic heterocyclic ring containing 1 to 3 hetero atoms selected from an oxygen atom, a nitrogen atom and/or a sulfur atom includes a 5- to 10-memebered monocyclic or bicyclic aromatic heterocyclic ring containing 1 to 3 hetero atoms selected from an oxygen atom, a nitrogen atom and/or a sulfur atom, which may be partially or fully saturated and the like. Examples include pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolidine, purine, phthalazine, pteridine, naphthyridine, quinoxaline, quinazoline, cinnoline, pyrrolopyridine, benzoxazole, benzothiazole, benzoimidazole, chromene, benzofurazan, benzothiadiazole, benzotriazole, benzodioxol, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, benzooxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzoimidazole, perhydrobenzoimidazole, dioxolane, dioxane, dithiolane, dithiane, dioxaindane, benzodioxane, chromane, benzodithiolane, benzodithiane, azaspiro[4.4]nonane, oxazaspiro[4.4]nonane, oxazaspiro[2.5]octane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2]octane and diazabicyclo[2.2.2]octane rings and the like. More preferred are a C5-10 monocyclic carbon ring and a 5- to 10-membered monocyclic heterocyclic ring containing 1 to 3 hetero atoms selected from an oxygen atom, a nitrogen atom and/or a sulfur atom. More preferred ring A is, for examples, a benzene, pyridine, oxazole or thiazole ring which may have a substituent. Ring A is preferably unsubstituted or substituted with 1 or 2 substituent(s) selected from alkyl which may have a substituent(s), a carbon ring which may have a substituent(s), a heterocyclic ring which may have a substituent(s), a halogen atom and alkoxy which may have a substituent(s). Preferred substituents for ring A are, for example, 1 or 2 substituent(s) selected from C1-4 alkyl (such as methyl or trifluoromethyl or the like) which may have a substituent(s) (such as a halogen atom), C5-10 monocyclic carbon ring which may have a substituent(s) (such as methyl, trifluoromethyl or a halogen atom), 5- to 10-membered monocyclic heterocyclic ring which may have a substituent(s) (such as methyl, trifluoromethyl or a halogen atom), and C1-6 alkoxy (such as methoxy, ethoxy, propoxy, hexyloxy or trifluoromethoxy) which may have a substituent(s) (such as a halogen atom) and the like.

Ring B is preferably, for example, a benzene or pyridine ring which may have a substituent(s). Ring B is preferably unsubstituted or substituted with 1 or 2 substituent(s) selected from alkyl which may have a substituent(s) (such as methyl or trifluoromethyl), nitro, a halogen atom and alkoxy which may have a substituent(s) (such as methoxy or trifluoromethoxy).

Ring D is preferably, for example, a benzene or pyridine ring which may have a substituent(s). Ring D is preferably unsubstituted or substituted with 1 or 2 substituent(s) selected from alkyl which may have a substituent(s) (such as methyl or trifluoromethyl), nitro, a halogen atom and alkoxy which may have a substituent(s) (such as methoxy or trifluoromethoxy).

The spacer having 1 to 8 atoms in its main chain represented by W is preferably C3-8 alkylene, C3-8 alkenylene or C3-8 alkynylene which may have a substituent(s) in which 1 or 2 carbon atom(s) is (are) substituted with an oxygen atom, a sulfur atom which may be oxidized, carbonyl or a nitrogen atom which may have a substituent(s), and more preferably butylene, butenylene or butynylene which may have a substituent(s) in which one carbon atom is substituted with an oxygen atom.

W is more preferably, for example, -R¹-M- wherein R¹ is C1-6 alkylene, C2-6 alkenylene or C2-6 alkynylene, and M represents a spacer having 1 or 2 atoms in its main chain selected from an oxygen atom, carbonyl and a nitrogen atom which may have a substituent(s). R¹ is preferably, for example, ethylene, propylene, butylene, ethenylene, propenylene, ethynylene, propynylene or the like, and M is preferably, for example, -O-, -NH-, -NHCO-, -CONH- or the like.

The spacer having 1 or 2 carbon atom(s) in its main chain represented by X is prefearably -O-, -S-, -CO- or -CONR²-, and more preferably -O- or -CONR²-.

The binding bond or the spacer having 1 to 8 atoms in its main chain represented by Y is preferably a binding bond or C1-2 alkylene which may have a substituent(s), and more preferably a binding bond or methylene which may have a substituent(s).

The acidic group represented by Z is preferably carboxyl which may be esterified, and more preferably carboxyl, methoxycarboxyl or ethoxycarboxyl.

The compound represented by formula (I) preferably inlcudes compounds described in Examples, compounds represented by the following formulae, and the like: 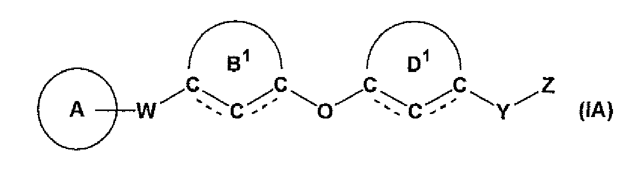 wherein ring B¹ and ring D¹ each independently represents a carbocyclic ring which may have a substituent(s); 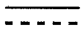 represents a single bond or a double bond; other symbols have the same meanings as described above, and wherein neither ring B¹ nor ring D¹ forms an arene structure, 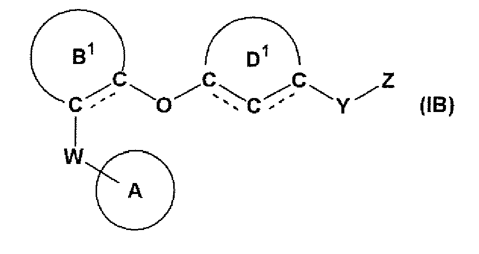 wherein all symbols have the same meanings as described above, 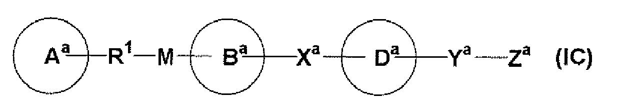 wherein ring A^{a}, ring B^{a} and ring D^{a} each independently represents a C5-10 monocyclic or bicyclic carbon ring which may have a substituent(s), or a 5- to 10-membered monocyclic or bicyclic heterocyclic ring containing 1 to 3 hetero atoms selected from an oxygen atom, a nitrogen atom and/or a sulfur atom; X^{a} represents -O-, -S-, -CO- or -CONR²-; Y^{a} represents a binding bond or methylene which may have a substituent(s); Z^{a} represents carboxyl which may be esterified; other symbols have the same meanings as described above, 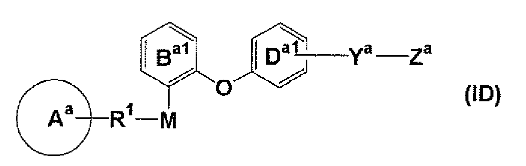 wherein ring B^{a1} and ring D^{a1} each independently represents a benzene ring which may have a substituent(s); other symbols have the same meanings as described above, 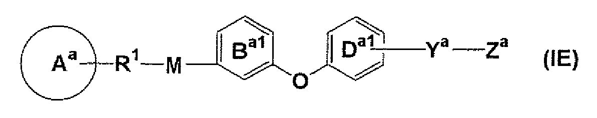 wherein all symbols have the same meanings as described above, 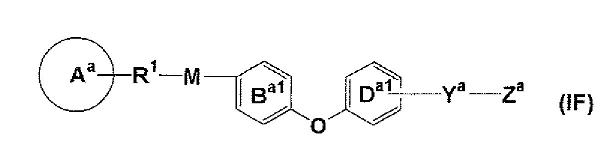 wherein all symbols have the same meanings as described above,
More preferred compounds include: 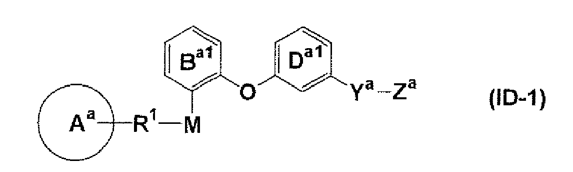 wherein all symbols have the same meanings as described above, 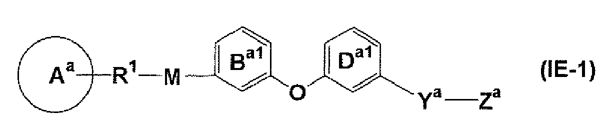 wherein all symbols have the same meanings as described above, 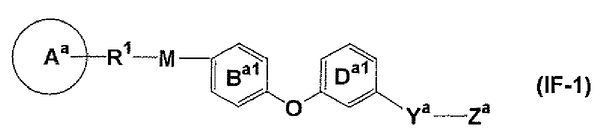 wherein all symbols have the same meanings as described above.

More preferred compounds include, for example, {4-methoxy-3-[3-({(2E)-3-[4-(trifluoromethyl)phenyl]-2-propen-1-yl} oxy)phenoxy]phenyl} acetic acid, {2-chloro-4-methyl-3-[3-({(2E)-3-[4-(trifluoromethyl)phenyl]-2-propen-1-yl}oxy)phenoxy]phenyl} acetic acid, {2-methyl-3-[2-({(2E)-3-[4-(trifluoromethyl)phenyl]-2-propenyl}oxy)phenoxy]phenyl}acetic acid, 2-chloro-4-methyl-3-[3-({(2E)-3-[4-(trifluoromethyl)phenyl]-2-propenyl}oxy)phenoxy]benzoic acid, 3-methyl-5-[2-({(2E)-3-[4-(triftuoromethyl)phenyl]-2-propenyl}oxy)phenoxy]benzoic acid, {3-methyl-5-[2-({(2E)-3-[4-(trifluoromethyl)phenyl]-2-propenyl}oxy)phenoxy]phenyl}acetic acid, {3-[4-methyl-2-({(2E)-3-[4-(trifluoromethyl)phenyl]-2-propenyl}oxy)phenoxy]phenyl}acetic acid, {3-[2-({3-[4-(trifluoromethyl)phenyl]-2-propynyl}oxy)phenoxy]phenyl}acetic acid, 3-methyl-5-[4-methyl-2-({3-[4-(trifluoromethyl)phenyl]-2-propynyl}oxy)phenoxy]benzoic acid, {3-methyl-5-[2-({3-[4-(trifluoromethyl)phenyl]-2-propynyl}oxy)phenoxy]phenyl}acetic acid, {3-methyl-5-[4-methyl-2-({3-[4-(trifluoromethyl)phenyl]-2-propynyl}oxy)phenoxy]phenyl}acetic acid, {3-methyl-5-[4-methyl-2-({(2E)-3-[4-(trifluoromethyl)phenyl]-2-propenyl}oxy)phenoxy]phenyl}acetic acid, [3-(2-{[3-(4-chlorophenyl)-2-propynyl]oxy}phenoxy)phenyl]acetic acid, {2-chloro-5-[2-({3-[4-(trifluoromethyl)phenyl]-2-propynyl}oxy)phenoxy]phenyl}acetic acid, {3-[2-chloro-6-({3-[4-(trifluoromethyl)phenyl]-2-propynyl}oxy)phenoxy]phenyl}acetic acid, 3-methyl-5-[4-methyl-2-({3-[6-(trifluoromethyl)-3-pyridinyl]-2-propynyl}oxy)phenoxy]benzoic acid, {3-[2-({(2E)-3-[4-(trifluoromethyl)phenyl]-2-propenyl}amino)phenoxy]phenyl}acetic acid, 3-(2-{[3-(4-chlorophenyl)-2-propynyl]oxy}phenoxy)-5-methylbenzoic acid, [3-(4-methyl-2-{[3-(4-methylphenyl)-2-propynyl]oxy}phenoxy)phenyl]acetic acid, [3-(2-{[3-(4-chlorophenyl)-2-propynyl]oxy}phenoxy)-5-methylphenyl]acetic acid, [3-methyl-5-(4-methyl-2-{[3-(4-methylphenyl)-2-propynyl]oxy}phenoxy)phenyl]acetic acid, or {3-methyl-5-[4-methyl-2-({3-[6-(trifluoromethyl)-3-pyridinyl]-2-propynyl}oxy)phenoxy]phenyl}acetic acid,
and the like, or a salt thereof, a solvate thereof or a prodrug thereof

Unless otherwise specified, all isomers are included in the present invention. For example, alkyl, alkenyl, alkynyl, alkoxy, alkylthio, alkylene alkenylene and alkynylene group means straight-chain or branched-chain ones. In addition, isomers on double bond, ring, fused ring (E-, Z-, cis-, trans-isomer), isomers generated from asymmetric carbon atom(s) (R-, S-isomer, α-, β-configuration, enantiomer, diastereomer), optically active isomers (D-, L-, d-, 1-isomer), polar compounds generated by chromatographic separation (more polar compound, less polar compound), equilibrium compounds, rotational isomers, mixtures thereof at voluntary ratios and racemic mixtures are also included in the present invention.

The compound represented by formula (I) is converted into a salt by a known method. The salt is preferably a pharmaceutically acceptable salt.

The salt includes an alkaline metal salt, an alkaline earth metal salt, an ammonium salt, an amine salt, an acid addition salt and the like.

The salt is preferably a water-soluble salt. Suitable salts include salts of alkali metal (potassium, sodium or the like), salts of alkaline earth metal (calcium, magnesium or the like), an ammonium salt, salts of a pharmaceutically acceptable organic amine (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine or the like), and the like.

The acid addition salt is preferably a water-soluble acid addition salt. Suitable acid addition salts include inorganic salts such as hydrochloride, hydrobromate, hydroiodate, sulfate, phosphate and nitrate, and organic acid salts such as acetate, lactate, tartrate, benzoate, citrate, methanesulfonate, ethanesulfonate, benzenesulfonate, taluenesulfonate, isethionate, glucuronate and gluconate.

The compound represented by formula (I) and a salt thereof can also be converted into a solvate thereof

The solvate is preferably a non-toxic or water-soluble solvate. Suitable solvates include, for example, solvates of water or alcoholic solvents (such as ethanol).

All of the compounds represented by formula (I) and pharmaceutically acceptable salts thereof are preferred. Examples include compounds described in Examples and pharmaceutically acceptable salts thereof

Furthermore, the salt includes a quaternary ammonium salt. The quaternary ammonium salt is one in which the nitrogen atom of the compound of the present invention is quaternarized by R⁰.

R⁰ represents C1-8 alkyl or C1-8 alkyl substituted with phenyl.

The compound of the present invention can be converted into an N-oxide by any method. The N-oxide means the nitrogen atom in the compound of the present invention is oxidized.

Additionally, the prodrug of the compounds represented by formula (I) means a compound is the compound represented by formula (I) by reaction with enzymes, gastric acids and so on within an organism. The prodrug of the compounds represented by formula (I) include, when the compounds represented by formula (I) have amino, the prodrug is the compounds the amino of which is acylated, alkylated, phosphorylated (e.g., the compounds are that the amino of the compounds represented by formula (I) is eicosanoated, alanylated, pentylaminocarbonylated, (5-methyl-2-oxo-1,3-dioxolan-4-yl)methoxycarbonylated, tetrahydrofuranated, pyrrolidylmethylated, pivaloyloxymethylated, acetoxymethylated, tert-butylated, *etc*.); when the compounds represented by formula (I) have hydroxyl, the prodrug is the compounds the hydroxyl of which are acylated, alkylated, phosphorylated, borated (*e.g*., the compounds are that the hydroxyl of the compounds represented by formula (I) are acetylated, palmitoylated, propanoylated, pivaloylated, succinylated, fumarylated, alanylated, dimethylaminomethylcarbonylated *etc.);* when the compounds represented by formula (I) have carboxyl, the prodrug is the compound the carboxyl of which are esterified, amidated (e.g., the compounds are that the carboxyl of the compounds represented by formula (I) is ethylesterified, phenylesterified, carboxymethylesterifted, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, methylamidated *etc*.); and so on. These compounds can be produced by known methods. In addition, the prodrug of the compound represented by formula (I) may be either hydrate or non-hydrate. Additionally, the prodrug of the compound represented by formula (I) may be converted into the compound represented by formula (I) under the physiological condition which is described in "the development of medicine" vol.7 "molecular design" published in 1991 Hirokawa shoten p.p. 163-198. Further, the compound represented by formula (I) may be labeled with isotopes (*e.g*. ³H, ¹⁴C, ³⁵S, ¹²⁵I, *etc.*) and so on.

In the present invention, PPAR agonist and antagonist includes all mode of action, that is, PPAR α, γ, δ, α+γ, α+δ, γ+δ, and α+γ+δ agonist and antagonist. In addition, preferable mode of action of the present invention is PPAR δ agonist.

### Processes for the Preparation of the compound of the present invention:

The compound of the present invention represented by formula (I) can be prepared by combining the known processes, for example, the following processes, or the processes shown in Examples, which is the properly improved processes described in *"Comprehensive Organic Transformations : A Guide to Functional Group Preparations,* 2nd Edition "Richard C. Larock, Wiley & Sons Inc, 1999" and so on, Still, ingredients may be used as salts in the following each processes for the preparation. As these salts, the salts described as the above described ones in the above-mentioned formula (I) are used.

Among the compounds of the present invention represented by formula (I), that where W is -R¹-M- and M is -O- or, in other words, the compound represented by formula (I-1): 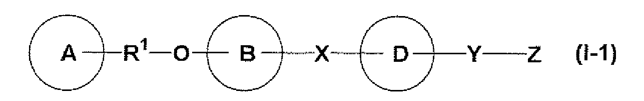 wherein all symbols have the same meanings as described above,
can be produced by the following process (A) or (B).
(A) The compound represented by formula (I-1) can be produced in such a manner that a compound represented by formula (II): 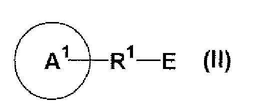
wherein ring A¹ has the same meaning as ring A and the carboxyl, hydroxyl, amino or mercapto included in the group represented by the ring A¹ may be protected, if protection is necessary; E represents a halogen atom or a leaving group; and other symbols have the same meanings as described above,
and the compound represented by formula (III): 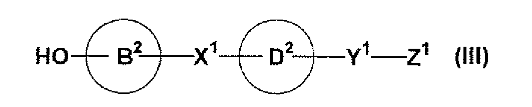 wherein ring B², X¹, ring D², Y¹ and Z¹ have the same meanings as ring B, X, ring D, Y and Z, respectively, and carboxyl, hydroxyl, amino or mercapto included in the group represented by ring B², X¹, ring D², Y¹ and Z¹ may be protected, if protection is necessary,
are subjected to an etherifying reaction to thereby produce a compound represented by formula (I-2): 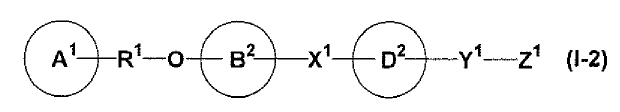 wherein all symbols have the same meanings as described above,
and, if necessary, it is subjected to a deprotecting reaction for the protective group.

The etherifying reaction has been known and is carried out, for example, by conducting the reaction at 0 to 100°C in an organic solvent (such as dimethylformamide, dimethyl sulfoxide, chloroform, dichloromethane, diethyl ether, tetrahydrofuran and methyl tert-butyl ether) in the presence of a base (such as an inorganic base including alkali metal or alkali earth metal hydride such as sodium hydride and potassium hydride, alkyl lithium such as butyl lithium, sec-butyl lithium and tert-butyl lithium, alkali metal alkoxide such as sodium methoxide and sodium ethoxide, alkali metal such as metal sodium and metal potassium; an organic base including alkylamine such as triethylamine, tributylamine and diisopropylethylamine, aromatic amine such as N,N-dimethylaniline, pyridine, lutidine, collidine and 4-(dimethylamino)pyridine and DBU (1,8-diazabicyclo[5,4,0]undecene-7); alkali metal hydroxide (such as sodium hydroxide, potassium hydroxide and lithium hydroxide), alkali earth metal hydroxide (such as barium hydroxide and calcium hydroxide) or carbonate (such as sodium carbonate and potassium carbonate) or an aqueous thereof or a mixture thereof).

In the present specification, the halogen atom represented by E includes, for example, fluorine, chlorine, bromine and iodine.

In the present invention, the leaving group represented by E includes, for example, methanesulfonyloxy, toluenesulfonyloxy, chloromethanesulfonyloxy, trichloromethanesulfonyloxy, diphenylphosphonoxy, diethylphosphonoxy, trifluoromethyloxy, benzenesulfonyloxy, naphthalenesulfonyloxy, p-bromobenzenesulfonyloxy, p-nitrobenzenesulfonyloxy, m-nitrobenzenesulfonyloxy, o-nitrobenzenesulfonyloxy and the like.
(B) The compound represented by formula (I-1) can be produced in such a manner that a compound represented by formula (II-1): 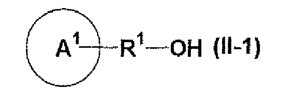 wherein all symbols have the same meanings as described above,
and a compound represented by formula (III): 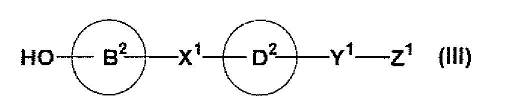 wherein all symbols have the same meanings as described above,
are subjected to Mitsunobu reaction to thereby produce a compound represented by the above-described formula (I-2) and, if necessary, it is subjected to a deprotecting reaction for the protective group.

The Mitsunobu reaction has been known and is carried out, for example, by conducting the reaction at 0 to 60°C in an organic solvent (such as dichloromethane, diethyl ether, tetrahydrofuran, acetonitrile, benzene and toluene) in the presence of an azo compound (such as diethyl azodicarboxylate (DEAD), diisopropyl azodicarboxylate, 1,1'-(azodicarbonyl)dipiperidine and 1,1'-azobis(N,N-dimethylformamide) and a phosphine compound (such as triphenyl phosphine, tributyl phosphine, trimethyl phosphine and polymer support triphenyl phosphine).

The deprotection reaction of the protective group may be carried out by following method. The deprotection reaction of a protective group for carboxyl, hydroxyl, amino, or mercapto is known, and it includes
(1) alkaline hydrolysis,
(2) deprotection reaction under acidic conditions,
(3) deprotection reaction by hydrogenolysis,
(4) deprotection reaction of a silyl group,
(5) deprotection reaction using metals,
(6) deprotection reaction using metal complexes, and so on.

These methods are described concretely as follows.
(1) The deprotection reaction by alkaline hydrolysis is, for example, carried out in an organic solvent (*e.g*., methanol, tetrahydrofuran, or dioxane *etc*.) using a hydroxide of an alkali metal (*e.g*., sodium hydroxide, potassium hydroxide, or lithium hydroxide *etc.),* a hydroxide alkaline earth metal (e.g., barium hydroxide, or calcium hydroxide *etc*.), or a carbonate (*e.g.*, sodium carbonate or potassium carbonate, *etc*.), or an aqueous solution thereof, or a mixture thereof at a temperature of 0 to 100°C.
(2) The deprotection reaction under acidic conditions is carried out, for example, in an organic solvent (*e*.*g*., dichloromethane, chloroform, dioxane, ethyl acetate, or anisole *etc*.) in an organic acid (*e.g.*, acetic acid, trifuloroacetic acid, methansulfonic acid, or p-tosylate, *etc.),* or an inorganic acid *(e.g*., hydrochloric acid, or sulfuric acid, *etc*.) or a mixture thereof (*e.g.*, hydrogen bormide/acetic acid, *etc.*) at a temperature of 0 to 100°C.
(3) The deprotection reaction by hydrogenolysis is carried out, for example, in a solvent (*e.g*., ethers (*e.g*., tetrahydrofuran, dioxane, dimethoxyethane, or diethylether, *etc*.), alcohols (*e.g.,* methanol, or ethanol, *etc*.), benzenes (*e.g.*, benzene, or toluene *etc*.), ketones (*e.g*., acetone, or methylethylketone, *etc.*)*,* nitriles (*e*.*g*., actetonitrile *etc.*), amides (*e.g*., dimethylformamide *etc*.), water, ethyl acetate, acetic acid, or a mixed solvent of at least two of these *etc.)* in the presence of a catalyst (*e.g.*, palladium-carbon, palladium black, palladium hydroxide, platinum oxide, or Raney nickel, *etc.)* under the hydrogen atmosphere at normal pressure or under pressurization, or in the presence of ammonium formate at a temperature of 0 to 200°C.
(4) The deprotection reaction of a silyl group is carried out, for example, in a water-miscible organic solvent *(e.g.,* tetrahydrofuran, or acetonitrile, *etc.)* using tetrabutylammonium fluoride at a temperature of 0 to 40°C.
(5) The deprotection reaction using metals is carried out, for example, in an acidic solvent (*e*.*g*., acetic acid, pH4.2-7.2 buffer solution, or a mixture of a solution thereof and an organic solvent of tetrahydrofran *etc.)* in the presence of zinc powder, if necessary sonicating, at the temperature of 0 to 40°C.
(6) The deprotection reaction using metal complexes is carried out, for example, in an organic solvent (*e.g*., dichloromethane, dimethylformamide, tetrahydrofuran, ethyl acetate, acetonitrile, dioxane, ethanol *etc.),* water, or a mixture thereof, in the presence of a trap reagent (*e.g.*, tributyltine hydride, triethylsilane, dimedone, morpholine, diethylamine, pyrrolidine, *etc*.), an organic acid *(e.g.,* acetic acid, formic acid, 2-ethyl hexanoic acid, *etc.)* and/or salts of organic acid (*e.g.*, sodium 2-ethylhexanoate, potassium 2-ethylhexanoate *etc.),* in the presence or absence of a phosphine reagent (*e.g.*, triphenylphosphine *etc*.*),* using metal complexes (*e.g.*, tetrakistriphenylphosphinepalladium(0), dichlorobis(triphenylphosphine)palladium(II), palladium acetate(II), tris(triphenylphosphine)rhodium(I) chloride *etc*.) at the temperature of 0 to 40°C.

In addition, the deprotection reaction except the above-mentioned processes can be carried out, for example, by the process described in T.W. Greene, *Protective Groups in Organic Synthesis,* Wiley, New York, 1999. As is easily understood by those skilled in the art, the intended compounds of the present invention may be readily prepared through selective use of these deprotecting reactions.

The protection group for carboxyl includes, for example, methyl, ethyl, allyl, t-butyl, trichloroethyl, benzyl (Bn), phenacyl, p-methoxybenzyl, trityl , 2-chlorotrityl, solid phase carriers to which these structures are bound, and so on. The protection group for hydroxyl includes, for example, methyl, trytyl, methoxymethyl (MOM), 1-ethoxyethyl (EE), methoxyethoxymethyl (MEM), 2-tetrahydropyranyl (THP), trimethylsyryl (TMS), triethylsyryl (TES), t-butyldimethylsyryl (TBDMS), t-butyldiphenylsyryl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc) and so on. The protection group of amino includes benzyloxycarbonyl, t-butoxycarbonyl, allyloxycarbonyl (Alloc), 1-methyl-1-(4-biphenyl) ethoxycarbonyl (Bpoc), trifluoroacetyl, 9-fluorenylmethoxycarbonyl, benzyl (Bn), p-methoxybenzyl, benzyloxymethyl (BOM), 2-(trimethylsyryl) ethoxymethyl (SEM) and so on. The protection group of thiol includes, for example, benzyl (Bn), methoxybenzyl, methoxymethyl (MOM), 2-tetrahydropyranyl (THP), diphenylmethyl, acetyl (Ac) and so on. The protective group for carboxyl, hydroxyl, amino or thiol is not particularly limited to the above mentioned groups, so long as it can be easily and selectively left.

Among the compounds represented by formula (I), a compound wherein W is -R¹-M- and M is -NH- or, in other words, a compound represented by formula (I-3): 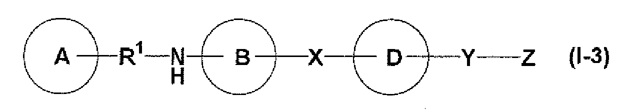 wherein all symbols have the same meanings as described above,
can be produced by subjecting the compound represented by formula (II) and the compound represented by formula (IV): 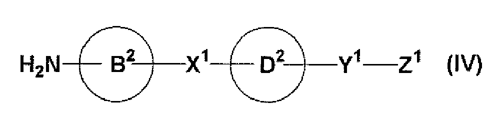 wherein all symbols have the same meanings as described above,
to the following reaction and, if necessary, the protective group is subjected to a deprotecting reaction.

This reaction is known and is carried out, for example, at 0°C to a refluxing temperature in an organic solvent (such as tetrahydrofuran, dimethylformamide and dioxane) using a base (such as sodium hydride, potassium hydride, sodium hydroxide, potassium hydroxide, potassium carbonate, sodium carbonate and cesium carbonate).

The deprotecting reaction for the protective group may be carried out in the same manner as mentioned above.

In addition, the compound represented by formula (I-3) may also be produced by such a manner that the compound represented by formula (II-1) and the compound represented by formula (IV) are subjected to the following reaction and, if necessary, further subjected to a deprotecting reaction of the protective group.

This reaction has been known and is carried out, for example, at 0°C to a refluxing temperature in an organic solvent (such as benzene, acetonitrile, tetrahydrofuran, N,N-dimethylformamide, toluene, dioxane and hexamethyl phosphoramide) in the presence of a palladium catalyst (such as palladium acetate, palladium chloride, tetrakistriphenylphosphinepalladium, palladium trifluoroacetate, palladium (II) acetylacetonate and dichlorobis(acetonitrile) palladium (II)) and a titanium catalyst (such as titanium (IV) isopropoxide, titanium (IV) ethoxide, titanium (IV) N-propoxide, tetra-n-butyl orthotitanate, titanium (IV) tetrachloride, titanium (IV) methoxide, titanium (IV) bis(acetylacetonate) diisopropoxide and chlorotitanium triisopropoxide) in the presence or absence of a dehydrating agent (such as molecular sieves (such as MS4Å and MS3Å), silica gel, active alumina and magnesium sulfate) and in the presence or absence of a phosphine compound (such as triphenyl phosphine, tributyl phosphine, 1,2-bis(diphenylphosphino)ethane, bis(diphenylphosphino)methane, 1,3-bis(diphenylphosphino)propane, 1,4-bis(diphenylphosphino)butane, triphenyl phosphite, tris(4-chlorophenyl) phosphine, tris(4-fluorophenyl) phosphine, tris(4-methylphenyl) phosphine, tris(4-methoxyphenyl) phosphine, tri-m-tolylphosphine, tris(2,6-dimethoxyphenyl) phosphine and tris(2,4,6-trimethoxyphenyl) phosphine.

A deprotecting reaction of the protective group is able to be carried out by the same manner as above.

Among the compounds of the present invention represented by formula (I), a compound in which W is -R¹-M- and M is -CONH- or, that is, a compound represented by formula (I-4): 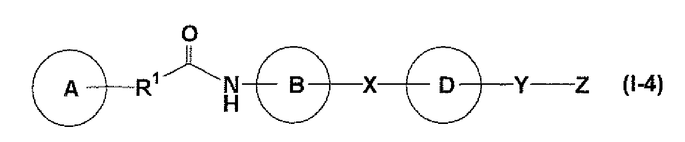 wherein all symbols have the same meanings as described above,
can be produced in such a manner that a compound represented by formula (V): 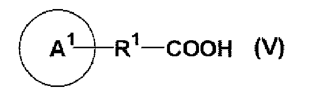 wherein all symbols have the same meanings as described above,
and a compound represented by formula (IV) are subjected to an amidation reaction and, if necessary, further subjected to a deprotecting reaction of the protective group.

The amidation is known. For example, it includes the method
(1) via an acyl halide,
(2) via a mixed acid anhydride,
(3) using a condensing agent.

These methods are explained as follows.
(I) The method via an acyl halide may be carried out, for example, by reacting carboxylic acid with an acyl halide (e.g., oxalyl chloride or thionyl chloride) in an organic solvent (*e*.*g*., chloroform, dichloromethane, diethyl ether or tetrahydrofuran) or without a solvent at about -20°C to reflux temperature. And then the obtained acyl halide derivative may be reacted with amine in an organic solvent (*e*.*g*., chloroform, dichloromethane, diethyl ether or tetrahydrofuran) in the presence of a base (e.g., pyridine, triethylamine, dimethylaniline, dimethylaminopyridine or diisopropylethylamine *etc*.) at about 0 to 40°C. As an alternative, the obtained acyl halide derivative may be reacted with amine in an organic solvent (*e*.*g*., dioxane, tetrahydrofuran) using an alkaline aqueous solution (e.g., sodium hydrogen carbonate, sodium hydroxide) at about 0 to 40°C.
(2) The method via a mixed acid anhydride may be carried out, for example, by reacting carboxylic acid with an acyl halide *(e.g.,* pivaloyl chloride, tosyl chloride or mesyl chloride) or an acid derivative (*e.g.,* ethyl chloroformate or isobutyl chloroformate) in an organic solvent (*e*.*g*., chloroform, dichloromethane, diethyl ether, tetrahydrofuran) or without a solvent, in the presence of a base (*e*.*g*., pyridine, triethylamine, dimethylaniline, dimethylaminopyridine or diisopropylethylamine) at about 0 to 40°C. And then the obtained mixed acid anhydride derivative may be reacted with amine in an organic solvent (*e.g.*, chloroform, dichloromethane, diethyl ether or tetrahydrofuran) at about 0 to 40°C.
(3) The method using a condensing agent may be carried out, for example, by reacting carboxylic acid with amine in an organic solvent (*e.g.*, chloroform, dichloromethane, dimethylformamide, diethyl ether or tetrahydrofuran) or without a solvent, in the presence or absence of a base (*e.g.*, pyridine, triethylamine, dimethylaniline or dimethylaminopyridine), using a condensing agent (*e*.*g*., 1,3-dicyclohexyl carbodiimide (DCC), 1-ethyl-3-[3-(ditnethylamino)propyl] carbodiimide (EDC), 1,1'-carbonyldiimidazole (CDI), 2-chloro-1-methylpyridinium iodide, or 1-propanephosphonie acid cyclic anhydride (PPA)), in the presence or absence of 1-hydroxybenzotiazole (HOBt), at about 0 to 40°C.

These reactions (1), (2) and (3) are all preferably carried out under the anhydrous condition in the presence of inert gases *(e.g.* argon, nitrogen *etc.).*

The deprotection reaction of the protecting group can be carried out in the same manner as described above.

Among the compounds of the present invention represented by formula (I), the compounds except above described ones, the compounds as the other starting materials or reagents are known in themselves, or can be easily prepared by known method. For example, they can be produced, for example, by appropriately improving or combining the methods described in Examples, methods described in *Comprehensive Organic Transformations : A Guide to Functional Group Preparations,* 2nd Edition (Richard C. Larock, John Wiley & Sons Inc, 1999) and the like.

In each reaction in the present specification, solid-phase supported reagent accordingly supported to macromolecule polymer (*e*.*g*. polystyrene, polyacrylamide, polypropylene, polyethyleneglycol *etc.)* may be used.

In each reaction in the present specification, reaction products may be purified in an ordinary manner, for example, through normal-pressure or reduced-pressure distillation, or through high-performance liquid chromatography with silica gel or magnesium silicate, thin-layer chromatography, ion-exchange resin, scavenger resin or column chromatography, or through washing or recrystallization and so on. The purification may be effected in each reaction stage or after some reaction stages.

In each reaction in the present specification, as will be understood by those skilled in the art, the reaction with heating can be performed using water bath, oil bath, sand bath or microwave.

### Pharmacological Activity:

As pharmacological test other than ones described in Example, particularly in vivo measurement using animals, for example, the following method is shown. The hypoglycemic effect and hypolipidemic effect of the compound of the present invention can be measured by methods as follows.

### Hypoglycemic effect and hypolipidemic effect (1):

Body weight of KKAy/Ta Jcl mice and blood sugar level therein were measured and the mice were grouped using the blood sugar level as an index and assigned. During six days starting from the next day, breeding was conducted using a feed or a powdery feed containing the compound of the present invention. After the repeated administration, body weight and amount of ingested feed were measured and the administered dose was calculated from an average ingested feed amount. Further, in addition to blood sugar level and TG level in plasma, measurement was also conducted for insulin and free fatty acid (NEFA) in blood, GOT and GPT.

It is suggested that the lowering action for blood sugar, insulin in blood, NEFA level or TG level in plasma in the well-fed KKAy/Ta mice has the possibility as an agent for preventive and/or treating for diabetes, hyperlipemia, arteriosclerosis, *etc.*

### Hypoglycemic effect and hypolipidemic effect (2)

Body weight and levels of blood sugar, NEFA, TG and HbAlc were measured in Zucker fa/fa rats (strain name: Crj-[ZUC]-fa/fa) and in lean rats which are normal control animal (strain name: Crj-[ZUC]-lean). The rats were grouped using HbAlc level and body weight as indexes and, as from the next day, the compound of the present invention was repeatedly administered *per os.* Incidentally, in the control group, a medium was administered.

After starting the repeated administration, an average ingested feed amount was calculated and levels of blood sugar, NEFA, TG and HbAlc were measured. An oral glucose tolerance test (OGTT) was also carried out and an improving action for glucose tolerance was evaluated. In the OGTT, the rats were fasted from the day before the test and, on the next day, 2 g/5mL/kg of a glucose solution was loaded to the rats and then 60 and 120 minutes after loading, blood sugar and blood insulin, NEFA, TG and HbAlc levels and wet liver weight were measured.

It is suggested that the lowering action for blood sugar, insulin in blood, NEFA, HbAlc level or TG level in plasma in the well-fed Zucker fa/fa rats has the possibility as a preventive and/or treating agent for diabetes, hyperlipemia, arteriosclerosis, *etc*. In addition, it is suggested that action of lowering of blood sugar level upon hunger and action of improving the glucose tolerance in OGTT have the possibility as a preventive and/or treating agent for diabetes.

### Hypoglycemic effect and hypolipidemic effect (3):

Cynamolgus monkeys were further subjected to an institutional quarantine and an acclimation in an institute where the test will be carried out. Body weight of the animals was measured, grouping was conducted and a medium or a drug solution containing 3 to 100 mg/kg/day of the compound of the present invention was repeatedly administered into stomach via nose once daily using a nutrition catheter and injection syringe. After the administration, blood was collected and subjected to the above-described hematological tests (measurements of red blood cell count, hematocrit, hemoglobin content, platelet count and white blood cell count) and hematochmical tests (measurements of GOT, GPT, alkaline phosphatase, total protein, nitrogen in urine, creatinine, creatinine kinase, total bilirubin, blood sugar, TC, HDL, LDL and TG) were conducted. Further, blood was collected before starting the administration of the compound of the present invention, after 1, 2 and 4 hour(s) from administration on the 14th day since the start of the administration and after 1, 2 and 3 hour(s) from feeding (ingested for 1 hour) and blood sugar, TC, HDL, LDL and TG were measured.

It is suggested that the lowering effect for plasma TG level, TC level and LDL level in fasted normal cynomolgus monkeys has the possibility as a preventive and/or treating agent for hyperlipemia, arteriosclerosis, *etc*. That is also confirmed by the fact that a rise in TG after loading with a feed is suppressed. Further, the fact that a rise in blood sugar after loading with a feed is suppressed suggests the possibility as a preventive and/or treating agent for diabetes. Moreover, the fact whether the toxicity change is available is also able to be evaluated from other hemato-biochemical parameters.

### Toxicity:

Toxicity of the compound of the present invention represented by formula (I) is very low, and it is safe enough to use as a pharmaceutical agent.

### Applications to pharmaceuticals:

The compound of the present invention represented by formula (I) or a salt thereof has a PPARδ agonistic action and has, for example, a raising action for HDL cholesterol, an increasing action for LDL clearance, promoting actions for carrying-out and for reverse transfer of lipid (particularly, cholesterol), a suppressive action for foaming of macrophage and an inhibitory action for biosynthesis of cholesterol whereby its application as a preventive and/or treating agent for, for example, diseases where sugar and lipid metabolisms are abnormal [such as diabetes, hyperlipemia (such as hypercholesterolemia, hypo-HDL-emia, hyper-LDL-emia and hypertriglyceridemia), arteriosclerosis, cardiovascular diseases, obesity and metabolic syndrome], hypertension, circulatory diseases and skin inflammation diseases are/is expected.

The compound represented by formula (I) or the salts thereof may be administered in combination with other drugs for the purpose of (1) complement and/or enhancement of preventing and/or treating effect of the compound, (2) improvement of dynamics and absorption of the compound, and lowering of dose, and/or (3) alleviation of side effect of the compound.

The compound represented by formula (I) or the salts thereof and other pharmaceutical preparations may be administered in the form of formulation having these components incorporated in one preparation or may be administered in separate preparations. In the case where these pharmaceutical preparations are administered in separate preparations, they may be administered simultaneously or at different times. In the latter case, the compound represented by formula (I) or the salts thereof may be administered before the other pharmaceutical preparations. Alternatively, the other pharmaceutical preparations may be administered before the compound represented by formula (I) or the salts thereof The method for the administration of these pharmaceutical preparations may be the same or different.

Other drug may be a low-molecular compound or may be a high-molecular protein, polypeptide, polynucleotide (DNA, RNA and gene), antisense, decoy, antibody, vaccine, *etc*. Dose of the other drug is able to be appropriately selected using the clinically used dose as a standard. Compounding ratio of the compound of the present invention to the other drug may be appropriately selected in view of age and body weight of the person to be administered, administering method, administering time, object disease, symptom, combination, *etc*. For example, 0.01 to 100 parts by weight of other drug may be used to 1 part by weight of the compound of the present invention. Other drug may be administered by combining any two or more thereof in an appropriate ratio.

The diseases on which the preventive and/or treatment effect of the above-mentioned combined preparations works are not specifically limited but may be those for which the preventive and/or treatment effect of the compound represented by formula (I), a salt thereof, a solvate thereof or a prodrug thereof is compensated for and/or enhanced.

As other drugs to compensate and/or enhance for hypolipidemic effect of the compound represented by formula (I) or a salt thereof, i.e. lipid improvement agents, they include, for example, MTP (Microsomal Triglyceride Transfer Protein) inhibitor, HMG-CoA reductase inhibitor, squalene synthase inhibitor, fibrate (fibric acid derivative), ACAT (acyl CoA: Cholesterol 0-acyltransferase) inhibitor, 5-lipoxygenase inhibitor, cholesterol absorption inhibitor, bile acid absorption inhibitor, ileal Na⁺/bile acid transporter (IBAT) inhibitor, LDL receptor activator/expression enhancer, pancreatic lipase inhibitor, probucol formulation, nicotine acid formulation, cholesterol ester transport protein (CETP) inhibitor, other anti-hypercholesterolemia therapeutic agent and so on.

Examples of MTP inhibitor include BMS-201038, BMS-212122, BMS-200150, GW-328713, R-103757 and so on. Examples of HMG-CoA reductase inhibitor include atorvastatin, fulvastatin, lovastatin, pitavastatin, pravastatin, rosuvastatin, simvastatin and so on. Examples of ACAT inhibitor include F-12511, F-1394, CI-1011, melinamide and so on. Examples of squalene synthase inhibitor include TAK-475 and so on. Examples of fibrate include gemfibrozil, clofibrate, bezafibrate, fenofibrate, clinofibrate, simfibrate and so on. Examples of ACAT inhibitor include Cl-1101, FCE27677, RP73163 and so on. Examples of cholesterol absorption inhibitor include ezetimibe, soysterol and so on. Examples of bile acid absorption inhibitor include cholestyramine, colesevelam, colestimide and so on. Examples of LDL receptor activator/expression enhancer include MD-700, LY295427 and so on. Examples of pancreatic lipase inhibitor include orlistat and so on. It is known that there are sometimes associated with rhabdomyolysis in case of a combination of fibrate and HMG-CoA reductase inhibitor and this combination are contraindicated in renal failure patients and patients with impaired renal function. In the above-mentioned combination of the compound of the present invention, a salt thereof, a solvate thereof or a prodrug thereof with the above lipid improvement agent, there is possibility to correct abnormal lipid metabolism without developing rhabdomyolysis. As combination drugs with the compound of the present invention, a salt thereof, a solvate thereof or a prodrug thereof, preferred are HMG-CoA reductase inhibitor, fibrate agent (fibrin acid derivatives), cholesterol absorption inhibitor, bile acid absorption inhibitor, pancreatic lipase inhibitor and nicotine acid formulation.

As other drugs to compensate and/or enhance for hypoglycemic effect of the compound represented by formula (I), and to enhance effect of the treatment of complication of diabetes, i.e. therapeutic agents for diabetes, they include, for example, sulfonylurea type hypoglycemic agent, biguanide preparation, α-glucosidase inhibitor, fast-acting insulin secretion accelerator, insulin preparation, DPP (dipeptidyl peptidase) 4 inhibitor, GLP-1 agonist, β3 adrenaline receptor activator, other therapeutic agents for diabetes, therapeutic agents for complication of diabetes and so on.

Examples of sulfonylurea type hypoglycemic agents include acetohexamide, glibenclamide, gliclazide, glyclopyramide, chlorpropamide, tolazamide, tolbutamide, glimepiride and so on. Examples of biguanide preparations include buformin hydrochloride, metformin hydrochloride and so on. Examples of α-glucosidase inhibitors include acarbose, voglibose and so on. Examples of fast-acting insulin secretion accelerators include nateglinide, repaglinide and so on. Examples of DPP 4 inhibitor include NVP-DPP728A and so on. Examples of GLP-1 agonist includes exendin-4. Examples of β3 adrenaline receptor activators include AJ-9677, BMS-210285, CP-331679, KUL-1248, LY-362884, L-750335, CP331648 and so on. Examples of therapeutic agents for complication of diabetes include epalrestat, zenarestat, fidarestat, zopolrestat, AS-3201, SG-210 and so on.

As other drugs to compensate and/or enhance for anti-adiposity effect of the compound represented by formula (I), a salt thereof, a solvate thereof or a prodrug thereof, i.e., anti-adiposity agent, they include, for example, appetite suppressing agent, pancreatic lipase inhibitor, β3 adrenaline receptor activator, serotonin norepinephrine dopamine reuptake inhibitor and so on. Examples of appetite suppressing agent include leptin, mazindol, amphetamine, methamphetamine and so on. Examples of pancreatic lipase inhibitor include orlistat and so on. Examples of β3 adrenaline receptor activator include AJ-9677, BMS-210285, CP-331679, KUL-1248, LY-362884, L-750335, CP-331648 and so on. Examples of serotonin norepinephrine dopamine reuptake inhibitor include sibutramine and so on.

The weight proportion of the compound represented by formula (I), a salt thereof, a solvate thereof or a prodrug thereof and the other drugs is not specifically limited.

Arbitrary two or more of the other drugs may be administered in combination. Examples of the other pharmaceutical preparations for compensating for and/or enhancing the preventive and/or treatment effect of the compound represented by formula (I), a salt thereof, a solvate thereof or a prodrug thereof include not only those which have so far been found but also those which will be found on the basis of the above-mentioned mechanism.

In order to use the compound of the present invention represented by formula (I), a salt thereof, a solvate thereof or a prodrug thereof, or the compound represented by formula (I), a salt thereof, a solvate thereof or a prodrug thereof in combination with the other pharmaceutical preparations, these compounds are normally administered to the entire of human body or topically orally or parenterally.

The dose of these compounds depends on the age, weight and symptom of the patient, the remedial value, the administration method, the treatment time, *etc.* In practice, however, these compounds are administered orally once or several times per day each in an amount of from 1 mg to 1000 mg per adult, parenterally once or several times per day each in an amount of from 1 mg to 100 mg per adult or continuously administered into vein for 1 hour to 24 hours per day.

It goes without saying that the dose of these compounds may be less than the above-mentioned value or may need to exceed the above-mentioned range because the dose varies under various conditions as mentioned above.

When the compound of the present invention represented by formula (I), a salt thereof, a solvate thereof or a prodrug thereof, or the compound represented by formula (I), a salt thereof, a solvate thereof or a prodrug thereof is administered in combination with the other pharmaceutical preparations, they are used in the form of solid or liquid agent for oral administration, injection, agent for external application, suppository, eye drops or inhalant for parenteral administration or the like.

Examples of the solid agent for oral administration include tablet, pill, capsule, powder, and pellet. Examples of the capsule include hard capsule, and soft capsule.

In such a solid agent for internal application, one or more active materials are used in the form of preparation produced by an ordinary method singly or in admixture with a vehicle (*e.g.*, lactose, mannitol, glucose, microcrystalline cellulose, starch *etc*.), binder (*e.g.*, hydroxypropyl cellulose, polyvinyl pyrrolidone, magnesium metasilicoaluminate *etc*.), disintegrant (*e.g.*, calcium fibrinoglycolate *etc*.), glidant (*e*.*g*., magnesium stearate *etc*.), stabilizer, dissolution aid (*e*.*g*., glutamic acid, aspartic acid *etc*.) or the like. The solid agent may be coated with a coating agent (*e*.*g*., white sugar, gelatin, hydroxypropyl cellulose, hydroxypropyl methyl cellulose phthalate *etc.)* or two or more layers. Alternatively, the solid agent may be capsulized by an absorbable material such as gelatin.

Examples of the liquid agent for oral administration include pharmaceutically acceptable aqueous solution, suspension, emulsion, syrup, and elixir. In such a liquid agent, one or more active agents are dissolved, suspended or emulsified in a commonly used diluent (*e*.*g*., purified water, ethanol, mixture thereof *etc*.), Furthermore, such a liquid agent may comprise a wetting agent, a suspending agent, an emulsifier, a sweetening agent, a flavor, a preservative, a buffer, *etc*.

The agent for parenteral administration may be in the form of, e.g., ointment, gel, cream, wet compress, paste, liniment, nebula, inhalant, spray, aerosol, eye drops, collunarium or the like. These agents each contain one or more active materials and are prepared by any known method or commonly used formulation.

The ointment is prepared by any known or commonly used formulation. For example, one or more active materials are triturated or dissolved in a base to prepare such an ointment. The ointment base is selected from known or commonly used materials. In some detail, higher aliphatic acid or higher aliphatic acid ester (*e*.*g*., adipic acid, myristic acid, palmitic acid, stearic acid, oleic acid, adipic acid ester, myristic acid ester, palmitic acid ester, stearic acid ester, oleic acid ester *etc.),* wax (*e.g.*, beeswax, whale wax, ceresin *etc*.), surface active agent *(e.g.,* polyoxyethylenealkylether phosphoric acid ester *etc.),* higher alcohol (e.g., cetanol, stearyl alcohol, setostearyl alcohol *etc.),* silicon oil *(e.g.,* dimethyl polysiloxane *etc.),* hydrocarbon *(e.g.,* hydrophilic petrolatum, white petrolatum, purified lanolin, liquid paraffin *etc*.), glycol *(e.g.,* ethylene glycol, diethylene glycol, propylene glycol, polyethylene glycol, macrogol *etc*,), vegetable oil *(e.g.,* castor oil, olive oil, sesame oil, turpentine oil), animal oil (mink oil, vitelline oil, squalane, squalene), water, absorption accelerator and rash preventive may be used singly or in admixture of two or more thereof. The base may further comprise a humectant, a preservative, a stabilizer, an antioxidant, a perfume, *etc.*

The gel is prepared by any known or commonly used formulation. For example, one or more active materials are dissolved in a base to prepare such a gel. The gel base is selected from known or commonly used materials. For example, lower alcohol (*e.g.,* ethanol, isopropyl alcohol *etc*.), gelling agent (*e.g.,* carboxymethyl cellulose, hydroxyethyl cellulose, hydroxypropyl cellulose, ethyl cellulose *etc.*), neutralizing agent (*e*.*g*., triethanolamine, diisopropanolamine *etc*.), surface active agent (*e.g.*, polyethylene glycol monostearate *etc*.), gums, water, absorption accelerator, and rash preventive are used singly or in admixture of two or more thereof. The gel base may further comprise a preservative, an antioxidant, a perfume, *etc.*

The cream is prepared by any known or commonly used formulation. For example, one or more active materials are dissolved or emulsified in a base to prepare such a cream. The cream base is selected from known or commonly used materials. For example, higher aliphatic acid ester, lower alcohol, hydrocarbon, polyvalent alcohol (*e.g*., propylene glycol, 1,3-butylene glycol *etc*.), higher alcohol (*e.g.*, 2-hexyl decanol, cetanol *etc*.), emulsifier (*e*.*g*., polyoxyethylene alkyl ethers, aliphatic acid esters *etc*.), water, absorption accelerator, and rash preventive are used singly or in admixture of two or more thereof. The cream base may further comprise a preservative, an antioxidant, a perfume, *etc.*

The wet compress is prepared by any known or commonly used formulation. For example, one or more active materials are dissolved in a base to prepare a kneaded mixture which is then spread over a support to prepare such a wet compress. The wet compress base is selected from known or commonly used materials. For example, thickening agent (*e*.*g*., polyacrylic acid, polyvinyl pyrrolidone, gum arabic, starch, gelatin, methyl cellulose *etc*.), wetting agent (*e.g.*, urea, glycerin, propylene glycol *etc*.), filler (*e*.*g*., kaolin, zinc oxide, talc, calcium, magnesium *etc*.), water, dissolution aid, tackifier, and rash preventive may be used singly or in admixture of two or more thereof The wet compress base may further comprise a preservative, an antioxidant, a perfume, *etc*.

The pasting agent is prepared by any known or commonly used formulation. For example, one or more active materials are dissolved in a base to prepare a kneaded mixture which is then spread over a support to prepare such a pasting agent. The pasting agent base is selected from known or commonly used materials. For example, polymer base, fat and oil, higher aliphatic acid, tackifier and rash preventive may be used singly or in admixture of two or more thereof. The pasting agent base may further comprise a preservative, an antioxidant, a perfume, *etc.*

The liniment is prepared by any known or commonly used formulation. For example, one or more active materials are dissolved, suspended or emulsified in water, alcohol (*e*.*g*., ethanol, polyethylene glycol *etc.*)*,* higher aliphatic acid, glycerin, soap, emulsifier, suspending agent, *etc.,* singly or in combination of two or more thereof, to prepare such a liniment. The liniment may further comprise a preservative, an antioxidant, a perfume, *etc.*

The nebula, inhalant, spray and aerozol each may comprise a commonly used diluent, additionally, a stabilizer such as sodium hydrogen sulfite and a buffer capable of providing isotonicity such as isotonic agent (*e.g.*, sodium chloride, sodium citrate, or citric acid *etc.).* For the process for the preparation of spray, reference can be made to US Patents 2,868,691 and 3,095,355.

The injection for parenteral administration consists of solid injection used to be dissolved or suspended in the form of solution, suspension, emulsion and a solvent to be dissolved before use. The injection is prepared by dissolving, suspending or emulsifying one or more active materials in a solvent. As such a solvent there may be used distilled water for injection, physiological saline, vegetable oil, alcohol such as propylene glycol, polyethylene glycol and ethanol, *etc*., singly or in combination thereof The injection may further comprise a stabilizer, a dissolution aid (e.g., glutamic acid, aspartic acid, Polysolvate 80 (trade name) *etc.),* a suspending agent, an emulsifier, a soothing agent, a buffer, a preservative, *etc*. The injection is sterilized at the final step or prepared by an aseptic process. Alternatively, an aseptic solid agent such as freeze-dried product which has previously been prepared may be rendered aseptic or dissolved in an aseptic distilled water for injection or other solvents before use.

The inhalant for parenteral administration may be in the form of aerosol, powder for inhalation or liquid for inhalation. The liquid for inhalation may be dissolved or suspended in water or other proper medium in use.

These inhalants are prepared by a known method.

For example, the liquid for inhalation is prepared from materials properly selected from preservatives (*e.g.*, benzalconium chloride, Paraben *etc*.), colorants, buffering agents (*e.g.*, sodium phosphate, sodium acetate *etc*.), isotonic agents (*e.g.*, sodium chloride, concentrated glycerin *etc*.), thickening agents (*e.g.*, carboxyvinyl polymer *etc*.), absorption accelerators, *etc*. as necessary.

The powder for inhalation is prepared from materials properly selected from glidants (*e.g.*, stearic acid and salt thereof *etc*.), binders (*e*.*g*., starch, dextrin *etc*.), vehicles (*e*.*g*., lactose, cellulose *etc*.), colorants, preservatives (*e.g.*, benzalconium chloride, Paraben *etc.*), absorption accelerators, *etc*., if necessary.

In order to administer the liquid for inhalation, a sprayer (*e.g*., atomizer, nebulizer *etc*.) is normally used. In order to administer the powder for inhalation, a powder inhaler is normally used.

Other examples of the composition for parenteral administration include suppository for rectal administration and pessary for vaginal administration prepared by an ordinary formulation comprising one or more active materials.

### Best Mode for Carrying Out the Invention

The present invention is explained below in detail based on Examples, however, the present invention is not limited thereto.

The solvents in parentheses at chromatographic separations section and TLC section show the developing or eluting solvents and the ratios of the solvents used are indicated by volume. NMR is the measurement of ¹H NMR and the solvents in parentheses indicated in NMR section show solvents used in determination.

All compounds described in the specification are named by using of ACD/Name (Trade mark, Advanced Chemistry Development Inc.) or ACD/Name (Trade mark, Advanced Chemistry Development Inc.) batch which is the computer program to name according to IUPAC rule.

### Example 1

methyl 3-[3-(methoxymethoxy)phenoxy]benzoate:

Molecular sieves (38 mg) and copper acetate (200 mg) were added to a solution of [3-(methoxymethoxy)phenyl]boronic acid (597 mg) and methyl 3-hydroxymethylbenzoate (152 mg) in dichloromethane (12 mL) and stirred at room temperature for one night. After ethyl acetate was added to the reaction mixture, it was filtered using Celite (trade name). The filtrate was successively washed with a mixed solvent (saturated aqueous solution of sodium hydrogen carbonate : saturated aqueous ammonia = 4:1), water and a saturated saline solution, dried over anhydrous sodium sulfate and concentrated and the resulting residue was purified by a silica gel column chromatography (hexane : ethyl acetate = 8:1) to give the title compound (320 mg) having the following properties.
TLC: Rf 0.71 (hexane:ethyl acetate = 2:1).

### Example 2

methyl 3-(3-hydroxyphenoxy)benzoate:

The compound (320 mg) produced in Example 1 was dissolved in 4N hydrogen chloride/dioxane solution (8 mL) and stirred at room temperature for 20 minutes. The reaction mixture was concentrated and the resulting residue was purified by a silica gel thin layer chromatography (hexane : ethyl acetate = 2:1) to give the title compound (200 mg) having the following properties.
TLC: Rf 0.49 (hexane:ethyl acetate = 2:1);
NMR(CDCl₃) δ 7.78, 7.67, 7.41, 7.22, 7.19, 6.60, 6.57, 6.50, 5.18, 3.90.

### Example 3

methyl 3-[3-({(2E)-3-[4-(trifluoromethyl)phenyl]-2-propen-1-yl}oxy)phenoxy]benzoate:

Diisopropyl ethylamine (0.12 mL) and potassium carbonate (117 mg) were added to a solution of the compound (188 mg) produced in Example 2 and 1-[(1E)-3-bromo-1-propen-1-yl]-4-(trifluoromethyl)benzene (204 mg) in dimethylformamide (3 mL) and stirred at room temperature for 2.5 hours. Water was added to the reaction mixture and extraction with a mixed solvent (hexane : ethyl acetate = 1:2) was carried out. The organic layer was successively washed with water and a saturated saline solution, dried over anhydrous magnesium sulfate and concentrated. The resulting residue was purified by a silica gel column chromatography (hexane : ethyl acetate = 8:1) to give the title compound (236 mg) having the following properties.
TLC: Rf 0.70 (hexane:ethyl acetate = 2:1);
NMR(CDCl₃) δ 7.79, 7.67, 7.58, 7.49, 7.40, 7.30-7.21, 6.81-6.71, 6.66-6.61, 6.48, 4.70, 3.89.

### Example 4

3-[3-({(2E)-3-[4-(trifluoromethyl)phenyl]-2-propen-1-yl}oxy)phenoxy]benzoic acid:

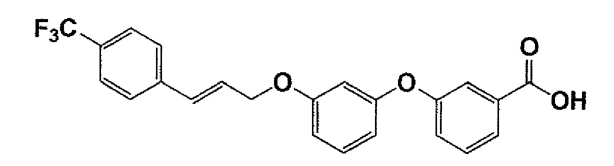

A 2N aqueous solution (1.1 mL) of sodium hydroxide and methanol (4.4 mL) were added to a solution of the compound (196 mg) produced in Example 3 in methanol (2.2 mL) and stirred at 60°C for 2 hours. The reaction mixture was cooled, acidified with 2N hydrochloric acid and extracted with ethyl acetate. The organic layer was successively washed with water and a saturated saline solution, dried over anhydrous magnesium sulfate and concentrated. The resulting curde crystal was washed with a mixed solvent (hexane : ethyl acetate = 4: 1), filtered and dried to give the title compound (150 mg) having the following properties.
TLC: Rf 0.54 (dichloromethane:methanol = 19:1);
NMR (DMSO-d₆) δ 4.76, 6.75-6.60, 6.89-6.80, 7.38-7.27, 7.44, 7.50, 7.74-7.65, 13.12.

### Example 5(1) to 5 (33)

The following compounds of the present invention were obtained by carrying out the procedures for similar objects in Example 3→Example 4 using a corresponding alcohol derivative instead of the compound produced in Example 2 and, if necessary, converting it to a corresponding salt by a known method.

### Example 5 (1)

4-[4-({(2E)-3-[4-(trifluoromethyl)phenyl]-2-propen-1-yl}oxy)phenoxy]benzoic acid: 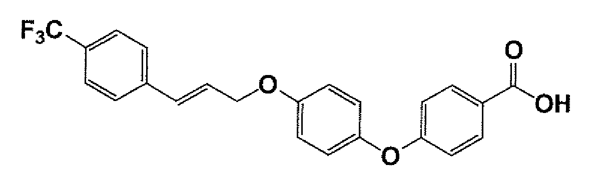 TLC: Rf 0.33 (dichloromethane:methanol = 19:1);
NMR (DMSO-d₆) δ 4.78, 6.70, 6.87, 6.95, 7.13-7.05, 7.75-7.66, 7.91, 12.75.

### Example 5 (2)

{3-[3-({(2E)-3-[4-(trifluoromethyl)phenyl]-2-propen-1-yl}oxy)phenoxy]phenyl} acetic acid:
TLC: Rf 0.42 (dichloromethane:methanol = 19:1);
NMR (DMSO-d₆) δ 3.55, 4.74, 6.56, 6.73-6.60, 6.83-6.75, 6.88, 6.94, 7.03, 7.34- 7.24, 7.69, 12.36.

### Example 5 (3)

{4-[4-({(2E)-3-[4-(trifluoromethyl)phenyl]-2-propen-1-yl}oxy)phenoxy]phenyl} acetic acid:
TLC: : Rf 0.49 (dichloromethane:methanol =19:1);
NMR (DMSO-d₆) δ 3.52, 4.75, 6.69, 6.86, 6.91-6.84, 7.01-6.95, 7,06-7.00, 7.21, 12.29.

### Example 5 (4)

4-fluoro-3-[3-({(2E)-3-[4-(trifluoromethyl)phenyl]-2-propen-1-yl}oxy)phenoxy]benzoic acid:
TLC: Rf 0.35 (dichloromethane: methanol = 19:1);
NMR (DMSO-d₆) δ 4.76, 6.75-6.58, 6.88-6.80, 7.32, 7.50, 7.56, 7.72-7:66, 7.77, 13.17.

### Example 5 (5)

2-methyl-3-[3-({(2E)-3-[4-(trifluoromethyl)phenyl]-2-propen-1-yl}oxy)phenoxy]benzoic acid:
TLC: Rf 0.37 (dichloromethane:methanol = 19:1);
NMR (DMSO-d₆) δ 2.34, 4.73, 6.42, 6.53, 6.65, 6.74, 6.82, 7.13, 7.26, 7.29, 7.62, 7.68, 13.04.

### Example 5 (6)

4-methyl-3-[3-({(2E)-3-[4-(trifluoromethyl)phenyl]-2-propen-1-yl}oxy)phenoxy]benzoic acid:
TLC: Rf 0.43 (dichloromethane:methanol = 19:1);
NMR (DMSO-d₆) δ 2.25, 4.75, 6.52, 6.71-6.59, 6.87-6.77, 7.29, 7.34, 7.42, 7.65, 7.68, 12.96.

### Example 5 (7)

4-[3-({(2E)-3-[4-(trifluoromethyl)phenyl]-2-propen-1-yl}oxy)phenoxy]benzoic acid:
TLC: Rf 0.39 (dichloromethane:methanol = 19:1);
NMR (DMSO-d₆) δ 4.77, 6.73-6.50, 6.76, 6.91-6.79, 7.03, 7.35, 7.68, 7.92, 12.81.

### Example 5 (8)

{4-methoxy-3-[3-({(2E)-3-[4-(trifluoromethyl)phenyl]-2-propen-1-yl}oxy)phenoxy]phcnyl} acetic acid:
TLC: Rf0.37 (dichloromethane:methanol = 19:1);
NMR (DMSO-d₆) δ 3.49, 3.70, 4.71, 6.39, 6.46, 6.69-6.59, 6.81, 6.95, 7.10-7.05, 7.20, 7.69, 12.26.

### Example 5 (9)

{4-[3-({(2E)-3-[4-(trifluoromethyl)phenyl]-2-propen-1-yl}oxy)phenoxy]phenyl}acetic acid:
TLC: Rf 0.37 (dichloromethane:methanol = 19:1);
NMR (DMSO-d₆) δ 3.54, 4.74, 6.54, 6.71-6.60, 6.86-6.76, 6.96, 7.31-7.20, 7.68, 12.32.

### Example 5 (10)

{2-[3-({(2E)-3-[4-(trifluoromethyl)phenyl]-2-propen-1-yl}oxy)phenoxy]phenyl}acetic acid:
TLC: Rf 0.42 (dichloromethane: methanol = 19:1);
NMR (DMSO-d₆) δ 3.56, 4.72, 6.50, 6.55, 6.65, 6.75, 6.90 -6.77, 7.11, 7.27-7.20, 7.35, 7.68, 12.26.

### Example 5 (11)

{4-methyl-3-[3-({(2E)-3-[4-(trifluoromethyl)phenyl]-2-propen-1-yl}oxy)phenoxy]phenyl}acetic acid sodium salt:
TLC: Rf 0.23 (ethyl acetate);
NMR. (DMSO-d₆) δ 2.06, 3.14, 4.72, 6.43-6.48, 6.60-6.69, 6.82, 6.96, 7.10, 7.21, 7. 68.

### Example 5 (12)

{3-methyl-5-[3-({(2E)-3-[4-(trifluoromethyl)phenyl]-2-propen-1-yl}oxy)phenoxy]phenyl}acetic acid:
TLC: Rf 0.24 (ethyl acetate);
NMR(CDCl₃) δ 2.28, 3.55, 4.67, 6.46, 6.59-6.62, 6.68-6.76, 6.83, 7.20-7.23, 7.47, 7.57.

### Example 5 (13)

{2-methyl-3-[3-({(2E)-3-[4-(trifluoromethyl)phenyl]-2-propen-1-yl}oxy)phenoxy]phenyl}acetic acid:
TLC: Rf 0.31 (dichloromethane:methanol = 19:1);
NMR (DMSO-d₆) δ 2.06, 3.65, 4.72, 6.41, 6.48, 6.74-6.59, 6.87-6.76, 7.06, 7.14, 7.24, 7.68, 12.36.

### Example 5 (14)

{4-chloro-3-[3-({(2E)-3-[4-(trifluoromethyl)phenyl]-2-propen-1-yl}oxy)phenoxy]phenyl}acetic acid:
TLC: Rf 0.43 (dichloromethane:methanol = 19:1);
NMR (DMSO-d₆) δ 3.56, 4.74, 6.49, 6.58, 6.66, 6.87-6.75, 7.05, 7.11, 7.28, 7.51, 7.69, 12.39.

### Example 5 (15)

{2-chloro-4-methyl-3-[3-({(2E)-3-[4-(trifluoromethyl)phenyl]-2-propen-1-yl}oxy)phenoxy]phenyl}acetic acid:
TLC: Rf 0.48 (dichloromethane:methanol = 19:1);
NMR (DMSO-d₆) δ 2.08, 3.71, 4.71, 6.31, 6.37, 6.72-6.58, 6.80, 7.28-7.18, 7.68, 12.44.

### Example 5 (16)

(2E)-3-{3-[3-({(2E)-3-[4-(trifluoromethyl)phenyl]-2-propen-1-yl}oxy)phenoxy]phenyl}arcrylic acid:
TLC: Rf 0.41 (dichloromethane:methanol = 19:1);
NMR (DMSO-d₆) δ 4.75, 6.51, 6.58, 6.71-6.59, 6.87-6.78, 7.03, 7.30, 7.43-7.36, 7.47, 7.56, 7.68, 12.42.

### Example 5 (17)

3-{3-[3-({(2E)-3-[4-(trifluoromethyl)phenyl]-2-propen-1-yl}oxy)phenoxy]phenyl}propanic acid:
TLC: Rf 0.50 (dichloromethane:methanol = 19:1);
NMR (DMSO-d₆) δ 2.6-2.48, 2.79, 4.74, 6.54, 6.72-6.60, 6.87-6.74, 6.91, 7.00, 7.31 -7.23, 7.70, 12.11.

### Example 5 (18)

{2-fluoro-5-[3-({(2E)-3-[4-(trifluoromethyl)phenyl]-2-propen-1-yl}oxy)phenoxy]phenyl}acetic acid sodium salt:
TLC: Rf 0.38 (chloroform:methanol = 9: 1);
NMR (DMSO-d₆) δ 3.19, 4.73, 6.85-6.49, 7.05-6.99, 7.25, 7.66.

### Example 5 (19)

{4-fluoro-3-[3-({(2E)-3-[4-(trifluoromethyl)phenyl]-2-propen-1-yl}oxy)phenoxy]phenyl}acetic acid sodium salt:
TLC: Rf 0.43 (chloroform:methanol = 9:1);
NMR (DMSO-d₆) δ 3.19, 4.73, 6.49, 6.85-6.58, 7.27-7.03, 7.68.

### Example 5 (20)

{3-[4-({(2E)-3-[4-(trifluoromethyl)phenyl]-2-propen-1-yl}oxy)phenoxy]phenyl}acetic acid:
TLC: Rf 0.27 (dichloromethane:methanol = 19:1);
NMR (DMSO-d₆) δ 3.53, 4.75, 6.69, 6.78, 6.90-6.80, 6.97, 7.07-6.97, 7.26, 7.75-7.65, 12.31.

### Example 5 (21)

{2-[4-({(2E)-3-[4-(trifluoromethyl)phenyl]-2-propen-1-yl}oxy)phenoxy]phenyl}acetic acid:
TLC: Rf0.33 (dichloromethane:methanol = 19:1);
NMR (DMSO-d₆) δ 3.60, 4.74, 6.74-6.64, 6.95-6.82, 7.07-6.97, 7.21, 7.31, 7.70, 12.23.

### Example 5 (22)

{3-[2-methyl-5-({(2E)-3-[4-(trifluoromethyl)phenyl]-2-propen-1-yl}oxy)phenoxy]phenyl}acetic acid:
TLC: Rf 0.63 (dichloromethane:methanol = 19: 1);
NMR (DMSO-d₆) δ 2.07, 3.53, 4.68, 6.53, 6.62, 6.86-6.72, 6.96, 7.22, 7.24, 7.67, 12.32.

### Example 5 (23)

{3-methoxy-5-[3-({(2E)-3-[4-(trifluoromethyl)phenyl]-2-propen-1-yl}oxy)phenoxy]phenyl}acetic acid sodium salt:
TLC: Rf 0.38 (chloroform:methanol = 9:1);
NMR (DMSO-d₆) δ 3.09, 3.67, 4.74, 6.32, 6.45, 6.86-6.64, 7.26, 7.69.

### Example 5 (24)

{2-(trifluoromethyl)-5-[3-({(2E)-3-[4-(trifluoromethyl)phenyl]-2-propen-1-yl}oxy)phenoxy]phenyl} acetic acid:
TLC: Rf 0.36 (dichloromethane:methanol =19:1);
NMR (DMSO-d₆) δ 3.74, 4.77, 6.72-6.62, 6.92-6.76, 6.97, 7.16, 7.37, 7.62, 7.69, 12.51.

### Example 5 (25)

{3-[2-({(2E)-3-[4-(trifluoromethyl)phenyl]-2-propen-1-yl}oxy)phenoxy]phenyl}acetic acid:
TLC: Rf 0.54 (chloroform:methanol = 9:1);
NMR(CDCl₃) δ 3.57, 4.70, 6.31, 6.50, 6.89-6.85, 7.00-6.94, 7.05, 7.16-7.11, 7.25, 7.35, 7.52.

### Example 5 (26)

{2-methyl-3-[2-methyl-5-({(2E)-3-[4-(trifluoromethyl)phenyl]-2-propen-1-yl}oxy)phenoxy]phenyl}acetic acid:
TLC: Rf 0.43 (dichloromethane: methanol = 19: 1);
NMR (DMSO-d₆) δ 2.09, 2.14, 3.64, 4.63, 6.23, 6.58, 6.72-6.63, 6.74, 6.99, 7.07, 7.19, 7.73-7.60, 12.38.

### Example 5 (27)

{4-methyl-3-[2-methyl-5-({(2E)-3-[4-(trifluoromethyl)phenyl]-2-propen-1-yl}oxy)phenoxy]phenyl}acetic acid:
TLC: Rf 0.43 (dichloromethane:methanol = 19:1);
NMR (DMSO-d₆) δ 2.13, 2.15, 3.46, 4.64, 6.28, 6.60, 6.73-6.65, 6.75, 6.93, 7.20, 7.69-7.61, 12.25.

### Example 5 (28)

{2-fluoro-3-[3-({(2E)-3-[4-(trifluoromethyl)phenyl]-2-propen-1-yl}oxy)phenoxy]phenyl}acetie acid:
TLC: Rf 0.49 (chloroform:methanol = 9:1);
NMR(CDCl₃) δ 3.74, 4.68, 6.46, 6.60-6.57, 6.76-6.67, 7.06-6.90, 7.25-7.20, 7.48, 7.57.

### Example 5 (29)

{4-[2-({(2E)-3-[4-(trifluoromethyl)phenyl]-2-propen-1-yl}oxy)phenoxy]phenyl}acetic acid:
TLC: Rf 0.51 (chloroform:methanol = 9:1);
NMR(CDCl₃) δ 3.59, 4.72, 6.34, 6.54, 6.94-6.90, 6.99-6.96, 7.06-7.03, 7.21-7.10, 7.37, 7.54.

### Example 5 (30)

3,4-dimethoxy-5-[3-({(2E)-3-[4-(trifluoromethyl)phenyl]-2-propen-1-yl}oxy)phenoxy]benzoic acid:
TLC: Rf0.36 (dichloromethane:methanol =19:1);
NMR (DMSO-d₆) δ 3.74, 3.87, 4.75, 6.52, 6.71-6.60, 6.87-6.75, 7.13, 7.27, 7.39, 7.70-7.65, 13.03.

### Example 5 (31)

{3,4-dimethoxy-5-[3-({(2E)-3-[4-(trifluoromethyl)phenyl]-2-propen-1-yl}oxy)phenoxy]phenyl}acetic acid:
TLC: Rf0.35 (dichloromethane:methanol =19:1);
NMR (DMSO-d₆) δ 3.49, 3.61, 3.79, 4.72, 6.47, 6.58-6.52, 6.64, 7.74-7.66, 7.23, 7.6, 12.31.

### Example 5 (32)

[3-(3-{3-[4-(trifluoromethyl)phenyl]propoxy}phenoxy)phenyl]acetic acid:
TLC: Rf 0.42 (dichloromethane:methanol = 19:1);
NMR (DMSO-d₆) δ 2.08-1.95, 2.86-2.75, 3.56, 3.94, 6.57-6.50, 6.69, 6.88, 6.93, 7.03, 7.25, 7.31, 7.45, 7.62, 12.27.

### Example 5 (33)

2-[3-({(2E)-3-[4-(trifluoromethyl)phenyl]-2-propen-1-yl}oxy)phenoxy]benzoic acid:
TLC: Rf 0.52 (dichloromethane:methanol = 19: 1);
NMR (DMSO-d₆) δ 4.73, 6.45, 6.54, 6.65, 6.74, 6.82, 7.02, 7.30-7.21, 7.53, 7.68, 7.82, 12.85.

### Example 6 (1) to 6 (40)

The following compounds of the present invention were obtained by carrying out similar procedures to those in Example 3→Example 4 using the compound produced in Example 2 or an alcohol derivative corresponding to a substituent thereof and 1-[(1E)-3-bromo-1-propen-1-yl]-4-(trifluoromethyl)benzene or a halide derivative corresponding to a substituent thereof.

### Example 6 (1)

4-methoxy-3-[3-({(2E)-3-[4-(trifluoromethyl)phenyl]-2-propenyl}oxy)phenoxy]benzoic acid: 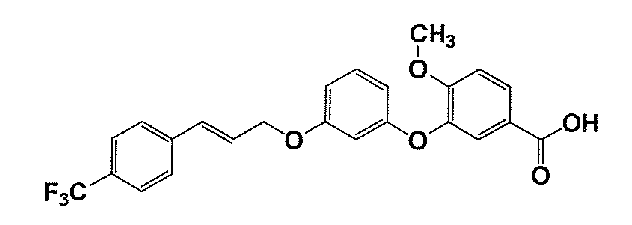 TLC: Rf 0.42 (dichloromethane:methanol = 19:1);
NMR (DMSO-d₆) δ 3.82, 4.73, 6.45, 6.55, 6.65, 6.74, 6.82, 7.28-7.21, 7.46, 7.68, 7.79, 12.80.

### Example 6 (2)

3,4-dimethoxy-5-[3-({(2E)-3-[4-(trifluoromethyl)phenyl]-2-propenyl}oxy)phenoxy]benzoic acid:
TLC: Rf 0.36 (dichloromethane:methanol = 19:1);
NMR (DMSO-d₆) δ 3.74, 3.87, 4.75, 6.52, 6.58-6.72, 6.74-6.89, 7.13, 7.27, 7.39, 7.68, 13.03.

### Example 6 (3)

{3,4-dimethoxy-5-[3-({(2E)-3-[4-(trifluoromethyl)phenylJ-2-propenyl}oxy)phenoxy]phenyl} acetic acid:
TLC: Rf 0.35 (dichloromethane:methanol = 19:1);
NMR (DMSO-d₆) δ 3.49, 3.61, 3.79, 4.72, 6.47, 6.51-6.59, 6.58-6.75, 6.76-6.89, 7.23, 7.68, 12.31.

### Example 6 (4)

[3-(3-{3-[4-(trifluoromethyl)phenyl]propoxy}phenoxy)phenyl]acetic acid:
TLC: Rf 0.42 (dichloromethane:methanol = 19:1);
NMR (DMSO-d₆) δ 2.08-1.95, 2.86-2.75, 3.56, 3.94, 6.57-6.50, 6.69, 6.88, 6.93, 7.03, 7.25, 7.31, 7.45, 7.62, 12.27.

### Example 6 (5)

2-[3-({(2E)-3-[4-(trifluoromethyl)phenyl]-2-propenyl}oxy)phenoxy]benzoic acid:
TLC: Rf 0.52 (dichloromethane:methanol = 19:1);
NMR (DMSO-d₆) δ 4.73, 6.45, 6.54, 6.65, 6.74, 6.82, 7.02, 7.30-7.21, 7.53, 7.68, 7.82, 12.85.

### Example 6 (6)

2,4-dimethoxy-3-[3-({(2E)-3-[4-(trifluoromethyl)phenyl]-2-propenyl}oxy)phenoxy]benzoic acid:
TLC: Rf 0.48 (dichloromethane:methanol = 19:1);
NMR (DMSO-d₆) δ 3.68, 3.76, 4.71, 6.34, 6.41, 6.70-6.59, 6.81, 7.01, 7.18, 7.73-7.66, 12.67.

### Example 6 (7)

{2,4-dimethoxy-3-[3-({(2E)-3-[4-(trifluoromethyl)phenyl]-2-propenyl }oxy)phenoxy]phenyl}acetic acid:
TLC: Rf 0.42 (dichloromethane:methanol = 19:1);
NMR (DMSO-d₆) δ 3.49, 3.65, 3.67, 4.70, 6.41-6.32, 6.69-6.58, 6.80, 6.85, 7.09, 7.17, 7.68, 12.21.

### Example 6 (8)

[3-(3-{2-[4-(trifluoromethyl)phenyl]ethoxy}phenoxy)phenyl]acetic acid:
TLC: Rf 0.52 (chloroform:methanol = 9:1);
NMR(CDCl₃) δ 3.08, 3.52, 4.11, 6.53-6.61, 6.84-6.96, 7.13-7.22, 7.34, 7.53.

### Example 6 (9)

[2-methyl-3-(3-{3-[4-(trifluoromethyl)phenyl]propoxy}phenoxy)phenyl]acetic acid:
TLC: Rf 0.29 (hexane:ethyl acetate = 2:1);
NMR (DMSO-d₆) δ 2.12-1.95, 2.80, 3.66, 3.92, 6.42-6.36, 6.63, 6.84, 7.06, 7.24-7.13, 7.44, 7.62, 12.35.

### Example 6 (10)

[2-fluoro-3-(3-{3-[4-(trifluoromethyl)phenyl]propoxy}phenoxy)phenyl]acetic acid:
TLC: Rf 0.19 (hexane:ethyl acetate = 2:1);
NMR (DMSO-d₆) δ 2.07-1.96, 2.80, 3.67, 3.94, 6.53-6.46, 6.68, 7.18-7.03, 7.24, 7.45, 7.63, 12.50.

### Example 6 (11)

{3-[3-(2-phenylethoxy)phenoxy]phenyl}acetic acid:
TLC: Rf 0.47 (chloroform:methanol = 9:1);
NMR(CDCl₃) δ 3.07, 3.61, 4.13, 6.56-6.67, 6.89-6.95, 7.01, 7.17-7.33.

### Example 6 (12)

{3-[3-(3-phenylpropoxy)phenoxy]phenyl}acetic acid:
TLC: Rf 0.58 (chloroform:methanol = 9:1);
NMR (DMSO-d₆) δ 2.03-2.12, 2.76-2.81, 3.61, 3.92, 6.56-6.59, 6.63-6.66, 6.91-6.95, 7.01, 7.16-7.30.

### Example 6 (13)

{3-[3-(4-phenylbutoxy)phenoxy]phenyl}acetic acid:
TLC: Rf 0.62 (chloroform:methanol = 9:1);
NMR(CDCl₃) δ 1.76-1.82, 2.64-2.69, 3.61, 3.90-3.94, 6.55-6.56, 6.62-6.66, 6.90-6.96, 6.99-7.02, 7.14-7.30.

### Example 6 (14)

{3-[3-(benzyloxy)phenoxy]phenyl}acetic acid:
TLC: Rf 0.49 (chloroform: methanol = 9:1);
NMR(CDCl₃) δ 3.60, 5.01, 6.58-6.62, 6.64, 6.73, 6.90-6.96, 7.01, 7.19-7.42.

### Example 6 (15)

[3-(3 -{[4-(trifluoromethyl)benzyl]oxy}phenoxy)phenyl]acetic acid:
TLC: Rf 0.58 (chloroform: methanol = 9: 1);
NMR(CDCl₃) δ 3.60, 5.07, 6.59-6.63, 6.70, 6.90, 6.95, 7.01, 7.21, 7.27, 7.51, 7.62.

### Example 6 (16)

[3-(3-{[(2E)-3-phenyl-2-propenyl]oxy}phenoxy)phenyl]acetic acid:
TLC: Rf 0.53 (chloroform:methanol = 9:1);
NMR(CDCl₃) δ 3.60, 4.65, 6.37, 6.58-6.63, 6.67-6.72, 6.91-6.96, 7.01, 7.20-7.41.

### Example 6 (17)

3-[3-(4-phenylbutoxy)phenoxy]benzoic acid:
TLC: Rf 0.57 (chloroform:methanol = 9:1);
NMR(CDCl₃) δ 1.79-1.81, 2.67, 3.93, 6.57-6.60, 6.66-6.69, 7.14-7.29, 7.42, 7.73, 7.84.

### Example 6 (18)

2-(3-{3-[4-(trifluoromethyl)phenyl]propoxy}phenoxy)benzoic acid:
TLC: Rf 0.51 (chloroform:methanol = 9:1);
NMR(CDCl₃) δ 2.06-2.14, 2.86, 3.94, 6.64-6.67, 6.75, 6.90, 7.20, 7.25-7.32, 7.45-7.55, 8.16.

### Example 6 (19)

3-[3-(3-{2-[4-(trifluoromethyl)phenyl]ethoxy}phenoxy)phenyl]propanic acid:
TLC: Rf 0.49 (chloroform: methanol = 9:1);
NMR(CDCl₃) δ 2.65, 2.92, 3.12, 4.15, 6.54-6.64, 6.83-6.86, 6.95, 7.17-7.27, 7.38, 7.55.

### Example 6 (20)

4-methoxy-2-[3-({(2E)-3-[4-(trifluoromethyl)phenyl]-2-propenyl}oxy)phenoxy]benzoic acid:
TLC: Rf 0.51 (hexane: ethyl acetate =1:1);
NMR (DMSO-d₆) δ 3.74, 4.73, 6.45, 6.55-6.50, 6.65, 6.73, 6.82, 6.85, 7.24, 7.68, 7.86, 12.48.

### Example 6 (21)

2-[3-({(2E)-3-[6-(trifluoromethyl)pyridin-3-yl]-2-propenyl}oxy)phenoxy]benzoic acid: TLC: Rf 0.51 (chloroform:methanol = 9:1);
NNIR (DMSO-d₆) δ 4.76, 6.46, 6.55, 6.75, 6.81-6.85, 7.03, 7.22-7.29, 7.54, 7.82, 7.85, 8.19, 8.85, 12.86.

### Example 6 (22)

2-[3-({3-[4-(trifluoromethyl)phenyl]-2-propynyl}oxy)phenoxy]benzoic acid:
TLC: Rf 0.53 (chloroform:methanol = 9:1);
NMR (DMSO-d₆) δ 5.05, 6.50, 6.61, 6.78, 7.03, 7.26, 7.28, 7.52, 7.63, 7.74, 7.82, 12.86.

### Example 6 (23)

2-(3-{[4-(trifluoromethyl)benzyl]oxy}phenoxy)benzoic acid:
TLC: Rf 0.35 (chloroform:methanol = 9:1);
NMR(CDCl₃) δ 5.10, 5.69-6.73, 6.82-6.88, 7.22, 7.29-7.35, 7.46, 7.51, 7.63, 8.18.

### Example 6 (24)

2-[3-(3-phenylpropoxy)phenoxy]benzoic acid:
TLC: Rf 0.51 (chloroform:methanol = 9:1);
NMR(CDCl₃) δ 2.04-2.15, 2.77-2.82, 3.95, 6.64, 6.67, 6.78, 6.90, 7.16-7.32, 7.48, 8.19.

### Example 6 (25)

5-methyl-2-[3-({(2E)-3-[4-(trifluoromethyl)phenyl]-2-propenyl}oxy)phenoxy]benzoic acid:
TLC: Rf 0.64 (hexane:ethyl acetate = 1:1);
NMR (DMSO-d₆) δ 2.32, 4.71, 6.41, 6.49, 6.73-6.58, 6.81, 6.94, 7.22, 7.33, 7.62, 7.68, 12.78.

### Example 6 (26)

2-[3-(benzyloxy)phenoxy]benzoic acid:
TLC: Rf 0.50 (chloroform:methanol = 9:1);
NMR (DMSO-d₆) δ 5.05, 6.99-6.74, 6.85-6.90, 7.19-7.50, 8.20.

### Example 6 (27)

2-(3-{[(2E)-3-phenyl-2-propenyl]oxy}phenoxy)benzoic acid:
TLC: Rf 0.53 (chloroform:methanol = 9:1);
NMR (DMSO-d₆) δ 4.68, 6.37, 6.68-6.73, 6.82-6.86, 6.89, 7.20, 7.25-7.46, 8.19.

### Example 6 (28)

2-(3-{[(2E)-3-(1, 1'-biphenyl-4-yl)-2-propenyl]oxy}phenoxy)benzoic acid:
TLC: Rf 0.56 (chloroform:methanol = 9:1);
NMR (DMSO-d₆) δ 4.70, 6.44-6.56, 6.72-6.80, 7.01, 7.21-7.27, 7.32-7.37, 7.42-7.56, 7.63-7.68, 7.80.

### Example 6 (29)

2-(3-{2-[4-(trifluoromethyl)phenyl]ethoxy}phenoxy)benzoic acid:
TLC: Rf 0.51 (chloroform: methanol = 9:1);
NMR (DMSO-d₆) δ 3.14, 4.18, 6.63, 6,67, 6.76, 6.89, 7.21, 7.26-7.31, 7.39, 7.48, 7.57, 8.17.

### Example 6 (30)

2-[3-(2-phenylethoxy)phenoxy]benzoic acid:
TLC: Rf 0.57 (chloroform:methanol = 9:1);
NMR(CDCl₃) δ 3.09, 4.16, 6.64-6.69, 6.78, 6.88, 7.18-7.34, 7.44-7.50, 8.19.

### Example 6 (31)

2-[3-(4-phenylbutoxy)phenoxy]benzoic acid:
TLC: Rf 0.53 (chloraform:methanol = 9:1);
NMR(CDCl₃) δ 1.79-1.83, 2.68, 3.93-3.97, 6.65, 6.62-6.68, 6.89, 7.15-7.31, 7.44-7.50, 8.19.

### Example 6 (32)

2-[3-(2-{5-methyl-2-[4-(trifluoromethyl)phenyl]-1,3-oxazol-4-yl}ethoxy)phenoxy]benzoic acid:
TLC: Rf 0.44 (chloroform:methanol = 9: 1);
NMR (DMSO-d₆) δ 2.36, 2.92, 4.18, 6.42, 6.46, 6.67, 7.00, 7.21, 7.25, 7.53, 7.80, 7.84, 8.08, 12.84.

### Example 6 (33)

5-chloro-2-[3-({(2E)-3-[4-(trifluoromethyl)phenyl]-2-propenyl}oxy)phenoxy]benzoic acid:
TLC: Rf 0.65 (hexane:ethyl acetate = 1:1);
NMK (DMSO-d₆) δ 4.74, 6.50, 6.72-6.57, 6.87-6.74, 7.04, 7.27, 7.56, 7.69, 7.79, 13.22.

### Example 6 (34)

2-chloro-4-methyl-3-[3-({(2E)-3-[4-(trifluoromethyl)phenyl]-2-propenyl}oxy)phenoxy]benzaic acid:
TLC: Rf 0.32 (hexane:ethyl acetate = 1:1);
NMR (DMSO-d₆) δ 2.14, 4.73, 6.30, 6.42, 6.77-6.58, 6.82, 7.23, 7.40, 7.65, 7.69, 13.40.

### Example 6 (35)

3-chloro-2-[3-({(2E)-3-[4-(trifluoromethyl)phenyl]-2-propenyl}oxy)phenoxy]benzoic acid:
TLC: Rf 0.46 (hexane: ethyl acetate =1:1);
NMR (DMSO-dg) δ 4.71, 6.28, 6.38, 6.71-6.58, 6.82, 7.19, 7.41, 7.69, 7.84-7.77, 13.15.

### Example 6 (36)

2-{3-[(5-methyl-2-phenyl-1,3-axazal-4-yl)methoxy]phenoxy}henzoie acid:
TLC: Rf 0.46 (chloroform: methanol = 9:1);
NNM(CDCl₃) δ 2.41, 4.96, 6.70, 6.77, 6.86-6.90, 7.15-7.21, 7.30, 7.40-7.47, 7.96-8.00, 8.15.

### Example 6 (37)

2-[3-({4-methyl-2-[4-(trifluoromethyl)phenyl]-1,3-thiazol-5-yl}methoxy)phenoxy]benzoic acid:
TLC: Rf0.47 (chloroform:methanol = 9:1);
NMR(CDCl₃) δ 2.48, 5.18, 6.71-6.75, 6.83-6.87, 6.90, 7.20-7.22, 7.34, 7.48, 7.67, 8.01, 8.18.

### Example 6 (38)

3-[3-({3-[4-(trifluoromethyl)phenyl]-2-propynyl}oxy)phenoxy]benzoic acid:
TLC: Rf 0.36 (dichloromethane:methanol = 19:1);
NMR (DMSO-d₆) δ 5.09, 6.67, 6.78, 6.88, 7.27-7.32, 7.36, 7.42-7.45, 7.49, 7.61, 7.67-7.72, 7.73, 13.11.

### Example 6 (39)

3-[3-({5-methyl-2-[4-(trifluoromethyl)phenyl]-1,3-oxazol-4-yl}methoxy)phenoxy]benzoic acid:
TLC: Rf 0.49 (dichloromethane:methanol = 9:1);
NMR (DMSO-d₆) δ 2.44, 5.03, 6.64, 6.78, 6.88, 7.29, 7.34, 7.43, 7.50, 7.69, 7.87, 8.11, 13.10.

### Example 6 (40)

{3-[3-({3-[4-(trifluoromethyl)phenyl]-2-propynyl}oxy)phenoxy]phenyl}acetic acid:
TLC: Rf 0.45 (dichloromethane:methanol = 19: 1);
NMR (DMSO-d₆) δ 3.55, 5.07, 6.61, 6.70, 6.83, 6.89, 6.95, 7.03, 7.28, 7.31, 7.63, 7.75, 12.38.

### Example 7

methyl 4-chloro-3-(2-formylphenoxy)benzoate:

Potassium carbonate (1.02 g) was added to a solution of 2-fluorobenzaldehyde (765 mg) and methyl 4-chloro-3-hydroxybenzoate (1.15g) in dimethylformamide (6 mL) and stirred at 135°C for 2 hours. The reaction mixture was cooled down to room temperature, water was added thereto and the mixture was extracted with a mixed solvent (hexane : ethyl acetate = 1:2) for two times. The organic layer was successively washed with water and a saturated saline solution, dried over anhydrous sodium sulfate and concentrated. The resulting residue was purified by a silica gel column chromatography (hexane : ethyl acetate = 20:1) to give the title compound (609 mg) having the following properties.
TLC: Rf 0.49 (hexane:ethyl acetate = 4:1);
NMR(CDCl₃) δ 10.57, 7.98, 7.86, 7.73, 7.59, 7.52, 7.24, 6.76, 3.90.

### Example 8

4-chloro-3-[2-({(2E)-3-[4-(trifluoromethyl)phenyl]-2-propenyl}oxy)phenoxy]benzoic acid:

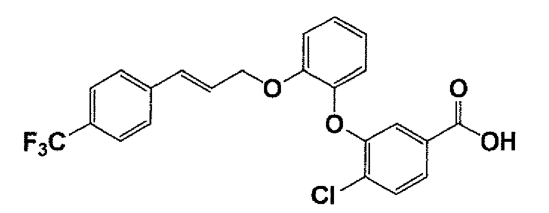

m-Chloro-perbenzoic acid (633 mg) was added to a solution of the compound (609 mg) produced in Example 7 in dichloromethane (8 mL) and stirred at room temperature for one night. A saturated aqueous solution of sodium thiosulfate was added and, after the mixture was stirred for a while, it was extracted with ethyl acetate. The organic layer was successively washed with a saturated aqueous solution of sodium hydrogen carbonate, water and saturated saline solution, dried over anhydrous sodium sulfate and concentrated. A few drops of concentrated hydrochloric acid were added to a solution of the resulting residue in methanol (4 mL) and the mixture was stirred at room temperature for 30 minutes and concentrated. The resulting crystals were recrystallized from a mixed solvent of hexane and ethyl acetate. The same operations as in Example 3 to Example 4 were carried out using the crystals produced hereinabove instead of the compound produced in Example 2 to give the compound (582 mg) of the present invention having the following properties.
TLC: Rf 0.41 (dichloronnethane:methanol = 9:1);
NMR (DMSO-d₆) δ 4.75, 6.47, 7.07, 7.16, 7.24, 7.29, 7.53, 7.59, 7.71-7.63, 13.21.

### Example 9 (1) to 9 (131)

The following compounds of the present invention were obtained by carrying out similar procedures to those in Example 8 using a corresponding aldehyde derivative instead of the compound produced in Example 7 and 1-[(1E)-3-bromo-1-propen-1-yl]-4-(trifluoromethyl)benzene or a halide derivative corresponding to a substituent thereof and, if necessary, converting it to hydrochloride by a known method.

### Example 9 (1)

3-[2-({(2E)-3-[4-(trifluoromethyl)phenyl]-2-propenyl}oxy)phenoxy]benzoic acid: 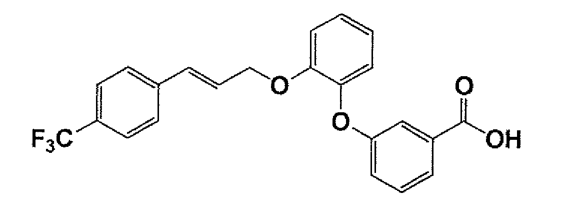 TLC: Rf 0.33 (dichloromethane:methanol = 19:1);
NMR (DMSO-d₆) δ 4,74, 6.46, 7.04, 7.33-7.16, 7.45, 7.52, 7.61, 7.64, 13.04.

### Example 9 (2)

2-methyl-3-[2-({(2E)-3-[4-(trifluoromethyl)phenyl]-2-propenyl}oxy)phenoxy]benzoic acid:
TLC: Rf 0.38 (dichloromethane:methanol = 19:1);
NMR (DMSO-d₆) δ 2.49, 4.74, 6.50, 6.79, 7.05-6.98, 7.26-7.14, 7.48, 7.56, 7.65, 12.98.

### Example 9 (3)

4-methyl-3-[2-({(2E)-3-[4-(trifluoromethyl)phenyl]-2-propenyl}oxy)phenoxy]benzoic acid:
TLC: Rf 0.47 (dichloromethane:methanol = 19:1);
NMR (DMSO-d₆) δ 2.38, 4.75, 6.47, 7.09-6.99, 7.13, 7.28-7.18, 7.40, 7.57-7.49, 7.66, 12.85.

### Example 9 (4)

3-(2-{3-[4-(trifluoromethyl)phenyl]propoxy}phenoxy)benzoic acid:
TLC: Rf 0.46 (dichloromethane:methanol = 19:1);
NMR (DMSO-d₆) δ 1.88-1.74, 2.38, 3.89, 7.02, 7.32-7.13, 7.45, 7.62-7.52, 13.03.

### Example 9 (5)

{2-methyl-3-[2-({(2E)-3-[4-(trifluoromethyl)phenyl]-2-propenyl}oxy)phenoxy]phenyl} acetic acid:
TLC: Rf 0.38 (dichloromethane:methanol = 19:1);
NMR (DMSO-d₆) δ 2.18, 3.64, 4.76, 6.65-6.48, 6.88, 6.99-6.91, 7.04, 7.13, 7.21, 7.58, 7.67, 12.34.

### Example 9 (6)

{4-methyl-3-[2-({(2E)-3-[4-(trifluoromethyl)phenyl]-2-propenyl}oxy)phenoxy]pheny}acetic acid:
TLC: Rf 0.36 (dichlaromethane:methanol = 19:1);
NMR (DMSO-d₆) δ 2.23, 3.40, 4.74, 6.63-6.45, 6.87, 7.00-6.89, 7.13, 7.25-7.17, 7.58, 7.67, 12.20.

### Example 9 (7)

[3-(2- {3-[4-(trifluoromethyl)phenyl]propoxy}phenoxy)phenyl]acetic acid:
TLC: Rf0.38 (dichloromethane:methanol = 19:1);
NMR (DMSO-d₆) δ 1.90-1.75, 2.48-2.45, 3.50, 3.90, 6.72, 6.79, 6.90, 6.98, 7.26-7.07, 7.57, 12.28.

### Example 9 (8)

4-[2-({(2E)-3-[4-(trifluoromethyl)phenyl]-2-propenyl}oxy)phenoxy]benzoic acid:
TLC: Rf 0.48 (chloroform: methanol = 9:1);
NMR (DMSO-d₆) δ 12.69, 7.91, 7.62, 7.50, 7.29-7.24, 7.21, 7.09-7.00, 6.94, 6.46, 6.39, 4.73.

### Example 9 (9)

3-[4-({(2E)-3-[4-(trifluoromethyl)phenyl]-2-propenyl}oxy)phenoxy]benzoic acid
TLC: Rf 0.43 (chloroform:methanol = 9:1);
NMR (DMSO-d₆) δ 13.03, 7.72, 7.68, 7.63, 7.47, 7.34, 7.22, 7.06, 6.87, 6.70, 4.77.

### Example 9 (10)

2-[4-({(2E)-3-[4-(trifluoromethyl)phenyl]-2-propenyl}oxy)phenoxy]benzoic acid:
TLC: Rf 0.70 (dichloromethane:methanol = 19:1);
NMR (DMSO-d₆) δ 4.74, 6.68, 6.96-6.82, 7.05-6.99, 7.18, 7.49, 7.74-7-66, 7.77, 12.82.

### Example 9 (11)

2-[2-({(2E)-3-[4-(trifluoromethyl)phenyl]-2-propenyl}oxy)phenoxy]benzoic acid:
TLC: Rf 0.51 (dichloromethane:methanol = 19:1);
NMR (DMSO-d₆) δ 4.73, 6.51, 6.69, 7.25-6.96, 7.42, 7.56, 7.66, 7.80, 12.82.

### Example 9 (12)

3-(4-{3-[4-(trifluoromethyl)phenyl]propoxy}phenoxy)benzoic acid:
TLC: Rf 0.31 (hexane:ethyl acetate = 2:1);
NMR (DMSO-d₆) δ 2.12-2.00, 2.84, 3.98, 7.06-6.95, 7.22, 7.33, 7.51-7.43, 7.67-7.60, 13.05.

### Example 9 (13)

[3-(4-{3-[4-(trifluoromethyl)phenyl]propoxy}phenoxy)pheny]acetic acid
TLC: Rf 0.21 (hexane:ethyl acetate = 2:1);
NMR (DMSO-d₆) δ 2.11-1.98, 2.84, 3.53, 3.96, 6.77, 6.84, 7.01-6.92, 7.26, 7.47, 7.64, 12.30.

### Example 9 (14)

{2-[2-({(2E)-3-[4-(trifluoromethyl)phenyl]-2-propenyl}oxy)phenoxy]phenyl}acetic acid:
TLC: Rf 0.35 (hexane:ethyl acetate = 2: 1);
NMR (DMSO-d₆) δ 3.69, 4.75, 6.62-6.51, 7.04-6.94, 7.25-7.12, 7.33, 7.58, 7.66, 12.26.

### Example 9 (15)

3,4-dimethoxy-5-[2-({(2E)-3-[4-(trifluoromethyl)phenyl]-2-propenyl}oxy)phenoxy]benzoic acid:
TLC: Rf 0.61 (chloroform:methanol = 9:1);
NMR(CDCl₃) δ 3.89, 4.00, 4.70, 6.30, 6.51, 6.96, 7.03, 7.10-7.16, 7.34, 7.38, 7.52.

### Example 9 (16)

3-methyl-5-[2-({(2E)-3-[4-(trifluoromethyl)phenyl]-2-propenyl}oxy)phenoxy]benzoic acid:
TLC: Rf 0.60 (chloroform:methanol = 9:1);
NMR(CDCl₃) δ 2.35, 4.72, 6.31, 6.52, 6.96-7.16, 7.37, 7.41, 7.52, 7.58.

### Example 9 (17)

2-chloro-5-[2-({(2E)-3-[4-(trifluoromethyl)phenyl]-2-propenyl}oxy)phenoxy]benzoic acid:
TLC: Rf 0.41 (dichloromethane:methanol = 9:1);
NMR (DMSO-d₆) δ 4.75, 6.47, 7.11-7.01, 7.24-7.17, 7.26, 7.47, 7.55, 7.65, 13.46.

### Example 9 (18)

2-chloro-3-[2-({(2E)-3-[4-(trifluoromethyl)phenyl]-2-propenyl}oxy)phenoxy]benzoic acid:
TLC: Rf 0.38 (dichloromethane:methanol = 9:1);
NMR (DMSO-d₆) δ 4.74, 6.49, 6.84, 7.03, 7.15, 7.27-7.23, 7.30, 7.40, 7.56, 7.64, 13.49.

### Example 9 (19)

{3-methyl-5-[2-({(2E)-3-[4-(trifluoromethyl)phenyl]-2-propenyl }oxy)phenoxy]phenyl}acetic acid:
TLC: Rf 0.45 (dichloromethane:methanol = 9:1);
NMR (DMSO-d₆) δ 2.20, 3.46, 4.74, 6.62-6.44, 6.74, 7.08-6.95, 7.26-7.16, 7.56, 7.66, 12.28.

### Example 9 (20)

{2-chloro-5-[2-({(2E)-3-[4-(trifluoromethyl)phenyl]-2-propenyl}oxy)phenoxy]phenyl}acetic acid:
TLC: Rf 0.43 (dichloromethane:methanol = 9:1);
NMR (DMSO-d₆) δ 3.66, 4.74, 6.59-6.43, 6.75, 7.05-6.97, 7.12, 7.28-7.18, 7.34, 7.56, 7.66, 12.44.

### Example 9 (21)

{2-chloro-3-[2-({(2E)-3-[4-(trifluoromethyl)phenyl]-2-propenyl }oxy)phenoxy]phenyl}acetic acid:
TLC: Rf 0.43 (dichloromethane:methanol = 9:1);
NMR (DMSO-d₆) δ 3.77, 4.77, 6.57-6.46, 6.63, 7.11-6.97, 7.27-7.14, 7.56, 7.67, 12.48.

### Example 9 (22)

{4-chloro-3-[2-({(2E)-3-[4-(trifluoromethyl)phenyl]-2-propenyl}oxy)phenoxy]phenyl}acetic acid:
TLC: Rf 0.48 (dichloromethane:methanol = 9:1);
NMR (DMSO-d₆) δ 3.47, 4.74, 6.62-6.44, 6.68, 7.10-6.94, 7.28-7.17, 7.47, 7.57, 7.67, 12.31.

### Example 9 (23)

3-[2-({4-methyl-2-[4-(trifluoromethyl)phenyl]-1,3-thiazol-5-yl}methoxy)phenoxy]benzoic acid:
TLC: Rf 0.52 (chloroform:methanol = 9:1);
NMR (DMSO-d₆) δ 2.29, 5.30, 7.08, 7.13, 7.20, 7.24-7.31, 7.36, 7.42, 7.62, 7.82, 7.96, 13.02.

### Example 9 (24)

3-[2-(2-{5-methyl-2-[4-(trifluoromethyl)phenyl]-1,3-oxazol-4-yl}ethoxy)phenoxy]benzoic acid:
TLC: Rf 0.49 (chloroform:methanol = 9:1);
NMR (DMSO-d₆) δ 2.09, 2.73, 4.20, 6.94-7.03, 7.08-7.12, 7.17, 7.20-7.25, 7.27, 7.48, 7.82, 8.01, 12.92.

### Example 9 (25)

3-[2-chloro-6-({(2E)-3-[4-(trifluoromethyl)phenyl]-2-propenyl}oxy)phenoxy]benzoic acid:
TLC: Rf 0.47 (dichloromethane:methanol = 9:1);
NMR (DMSO-d₆) δ 4.78, 6.44, 7.35-7.18, 7.55-7.44, 7.68-7.59, 13.10.

### Example 9 (26)

3-[5-chloro-2-({(2E)-3-[4-(trifluoromethyl)phenyl]-2-propenyl}oxy)phenoxy]benzoic acid:
TLC: Rf 0.44 (dichloromethane:methanol = 9:1);
NMR (DMSO-d₆) δ 4.75, 6.44, 7.24, 7.33-7.27, 7.35, 7.47, 7.52, 7.67-7.61, 13.11.

### Example 9 (27)

3-[3-chloro-2-({(2E)-3-[4-(trifluoromethyl)phenyl]-2-propenyl}oxy)phenoxy]benzoic acid:
TLC: Rf 0.44 (dichloromethane:methanol = 9:1);
NMR (DMSO-d₆) δ 4.71, 6.51, 6.66, 7.11, 7.19, 7.28, 7.43-7.35, 7.49, 7.57, 7.70-7.62, 13.13.

### Example 9 (28)

3-[4-methyl-2-({(2E)-3-[4-(trifluoromethyl)phenyl]-2-propenyl}oxy)phenoxy]benzoic acid:
TLC: Rf0.44 (dichloromethane:methanol = 9:1);
NMR (DMSO-d₆) δ 2.33, 4.71, 6.52-6.38, 6.84, 7.10-7.04, 7.20, 7.29, 7.44, 7.51, 7.59, 7.64, 13.03.

### Example 9 (29)

{3-[2-chloro-6-({(2E)-3-[4-(trifluoromethyl)phenyl]-2-propenyl}oxy)phenoxy]phenyl}acetic acid:
TLC: Rf 0.74 (dichloromethane:methanol = 9:1);
NMR (DMSO-d₆) δ 3.54, 4.76, 6.52-6.48, 6.65, 6.82, 6.93, 7.31-7.16, 7.52, 7.65, 12.32.

### Example 9 (30)

{3-[5-chloro-2-({(2E)-3-[4-(trifluoromethyl)phenyl]-2-propenyl }oxy)phenoxy]phenyl}acetic acid:
TLC: Rf 0.60 (dichloromethane:methanol = 9:1);
NMR (DMSO-d₆) δ 3.55, 4.75, 6.58-6.43, 6.79, b.88, 6.98, 7.13, 7.31-7.20, 7.56, 7.66, 12.33.

### Example 9 (31)

{3-[3-chloro-2-({(2E)-3-[4-(trifluoromethyl)phenyl]-2-propenyl}oxy)phenoxy]phenyl}acetic acid:
TLC: Rf 0.56 (dichloromethane:methanol = 9:1);
NMR (DMSO-d₆) δ 3.54, 4.71, 6.54, 6.70, 6.85, 6.92, 7.04-6.95, 7.14, 7.29, 7.60, 7.67, 12.34.

### Example 9 (32)

{3-[4-methyl-2-({(2E)-3-[4-(trifluoromethyl)phenyl]-2-propenyl}oxy)phenoxy]phenyl}acetic acid:
TLC: Rf 0.63 (dichloromethane:methanol = 9:1);
NMR (DMSO-d₆) δ 2.31, 3.51, 4.70, 6.52-6.47, 6.70, 6.83-6.77, 6.90, 6.97, 7.05, 7.22, 7.54, 7.65, 12.31.

### Example 9 (33)

{3-[2-({4-methyl-2-[4-(trifluoromethyl)phenyl]-1,3-thiazol-5-yl}methoxy)phenoxy]phenyl}acetic acid:
TLC: Rf 0.53 (chloroform:methanol = 9:1);
NMR (DMSO-d₆) δ 2.32, 3.50, 5.31, 6.69, 6.75-6.79, 6.94, 7.00-7.11, 7.18-7.26, 7.34, 7.83, 8.01, 12.30.

### Example 9 (34)

{3-[2-(2-{5-methyl-2-[4-(trifluoromethyl)phenyl]-1,3-oxazol-4-yl }ethoxy)phenoxy]phenyl} acetic acid:
TLC: Rf 0.54 (chloroform: methanol = 9:1);
NMR (DMSO-d₆) δ 2.14, 2.76, 3.44, 4.20, 6.57, 6.72, 6.84, 6.95, 6.99-7.04, 7.07-7.14, 7.15-7.19, 7.84, 8.05, 12.33.

### Example 9 (35)

3-[2-({(2E)-3-[6-(trifluoromethyl)-3-pyridinyl]-2-propenyl}oxy)phenoxy]benzoic acid hydrochloride:
TLC: Rf 0.47 (chloroform:methanol = 9:1);
NMR (DMSO-d₆) δ 4.76, 6.43, 6.66, 6.99-7.09, 7.16-7.29, 7.31, 7.46, 7.60, 7.81, 8.04, 8.65.

### Example 9 (36)

3-[2-({3-[4-(trifluoromethyl)phenyl]-2-propynyl}oxy)phenoxy]benzoic acid:
TLC: Rf0.49 (chloroform:methanol =9:1);
NMR (DMSO-d₆) δ 5.09, 7.07, 7.15, 7.19, 7.24-7.31, 7.36, 7.44, 7.58, 7.61, 7.72, 13.03.

### Example 9 (37)

3-[4-fluoro-2-({(2E)-3-[4-(trifluoromethyl)phenyl]-2-propenyl}oxy)phenoxy]benzoic acid:
TLC: Rf 0.60 (dichloromethane:methanol = 9:1);
NMR (DMSO-d₆) δ 4.76, 6.45, 6.86, 7.28-7.18, 7.31, 7.46, 7.52, 7.67-7.58, 13.07.

### Example 9 (38)

3-[4-(trifluoromethyl)-2-({(2E)-3-[4-(trifluoromethyl)phenyl]-2-propenyl}oxy)phenoxy]benzoic acid:
TLC: Rf 0.56 (dichloromethane:methanol = 9:1);
NMR (DMSO-d₆) δ 4.87, 6.49, 7.35-7.25, 7.42-7.37, 7.58-7.47, 7.71-7.62, 13.14.

### Example 9 (39)

3-[4-nitro-2-({(2E)-3-[4-(trifluoromethyl)phenyl]-2-propenyl}oxy)phenoxy]benzoic acid:
TLC: Rf 0.60 (dichloromethane:methanol = 9:1);
NMR (DMSO-d₆) δ 4.93, 6.57, 7.27, 7.35, 7.47, 7.54, 7.59, 7.66, 7.74, 7.92, 8.04, 13.18.

### Example 9 (40)

{3-[2-({(2E)-3-[6-(trifluoromethyl)-3-pyridinyl]-2-propenyl}oxy)phenoxy]phenyl}acetic acid:
TLC: Rf 0.50 (chloroform:methanol = 9:1);
NMR (DMSO-d₆) δ 3.52, 4.76, 6.51, 6.68, 6.75, 6.81-6.84, 6.92, 7.00, 7.09, 7.16-7.28, 7.83, 8.05, 8.71, 12.30.

### Example 9 (41)

{3-[2-({3-[4-(trifluoromethyl)phenyl]-2-propynyl}oxy)phenoxy]phenyl}acetic acid:
TLC: Rf 0.57 (chloroform:methanol = 9:1);
NMR (DMSO-d₆) δ 3.51, 5.09, 6.71, 6.83, 6.93, 6.99-7.07, 7.17-7.27, 7.31-7.36, 7.60, 7.73, 12.31.

### Example 9 (42)

3-[2-({(2E)-3-[2-fluoro-4-(trifluoromethyl)phenyl]-2-propenyl}oxy)phenoxy]benzoic acid:
TLC: Rf 0.47 (dichloromethane:methanol = 19:1);
NMR (DMSO-d₆) δ 4.78, 6.65-6.46, 7.04, 7.21-7.15, 7.31-7.23, 7.43, 7.50, 7.63-7.55, 7.71, 13.01.

### Example 9 (43)

3-(2-{[(2E)-3-(2,4-dichlorophenyl)-2-propenyl]oxy}phenoxy)benzoic acid:
TLC: Rf 0.45 (dichloromethane:methanol = 19:1);
NMR (DMSO-d₆) δ 4.76, 6.37, 6.74, 7.04, 7.19-7.12, 7.45-7.22, 7.63-7.50, 13.00.

### Example 9 (44)

2-methyl-3-[2-({3-[4-(trifluoromethyl)phenyl]-2-propynyl}oxy)phenoxy]benzoic acid:
TLC: Rf 0.43 (dichloromethane:methanol = 19:1);
NMR (DMSO-d₆) δ 2.45, 5.10, 6.77-6.86, 6.87-6.93, 7.00, 7.12-7.23, 7.32, 7.48, 7.60, 7.73, 12.98.

### Example 9 (45)

3-methyl-5-[2-({3-[4-(trifluoromethyl)phenyl]-2-propynyl}oxy)phenoxy]benzoic acid:
TLC: Rf 0.39 (dichloromethane:methanol = 19:1);
NMR (DMSO-d₆) δ 2.28, 5.09, 6.98-7.17, 7.26, 7.36, 7.44, 7.57, 7.72, 12.95.

### Example 9 (46)

2-methyl-3-[4-methyl-2-({3-[4-(trifluoromethyl)phenyl]-2-propynyl}oxy)phenoxy]benzoic acid:
TLC: Rf 0.40 (dichloromethane:methanol = 19:1);
NMR (DMSO-d₆) δ 2.31, 2.45, 5.06, 6.77, 6.80-6.88, 7.11-7.18, 7.45, 7.60, 7.74, 12.96.

### Example 9 (47)

3-methyl-5-[4-methyl-2-({3-[4-(trifluoromethyl)phenyl]-2-propynyl}oxy)phenoxy]benzoic acid:
TLC: Rf 0.43 (dichloromethane: methanol = 19:1);
NMR (DMSO-d₆) δ 2.28, 2.34, 5.05, 6.86, 6.95-7.03, 7.07, 7.17, 7.41, 7.58, 7.72, 12.93.

### Example 9 (48)

{2-methyl-3-[2-({3-[4-(trifluoromethyl)phenyl]-2-propynyl}oxy)phenoxy]phenyl}acetic acid:
TLC: Rf 0.38 (dichloromethane:methanol = 19:1);
NMR (DMSO-d₆) δ 2.16, 3.64, 5.12, 6.57, 6.82, 6.91-7.01, 7.04, 7.10-7.23, 7.32, 7.55-7.68, 7.74, 12.36.

### Example 9 (49)

{3-methyl-5-[2-({3-[4-(trifluoromethyl)phenyl]-2-propynyl}oxy)phenoxy]phenyl}acetic acid:
TLC: Rf0.45 (dichloromethane:methanol = 19:1);
NMR (DMSO-d₆) δ 2.17, 3.45, 5.09, 6.53, 6.62, 6.74, 6.98-7.07, 7.21, 7.33, 7.60, 7.73, 12.29.

### Example 9 (50)

3-methyl-5-[4-methyl-2-({(2E)-3-[4-(trifluoromethyl)phenyl]-2-propenyl}oxy)phenoxy]benzoic acid:
TLC: Rf 0.47 (dichloromethane:methanol = 9:1);
NMR (DMSO-d₆) δ 2.30, 2.32, 4.71, 6.46-6.47, 6.83, 7.02-7.07, 7.41, 7.51, 7.64, 12.95.

### Example 9 (51)

2-methyl-3-[4-methyl-2-({(2E)-3-[4-(trifluoromethyl)phenyl]-2-propenyl}oxy)phenoxy]benzoic acid:
TLC: Rf 0.25 (dichloromethane:methanol = 9:1);
NMR (DMSO-d₆) δ 2.31, 2.48, 4.70, 6.46-6.48, 6.73, 6.78-6.81, 6.92, 7.05, 7.15, 7.43, 7.54, 7.64, 12.97.

### Example 9 (52)

{3-methyl-5-[4-methyl-2-({3-[4-(trifluoromethyl)phenyl]-2-propynyl}oxy)phenoxy]phenyl}acetic acid:
TLC: Rf0.43 (dichloromethane:methanol = 19:1);
NMR (DMSO-d₆) δ 2.16, 2.33, 3.44, 5.06, 6.50, 6.58, 6.71, 6.83, 6.92, 7.15, 7.60, 7.73, 12.28.

### Example 9 (53)

{2-methyl-3-[4-methyl-2-({3-[4-(trifluoromethyl)phenyl]-2-propynyl}oxy)phenoxy]phenyl}acetic acid:
TLC: Rf 0.39 (dichloromethane: methanol = 19:1);
NMR (DMSO-d₆) δ 2.16, 2.31, 3.63, 5.08, 6.51, 6.74, 6.79, 6.91, 7.00, 7.14, 7.61, 7.74, 12.35.

### Example 9 (54)

3-[4-methyl-2-({3-[4-(trifluoromethyl)phenyl]-2-propynyl}oxy)phenoxy]benzoic acid:
TLC: Rf 0.41 (dichloromethane: methanol = 19:1);
NMR (DMSO-d₆) δ 2.34, 5.05, 6.87, 7.03, 7.13-7.21, 7.24-7.30, 7.42, 7.53-7.63, 7.72, 13.01.

### Example 9 (S5)

3-[2-({5-methyl-2-[4-(trifluoromethyl)phenyl]-1,3-oxazol-4-yl}methoxy)phenoxy]benzoic acid:
TLC: Rf 0.53 (dichloromethane:methanol = 9:1);
NMR (DMSO-d₆) δ 2.23, 5.03, 7.04, 7.11, 7.14, 7.23, 7.27, 7.38, 7.55, 7.85, 8.04, 12.99.

### Example 9 (56)

{2-methyl-3-[4-methyl-2-({(2E)-3-[4-(trifluoromethyl)phenyl]-2-propenyl}oxy)phenoxy]phenyl}acetic acid:
TLC: Rf 0.43 (dichloromethane:methanol = 9:1);
NMR (DMSO-d₆) δ 2.19, 2.29, 3.63, 4.73, 6.48, 6.53-6.55, 6.75, 6.80, 6.90, 6.98-7.04, 7.56, 7.66, 12.33.

### Example 9 (57)

{3-methyl-5-[4-methyl-2-({(2E)-3-[4-(trifluoromethyl)phenyl]-2-propenyl}oxy)phenoxy]phenyl}acetic acid:
TLC: Rf 0.46 (dichloromethane:methanol = 9:1);
NMR (DMSO-d₆) δ 2.18, 2.31, 3.44, 4.71, 6.48-6.54, 6.69-6.72, 6.79, 6.95, 7.05, 7.55, 7.66, 1227.

### Example 9 (58)

3-{2-[(3-phenyl-2-propynyl)oxy]phenoxy}benzoic acid:
TLC: Rf 0.49 (chloroform:methanol = 9:1);
NMR (DMSO-d₆) δ 5.04, 7.05, 7.14, 7.18, 7.23-7.32, 7.32-7.40, 7.43, 7.61, 13.03.

### Example 9 (59)

{3-[4-methyl-2-({3-[4-(trifluoromethyl)phenyl]-2-propynyl}oxy)phenoxy]phenyl}acetic acid:
TLC: Rf 0.40 (dichloromethane:methanol = 19: 1);
NMR (DMSO-d₆) δ 2.33, 3.50, 5.06, 6.68, 6.79, 6.83, 6.90, 6.94, 7.15, 7.20, 7.60, 7.73, 12.31.

### Example 9 (60)

3-[2-({3-[4-(trifluoromethoxy)phenyl]-2-propynyl}oxy)phenoxy]benzoic acid:
TLC: Rf 0.38 (dichloromethane:methanol = 19:I);
NMR (DMSO-d₆) 5 5.05, 7.06, 7.14, 7.18, 7.26, 7.30, 7.32-7.39, 7.43, 7.51, 7.61, 13.03.

### Example 9 (61)

3-(2-{[3-(4-chlorophenyl)-2-propynyl]oxy}phenoxy)benzoic acid:
TLC: Rf 0.42 (dichloromethane:methanol = 19:1);
NMR (DMSO-d₆) δ 5.04, 7.06, 7.14, 7.18, 7.23-7.32, 7.32-7.48, 7.61, 13.03.

### Example 9 (62)

{3-[2-({3-[4-(trifluoromethoxy)phenyl]-2-propynyl}oxy)phenoxy]phenyl}acetic acid:
TLC: Rf0.40 (dichloromethane:methanol = 19:1);
NMR (DMSO-d₆) δ 3.51, 5.06, 6.70, 6.83, 6.93, 6.98-7.08, 7.17-7.27, 7.29-7.42, 7.52, 12.32.

### Example 9 (63)

[3-(2-{[3-(4-chlorophenyl)-2-propynyl]oxy}phenoxy)phenyl] acetic acid:
TLC: Rf 0.40 (dichloromethane:methanol = 19:1),
NMR (DMSO-d₆) δ 3.51, 5.05, 6.70, 6.83, 6.93, 6.99-7.07, 7.16.-7.27, 7.32, 7.37-7.49, 12.32.

### Example 9 (64)

3-(2-{[3-(1,1'-biphenyl-4-yl)-2-propynyl]oxy}phenoxy)benzoic acid:
TLC: Rf 0.53 (chloroform:methanol = 9:1);
NMR (DMSO-d₆) δ 5.07, 7.06, 7.15, 7.19, 7.24-7.33, 7.33-7.51, 7.62, 7.67, 13.04.

### Example 9 (65)

2-chloro-5-[2-({3-[4-(trifluoromethyl)phenyl]-2-propynyl}oxy)phenoxy]benzoic acid:
TLC: Rf0.41 (dichloromethane: methanol = 9:1);
NMR (DMSO-d₆) δ 5.07, 7.04, 7.07, 7.16-7.19, 7.28, 7.36, 7.45, 7.58, 7.72, 13.44.

### Example 9 (66)

4-methyl-3-[2-({3-[4-(trifluoromethyl)phenyl]-2-propynyl}oxy)phenoxy]benzoic acid:
TLC: Rf 0.59 (dichloromethane:methanol = 9:1);
NMR (DMSO-d₆) δ 2.35, 5.09, 7.05-7.09, 7.21-7.27, 7.33-7.39, 7.53-7.59, 7.73, 12.84.

### Example 9 (67)

3-[5-chloro-2-({3-[4-(trifluoromethyl)phenyl]-2-propynyl}oxy)phenoxy]benzoic acid:
TLC: Rf 0.55 (dichloromethane:methanol = 9:1);
NMR (DMSO-d₆) δ 5.11, 7.22, 7.26, 7.30-7.34, 7.35, 7.39, 7.47, 7.59, 7.62-7.67, 7.73, 13.09.

### Example 9 (68)

3-[3-chloro-2-({3-[4-(trifluoromethyl)phenyl]-2-propynyl}oxy)phenoxy]benzoic acid:
TLC: Rf 0.51 (dichloromethane:methanol = 9:1);
NMR (DMSO-d₆) δ 5.05, 7.09, 7.22, 7.30, 7.39, 7.44, 7.50, 7.52, 7.68, 7.71, 13.12.

### Example 9 (69)

(3-{2-[(3-phenyl-2-propynyl)oxy]phenoxy}phenyl)acetic acid:
TLC: Rf 0.49 (chloroform:methanol = 9:1);
NMR(DMSO-d₆) δ 3.51, 5.05, 6.70, 6.83, 6.92, 6.97-7.06, 7.16-7.27, 7.29-7.46, 12.32.

### Example 9 (70)

[3-(2-{[3-(1,1'-biphenyl-4-yl)-2-propynyl]oxy}phenoxy)phenyl]acetic acid:
TLC: Rf 0.51 (chloroform: methanol= 9:1);
NMR (DMSO-d₆) δ 3.51, 5.07, 6.71, 6.84, 6.93, 6.97-7.10, 7.14-7.27, 7.30-7.42, 7.42-7.54, 7.62-7.73, 12.33.

### Example 9 (71)

3-[2-({3-[4'-(trifluoromethyl)-1,1'-biphenyl-4-yl]-2-propynyl}oxy)phenoxy]benzoic acid:
TLC: Rf 0.62 (chloroform:methanol = 9:1);
NMR(CDCl₃) δ 4.94, 6.97-7.11, 7.15-7.31, 7.37, 7.42-7.56, 7.59-7-70, 7.74.

### Example 9 (72)

{3-[2-({3-[4'-(trifluoromethyl)-1,1'-biphenyl-4-yl]-2-propynyl}oxy)phenoxy]phenyl} acetic acid:
TLC: Rf 0.63 (chloroform:methanol = 9:1);
NMR(CDCl₃) δ 3.58, 4.95, 6.82-7.07, 7.10-7.20, 7.20-7.30, 7.39-7.58, 7.59-7.73.

### Example 9 (73)

3-[2-({3-[3'-(trifluoromethyl)-1,1'-biphenyl-4-yl]-2-propynyl}oxy)phenoxy]benzoic acid:
TLC: Rf 0.47 (chloroform:methanol = 9:1);
NMR(CDCl₃) δ 4.95, 6.97-7.11, 7.15-7.31, 7.38, 7.43-7.66, 7.68-7.83.

### Example 9 (74)

3-(2-{[3-(4-fluorophenyl)-2-propynyl]oxy}phenoxy)benzoic acid:
TLC: Rf 0.41 (dichloromethane:methanol = 19:1);
NMR (DMSO-d₆) δ 5.03, 7.06, 7.12, 7.14-7.28, 7.29, 7.35, 7.39-7.48, 7.61, 13.04.

### Example 9 (75)

3-[2-({3-[4-(2-thienyl)phenyl]-2-propynyl}oxy)phenoxy]benzoic acid:
TLC: Rf 0.44 (dichloromethane:methanol = 19:1);
NMR (DMSO-d₆) δ 5.06, 7.07, 7.12-7.22, 7.24-7.33, 7.34-7.49, 7.55-7.70, 13.04.

### Example 9 (76)

3-[2-({3-[4-(3-furyl)phenyl]-2-propynyl}oxy)phenoxy]benzoic acid:
TLC: Rf0.42 (dichloromethane:methanol = 19:1);
NMR (DMSO-d₆) δ 5.05, 6.97, 7.06, 7.14, 7.18, 7.23-7.33, 7.34-7.41, 7.44, 7.57-7.67, 7.75, 8.24, 13.04.

### Example 9 (77)

[3-(2-{[3-(4-fluorophenyl)-2-propynyl]oxy}phenoxy)phenyl]acetic acid:
TLC: Rf 0.42 (dichloromethane:methanol = 19:1);
NMR (DMSO-d₆) δ 3.51, 5.04, 6.70, 6.83, 6.93, 6.97-7.07, 7.15-7.28, 7.33, 7.40-7.52, 12.32.

### Example 9 (78)

{2-chloro-5-[2-({3-[4-(trifluoromethyl)phenyl]-2-propynyl}oxy)phenoxy]phenyl}acetic acid:
TLC: Rf 0.45 (dichloromethane:methanol = 9:1);
NMR (DMSO-d₆) δ 3.63, 5.09, 5.72, 6.99, 7.01-7.11, 7.25, 7.30-7.37, 7.60, 7.73.

### Example 9 (79)

{4-methyl-3-[2-({3-[4-(trifluoromethyl)phenyl]-2-propynyl}oxy)phenoxy]phenyl}acetic acid:
TLC: Rf 0.52 (dichloromethane:methanol = 9:1);
NMR (DMSO-d₆) δ 2.20, 3.40, 5.10, 6.59, 6.83, 6.89, 6.98, 7.14, 7.19, 7.31, 7.62, 7.74.

### Example 9 (80)

3-[2-chloro-6-({3-[4-(trifluoromethyl)phenyl]-2-propynyl}oxy)phenoxy]benzoic acid:
TLC: Rf 0.60 (dichloromethane:methanol = 9:1);
NMR (DMSO-d₆) δ 5.13, 7.16, 7.21, 7.24-7.29, 7.31-7.39, 7.44, 7.57, 7.61, 7.72.

### Example 9 (81)

{3-[2-({3-[3'-(trifluoromethyl)-1,1'-biphenyl-4-yl]-2-propynyl}oxy)phenoxy]phenyl} acetic acid:
TLC: Rf 0.35 (chloroform:methanol = 9:1);
NMR(CDCl₃) δ 3.59, 4.96, 6.81-6.91, 6.91-6.94, 6.94-7.06, 7.09-7.20, 7.20-7.30, 7.42-7.64, 7.73, 7.80.

### Example 9 (82)

{3-[2-({5-methyl-2-[4-(trifluoromethyl)phenyl]-1,3-oxazol-4-yl}methoxy)phenoxy]phenyl}acetic acid:
TLC: Rf 0.58 (dichloromethane:methanol = 9:1);
NMR (DMSO-d₆) δ 2.26, 3.49, 5.04, 6.68, 6.77-6.80, 6.89, 6.96-7.04, 7.16-7.23, 7.35, 7.87, 8.08, 12.30.

### Example 9 (83)

[3-(2-{[3-(4'-fluoro-1,1'-biphenyl-4-yl)-2-propynyl]oxy}phenoxy)phenyl]acetic acid:
TLC: Rf 0.33 (chloroform:methanol = 9:1);
NMR(CDCl₃) δ 3.58, 4.95, 6.83-7.05, 7.06-7.19, 7.20-7.30, 7.38-7.57.

### Example 9 (84)

3-(2-{[3-(4'-fluoro-1,1'-biphenyl-4-yl)-2-propynyl]oxy}phenoxy)benzoic acid:
TLC: Rf 0.43 (chloroform:methanol = 9:1);
NMR(CDCl₃) δ 4.94, 6.96-7.15, 7.15-7.31, 7.31-7.55, 7.57-7.66, 7.75.

### Example 9 (85)

3-[2-({3-[4-(3-thienyl)phenyl]-2-propynyl}oxy)phenoxy]benzoic acid:
TLC: Rf 0.42 (dichloromethane:methanol = 19:1);
NMR (DMSO-d₆) δ 5.05, 7.06, 7.14, 7.19, 7.24-7.33, 7.37, 7.40, 7.44, 7.57, 7.59-7.68, 7.72, 7.94, 13.03.

### Example 9 (86)

3-(2-{[3-(4-methylphenyl)-2-propynyl]oxy}phenoxy)benzoic acid:
TLC: Rf 0.44 (dichloromethane:methanol = 19:1);
NMR (DMSO-d₆) δ 2.29, 5.02, 7.05, 7.10-7.20, 7.22-7.31, 7.34, 7.43, 7.61, 13.05.

### Example 9 (87)

3-[2-({3-[3-(trifluoromethyl)phenyl]-2-propynyl}oxy)phenoxy]benzoic acid:
TLC: Rf 0.48 (dichloromethane:methanol = 19:1);
NMR (DMSO-d₆) δ 5.07, 7.06, 7.14, 7.18, 7.23-7.33, 7.37, 7.43, 7.54-7.64, 7.64-7.71, 7.75, 13.02.

### Example 9 (88)

{3-[5-chloro-2-({3-[4-(trifluoromethyl)phenyl]-2-propynyl}oxy)phenoxy]phenyl}acetic acid:
TLC: Rf 0.50 (dichloromethane:methanol = 9:1);
NMR (DMSO-d₆) δ 3.53, 5.11, 6.78, 6.85-6.90, 6.98, 7.07, 7.23-7.30, 7.36, 7.62, 7.74, 12.33.

### Example 9 (89)

{3-[2-chloro-6-({3-[4-(trifluoromethyl)phenyl]-2-propynyl}oxy)phenoxy]phenyl}acetic acid:
TLC: Rf 0.51 (dichloromethane:methanol = 9:1);
NMR (DMSO-d₆) δ 3.50, 5.12, 6.58, 6.79, 6.90, 7.19, 7.24, 7.28-7.38, 7.59, 7.74, 12.31.

### Example 9 (90)

[3-(2-{[3-(4'-methyl-1,1'-biphenyl-4-yl)-2-propynyl]oxy}phenoxy)phenyl]acetic acid:
TLC: Rf 0.47 (chloroform: methanol = 9:1);
NMR(CDCl₃) δ 2.39, 3.58, 4.95, 6.81-7.07, 7.10-7.20, 7.20-7.31, 7.36-7.55.

### Example 9 (91)

[3-(2- {[3-(3'-methyl-1,1'-biphenyl-4-yl)-2-propynyl]oxy}phenoxy)phenyl]acetic acid:
TLC: Rf 0.40 (chloroform: methanol = 9:1);
NMR(CDCl₃) δ 2.41, 3.58, 4.95, 6.83-7.06, 7.10-7.20, 7.20-7.39, 7.41-7.47, 7.48-7.55.

### Example 9 (92)

[3-(2-{[3-(3'-fluoro-1,1'-biphenyl-4-yl)-2-propynyl]oxy}phenoxy)phenyl]acetic acid:
TLC: Rf 0.22 (chloroform:methanol = 19:1);
NMR(CDCl₃) δ 3.58, 4.95, 6.82-7.09, 7.10-7.20, 7.20-7.30, 7.30-7.42, 7.41-7.53.

### Example 9 (93)

[3-(2-{[3-(4-methylphenyl)-2-propynyl]oxy}phenoxy)phenyl]acetic acid:
TLC: Rf 0.47 (dichloromethane:methanol = 19:1);
NMR (DMSO-d₆) δ 2.29, 3.51, 5.03, 6.70, 6.83, 6.92, 6.97-7.06, 7.12-7.36, 12.32.

### Example 9 (94)

{3-[2-({3-[3-(trifluoromethyl)phenyl]-2-propynyl}oxy)phenoxy]phenyl}acetic acid:
TLC: Rf 0.49 (dichloromethane:methanol = 19:1);
NMR (DMSO-d₆) δ 3.50, 5.08, 6.71, 6.83, 6.92, 6.98-7.09, 7.16-7.27, 7.34, 7.61, 7.67-7.81, 12.31.

### Example 9 (95)

3-methyl-5-(4-methyl-2-{[3-(4-methylphenyl)-2-propynyl]oxy}phenoxy)benzoic acid:
TLC: Rf 0.33 (dichloromethane:methanol = 19:1);
NMR (DMSO-d₆) δ 2.27, 2.29, 2.34, 4.99, 6.85, 6.94-7.03, 7.07, 7.13-7.21, 7.26, 7.41, 12.93.

### Example 9 (96)

3-(2-{[3-(4-chlorophenyl)-2-propynyl]oxy}-4-methylphenoxy)-5-methylbenzoic acid:
TLC: Rf 0.35 (dichloromethane:methanol = 19:1);
NMR (DMSO-d₆) δ 2.27, 2.34, 5.01, 6.85, 6.95-7.03, 7.06, 7.15, 7.33-7.47, 12.93.

### Example 9 (97)

3-(2-{[3-(4-methoxyphenyl)-2-propynyl]oxy}-4-methylphenoxy)-5-methylbenzoic acid
TLC: Rf 0.55 (dichloromethane:methanol = 9:1);
NMR (DMSO-d₆) δ 2.27, 2.34, 3.75, 4.97, 6.84, 6.91, 6.96-7.01, 7.04-7.07, 7.15, 7.32, 7.40, 12.92.

### Example 9 (98)

3-(2-{ [3-(2,2-difluoro-1,3-benzodioxol-5-yl)-2-propynyl]oxy}-4-methylphenoxy)-5-methylbenzoic acid:
TLC: Rf 0.54 (dichloromethane:methanol = 9:1);
NMR (DMSO-d₆) δ 2.28, 2.34, 5.00, 6.85, 6.97-7.01, 7.04, 7.15, 7.24, 7.39-7.42, 7.47, 12.91.

### Example 9 (99)

3-[2-({3-[4-(2-pyridinyl)phenyl]-2-propynyl}oxy)phenoxy]benzoic acid:
TLC: Rf 0.47 (chloroform:methanol = 9:1);
NMR (DMSO-d₆) δ 5.08, 7.07, 7.15, 7.19, 7.24-7.40, 7.41-7.51, 7.62, 7.88, 7.98, 8.09, 8.67, 13.04.

### Example 9 (100)

3-[2-({3-[4-(3-pyridinyl)phenyl]-2-propynyl}oxy)phenoxy]benzoic acid:
TLC: Rf 0.42 (chloroform:methanol = 9:1);
NMR (DMSO-d₆) δ 5.07, 7.07, 7.15, 7.19, 7.24-7.33, 7.37, 7.41-7.53, 7.58-7.65, 7.74, 8.08, 8.58,8.90, 13.04.

### Example 9 (101)

3-methyl-5-[4-methyl-2-({3-[3-(trifluoromethyl)phenyl]-2-propynyl}oxy)phenoxy]benzoic acid:
TLC: Rf 0.44 (dichloromethane: methanol = 19:1);
NMR (DMSO-d₆) δ 2.27, 2.34, 5.04, 6.86, 6.95-7.04, 7.07, 7.17, 7.40, 7.55-7.71, 7.75, 12.92.

### Example 9 (102)

3-methyl-5-[4-methyl-2-({3-[4-(trifluoromethoxy)phenyl]-2-propynyl}oxy)phenaxy]benzoic acid:
TLC: Rf 0.46 (dichloromethane:methanol = 19:1);
NMR (DMSO-d₆) δ 2.27, 2.34, 5.02, 6.86, 6.96-7.03, 7.06, 7,16, 7.36, 7.41, 7.50, 12.93.

### Example 9 (103)

3-(2-{[3-(4-fluorophenyl)-2-propynyl]oxy}-4-methylphenoxy)-5-methylbenzoic acid:
TLC: Rf 0.40 (dichloromethane:methanol = 9:1);
NR4R (DMSO-d₆) δ 2.28, 2.34, 5.00, 6.83-6.87, 6.95-7.01, 7.04-7.06, 7.16, 7.18, 7.22, 7.3 9-7.47, 12.92.

### Example 9 (104)

3-(2-{[3-(4-isopropylphenyl)-2-propynyl]oxy}-4-methylphenoxy)-5-methylbenzoic acid:
TLC: Rf 0.60 (dichloromethane:methanol =9:1);
NMR (DMSO-d₆) δ 1.15, 1.18, 2.27, 2.34, 2.80-2.94, 4.99, 6.85, 6.94-7.01, 7.06, 7.15, 7.20-7.25, 7.27-7.31, 7.39-7.42, 12.93.

### Example 9 (105)

3-methyl-5-[4-methyl-2-({3-[6-(trifluoromethyl)-3-pyridinyl]-2-propynyl}oxy)phenoxy]benzoic acid:
TLC: Rf 0.42 (chloroform:methanol = 9:1);
NMR (DMSO-d₆) δ 2.28, 2.35, 5.09, 6.84-6.90, 7.01, 6.99-7.01, 7.03-7.07, 7.18, 7.34-7.45, 7.91, 8.08, 8.69-8.78, 12.91.

### Example 9 (106)

2-methyl-2-{3-[2-({(2E)-3-[4-(trifluoromethyl)phenyl]-2-propenyl}oxy)phenoxy]phenyl}propanic acid:
TLC: Rf 0.42 (dichloromethane:methanol = 19:1);
NMR (DMSO-d₆) δ 1.39, 4.74, 6.49, 6.69, 6.90, 6.95-7.05, 7.09, 7.14-7.32, 7.53, 7.65, 12.37.

### Example 9 (107)

1-{3-[2-({(2E)-3-[4-(trifluoromethyl)phenyl]-2-propenyl}oxy)phenoxy]phenyl}cyclopropanecarboxylic acid:
TLC: Rf 0.37 (dichloromethane:methanol = 19:1);
NMR (DMSO-d₆) δ 1.03, 1.38, 4.74, 6.50, 6.72, 6.85, 6.94-7.05, 7.09, 7.14-7.28, 7.53, 7.65, 12.31.

### Example 9 (108)

2-methyl-2-{3-[2-({3--[4-(trifluoromethyl)phenyl]-2-propynyl}oxy)phenoxy]phenyl}propanic acid:
TLC: Rf 0.42 (dichloromethane:methanol = 19:1);
NMR (DMSO-d₆) δ 1.40, 5.09, 6.66, 6.91, 6.97-7.09, 7.17-7.28, 7.34, 7.60, 7.74, 12.35.

### Example 9 (109)

1-{3-[2-({3-[4-(trifluoromethyl)phenyl]-2-propynyl}oxy)phenoxy]phenyl}cyclopropanecarboxylic acid:
TLC: Rf 0.47 (dichloromethane:methanol = 19:1);
NMR (DMSO-d₆) δ 1.07, 1.36, 5.09, 6.69, 6.86, 6.94-7.11, 7.15-7.27, 7.34, 7.60, 7.74, 12.30.

### Example 9 (110)

3-(2-{[3-(4-chlorophenyl)-2-propynyl]oxy}phenoxy)-5-methylbenzoic acid:
TLC: Rf 0.57 (dichloromethane:methanol = 9:1);
NMR (DMSO-d₆) δ 2.276, 5.033, 6.990-7.119, 7.247, 7.312-7.438, 12.916.

### Example 9 (111)

3-(2-{[3-(4-chlorophenyl)-2-propynyl]oxy}phenoxy)-2-methylbenzoic acid:
TLC: Rf 0.40 (dichloromethane:methanol = 9:1);
NMR (DMSO-d₆) δ 2.423, 5.048, 6.799, 6.878, 6.985, 7.131-7.195, 7.302, 7.384-7.475, 12.936.

### Example 9 (112)

3-(2-{[3-(4-chlorophenyl)-2-propynyl]oxy}-4-methylphenoxy)benzoic acid:
TLC: Rf 0.47 (dichloromethane:methanol = 9:1);
NMR (DMSO-d₆) δ 2.34, 5.01, 6.86, 7.02, 7.11-7.18, 7.25, 7.35-7.45, 7.58, 13.00.

### Example 9 (113)

3-(4-methyl-2-{[3-(4-methylphenyl)-2-propynyl]oxy}phenoxy)benzoic acid:
TLC: Rf 0.44 (dichloromethane:methanol = 9:1);
NMR (DMSO-d₆) δ 2.286, 2.335, 4.974, 6.848, 7.006, 7.122-7.175, 7.239-7.265, 7.401, 7.575, 12.986.

### Example 9 (114)

3-methyl-5-(2- {[3-(4-methylphenyl)-2-propynyl]oxy}phenoxy)benzoic acid
TLC: Rf 0.45 (dichloromethane:methanol = 9:1);
NMR (DMSO-d₆) δ 2.28, 2.29, 5.02, 6.99, 7.04, 7.10, 7.11, 7.16, 7.21-7.29, 7.33, 7.43, 12.92.

### Example 9 (115)

3-[2-chloro-6-({3-[4-(trifluoromethyl)phenyl]-2-propynyl}oxy)phenoxy]-5-methylbenzoic acid:
TLC: Rf 0.47 (dichloromethane:methanol = 9:1);
NMR (DMSO-d₆) δ 2.29, 5.13, 6.99, 7.26, 7.33-7.38, 7.43, 7.57, 7.73, 12.97.

### Example 9 (116)

2-{3-[2-({(2E)-3-[4-(trifluoromethyl)phenyl]-2-propenyl}oxy)phenoxy]phenyl}butanoic acid:
TLC: Rf 0.35 (dichloromethane:methanol = 19:1);
NMR (DMSO-d₆) δ 0.75, 1.44-1.64, 1.77-1.95, 3.27-3.42, 4.72, 6.39-6.56, 6.74, 6.83, 6.91-7.05, 7.05-7.13, 7.15-7.32, 7.53, 7.65, 12.30.

### Example 9 (117)

2-{3-[2-({(2E)-3-[4-(trifluoromethyl)phenyl]-2-propenyl}oxy)phenoxy]phenyl}pentanoic acid:
TLC: Rf 0.38 (dichloromethane:methanol = 19:1);
NMR (DMSO-d₆) δ 0.75, 1.01-1.26, 1.40-1.58, 1.72-1.91, 3.45, 4.72, 6.42-6.52, 6.74, 6.83, 6.91-7.05, 7.06-7.13, 7.16-7.30, 7.52, 7.64, 12.30.

### Example 9 (118)

2-methyl-3-(2-{[3-(4-methylphenyl)-2-propynyl]oxy}phenoxy)benzoic acid:
TLC: Rf 0.50 (dichloromethane:methanol = 9:1);
NMR (DMSO-d₆) δ 2.289, 2.426, 5.028, 6.806, 6.870, 6.977, 7.128-7.224, 7.289-7.316, 7.466, 12.947.

### Example 9 (119)

2-methyl-3-(4-methyl-2-{[3-(4-methylphenyl)-2-propynyl]oxy}phenoxy)benzoic acid:
TLC: Rf 0.59 (dichloromethane:methanol = 9:1);
NMR (DMSO-d₆) δ 2.290, 2.305, 2.434, 4.988, 6.746, 6.795, 7.096-7.188, 7.262-7.289, 7.430, 12.924.

### Example 9 (120)

3-(2-{[3-(4-chlorophenyl)-2-propynyl]oxy}-4-methylphenoxy)-2-methylbenzoic acid:
TLC: Rf 0.44 (dichloromethane:methanol = 9:1);
NMR (DMSO-d₆) δ 2.305, 2.432, 5.007, 6.745, 6.804, 7.126, 7.383-7.457, 12.922.

### Example 9 (121)

2-{3-[2-({3-[4-(trifluoromethyl)phenyl]-2-propynyl}oxy)phenoxy]phenyl}butanoic acid:
TLC: Rf 0.47 (dichloromethane:methanol = 19:1);
NNM (DMSO-d₆) δ 0.76, 1.49-1.66, 1.76-1.96, 3.30-3.42, 5.08, 6.71, 6.84, 6.95, 6.99-7.09, 7.16-7.29, 7.34, 7.60, 7.73, 12.32.

### Example 9 (122)

2-{3-[2-({3-[4-(trifluoromethyl)phenyl]-2-propynyl}oxy)phenoxy]phenyl}pentanoic acid:
TLC: Rf 0.45 (dichloromethane:methanol = 19:1);
NMR (DMSO-d₆) δ 0.79, 1.04-1.30, 1.44-1.65, 1.74-1.95, 3.44, 5.08, 6.71, 6.85, 6.96, 7.00-7.09, 7.16-7.29, 7.34, 7.60, 7.73, 12.31.

### Example 9 (123)

[3-(2- {[3 -(4-chlorophenyl)-2-propynyl]oxy}phenoxy)-2-methylphenyl]acetic acid:
TLC: Rf 0.64 (dichloromethane: methanol = 9:1);
NMR (DMSO-d₆) δ 2.138, 3.624, 5.065, 6.554, 6.794, 6.918-7.051, 7.131, 7.291, 7.393-7.461, 12.32.

### Example 9 (124)

[3-(4-methyl-2-{[3-(4-methylphenyl)-2-propynyl]oxy}phenoxy)phenyl]acetic acid:
TLC: Rf 0.55 (dichloromethane:methanol = 9: 1);
NMR (DMSO-d₆) δ 2.288, 2.317, 3.478, 4.981, 6.660, 6.764-6.928, 7.128-7.208, 7.277, 12.28.

### Example 9 (125)

[3-(2-{[3-(4-chlorophenyl)-2-propynyl]oxy}phenoxy)-5-methylphenyl]acetic acid:
TLC: Rf 0.45 (dichloromethane:methanol = 9:1);
NMR (DMSO-d₆) δ 2.164, 3.430, 5.036, 6.510, 6.595, 6.275, 6.997, 7.014, 7.164-7.222, 7.303, 7.378-7.450, 12.28.

### Example 9 (126)

[3-(2-{[3-(4-chlorophenyl)-2-propynyl]oxy}-4-methylphenoxy)phenyl]acetic acid:
TLC: Rf 0.37 (dichloromethane:methanol = 9:1);
NMR (DMSO-d₆) δ 2.317, 3.471, 5.001, 6.658, 6.973-6.932, 7.129, 7.181, 7.383-7.454, 12.11.

### Example 9 (127)

[2-methyl-3-(2-{[3-(4-methylphenyl)-2-propynyl]oxy}phenoxy)phenyl]acetic acid:
TLC: Rf 0.58 (dichloromethane:methanol = 9:1);
NMR (DMSO-d₆) δ 2.139, 2.290, 3.624, 5.043, 6.559, 6.787, 6.923-6.980, 7.023, 7.099-7.187, 7.272-7.304, 12.331.

### Example 9 (128)

[3-methyl-5-(2-{[3-(4-methylphenyl)-2-propynyl]oxy}phenoxy)phenyl]acetic acid:
TLC: Rf 0.35 (dichloromethane:methanol = 9:1);
NMR (DMSO-d₆) δ 2.167, 2.286, 3.435, 5.015, 6.511, 6.597, 6.727, 6.993, 7.006, 7.151-7.315, 12.262.

### Example 9 (129)

[3-methyl-5-(4-methyl-2-{[3-(4-methylphenyl)-2-propynyl]oxy}phenoxy)phenyl]acetic acid:
TLC: Rf0.49 (dichloromethane:methanol = 9:1);
NMR (DMSO-d₆) δ 2.156, 2.288, 2.316, 3.418, 4.981, 6.478, 6.560, 6.695, 6.804, 6.896, 7.121, 7.153-7.184, 7.256-7.289, 12.24,

### Example 9 (130)

2,5-dimethyl-3-[2-({3-[4-(trifluoromethyl)phenyl]-2-propynyl}oxy)phenoxy]benzoic acid:
TLC: Rf 0.53 (dichloromethane:methanol = 9:1);
NNIR (DMSO-d₆) δ 2.135, 2.368, 5.102, 6.647, 6.855, 6.991, 7.164, 7.300-7.323, 7.598, 7.734, 12.906.

### Example 9 (131)

{3-methyl-5-[4-methyl-2-({3-[6-(trifluoromethyl)-3-pyridinyl]-2-propynyl} oxy)phenoxy]phenyl} acetic acid:
TLC: Rf 0.30 (chloroform:methanol = 9:1);
NMR (DMSO-d₆) δ 2.16, 2.33, 3.43, 5.09, 6.51, 6.57, 6.70, 6.83, 6.92, 7.15, 7.91, 8.11, 8.77, 12.25.

### Example 10

3-[2-({(2E)-3-[4-(trifluoromethyl)phenyl]-2-propenyl}amino)phenoxy]benzoic acid:

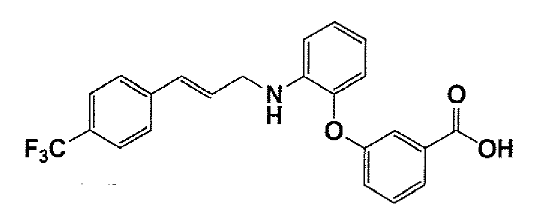

(2E)-3-[4-(Trifluoromethyl)phenyl]-2-propen-1-ol (242 mg), methyl 3-(2-aminophenoxy)benzoate (243 mg), triphenyl phosphine (11 mg) and molecular sieves 4Å (200 mg) were suspended in benzene (5 mL) under an argon atmosphere. Titanium (IV) isopropoxide (150 µL) and palladium acetate (6 mg) were added to the suspension and heated to reflux for 3 hours. The reaction mixture was cooled down to room temperature, 1N hydrochloric acid was added thereto and the mixture was extracted with ethyl acetate. 1N Sodium hydroxide was added to the aqueous layer to make alkaline and the mixture was extracted with ethyl acetate again. The organic layers were combined, successively washed with water and saturated saline solution, dried over anhydrous sodium sulfate and concentrated. The resulting residue was purified by a silica gel column chromatography (hexane : ethyl acetate = 20:1) to give methyl ester of the title compound (methyl 3-[2-({(2E)-3-[4-(trifluoromethyl)phenyl]-2-propenyl}amino)phenoxy]benzoate). The same operation as Example 4 was carried out using the methyl ester of the title compound instead of the compound produced in Example 3 to give the compound of the present invention (232 mg) having the following properties.
TLC: Rf 0.35 (dichloromethane:methanol = 19:1);
NMR (DMSO-d₆) δ 3.95, 5.68, 6.41-6.55, 6.56-6.65, 6.75, 6.89, 6.99-7.09, 7.19-7.30, 7.38, 7.47, 7.55, 7.59-7.67, 13.06.

### Example 11

{3-[2-({(2E)-3-[4-(trifluoromethyl)phenyl]-2-propenyl}amino)phenoxy]phenyl}acetic acid:

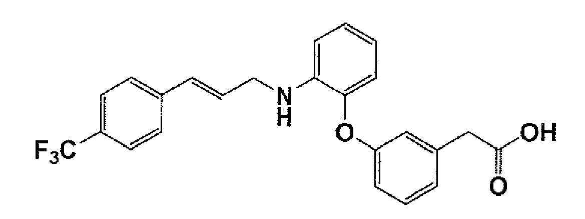

The same operation as Example 10 was carried out using methyl [3-(2-aminophenoxy)phenyl]acetate instead of methyl 3-(2-aminophenoxy)benzoate to give the compound of the present invention having the following properties.
TLC: Rf 0.52 (dichloromethane: methanol = 9:1);
NMR (DMSO-d₆) δ 3.54, 3.95, 5.55, 6.43-6.66, 6.73, 6.77-6.83, 6.86-6.91, 6.93-7.01, 7.27, 7.58, 7.64, 12.32.

### Example 12

3-[2-({3-[4-(trifluoromethyl)phenyl]-2-propynyl}amiuo)phenoxy]benzoic acid:

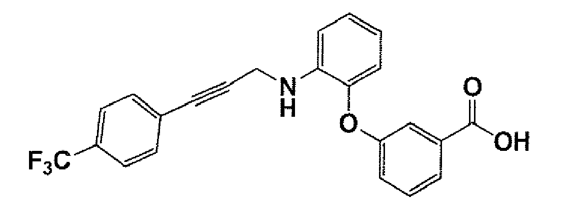

1-(3-Bromo-1-propyn-1-yl)-4-(trifluoromethyl)benzene (111 mg), methyl 3-(2-aminophenoxy)benzoate (103 mg) and cesium carbonate (179 mg) were suspended in dimethylformamide (2 mL) and stirred at 80°C for 1 hour. After the reaction mixture was cooled down to room temperature, water was added thereto followed by extracting with ethyl acetate. Hexane was added to the organic layer and the mixture was successively washed with water and saturated saline solution, dried over anhydrous sodium sulfate and concentrated. The resulting residue was purified by a silica gel thin layer chromatography (hexane : ethyl acetate = 4:1) to give methyl ester of the title compound (methyl 3-[2-({3-[4-(trifluoromethyl)phenyl]-2-propynyl}-amino)phenoxy]benzoate. The same operation in Example 4 was carried out using the methyl ester of the title compound instead of the compound produced in Example 3 to give the compound of the present invention (19 mg) having the following properties.
TLC: Rf 0.30 (dichloromethane:methanol = 19:1);
NMR (DMSO-d₆) δ 4.20, 5.84, 6.69, 6.90, 6.97, 7.13, 7.17-7.26, 7.33-7.39, 7.45, 7.54, 7.62, 7.69, 13.03.

### Example 13

3-[2-({(2E)-3-[4-(trifluoromethyl)phenyl]-2-propenoyl}amino)phenoxy]benzoic acid:

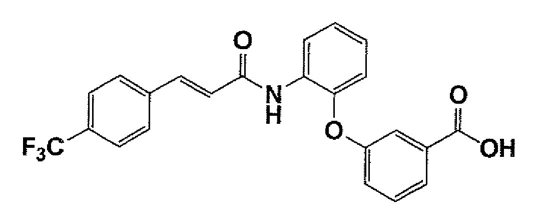

Triethylamine (0.25 mL) was added to a solution of (2E)-3-[4-(trifluoromethyl)phenyl]-2-propenoic acid (324 mg) and methyl 3-(2-aminophenoxy)benzoate (364 mg) in dimethylformamide (3 mL). 1-Ethyl-3-[3-(dimethylamino)propyl]carbodiimide hydrochloride (345 mg) and 1-hydroxybenzotriazole (243 mg) were added to the reaction mixture under cooling with ice. The reaction mixture was stirred at room temperature for 5 hours, water was added thereto and the mixture was extracted with a mixed solvent (ethyl acetate : n-hexane = 1:1). The resulting organic layer was successively washed with 2N hydrochloric acid, water, a saturated aqueous solution of sodium hydrogen carbonate and a saturate saline solution, dried over anhydrous magnesium sulfate and concentrated. The resulting residue was purified by a silica gel column chromatography (n-hexane : ethyl acetate = 9:1) to give methyl ester of the title compound (methyl 3-[2-({(2E)-3-[4-(trifluoromethyl)phenyl]-2-propenoyl}amino)phenoxy]benzoate. The same operation in Example 4 was carried out using the methyl ester of the title compound instead of the compound produced in Example 3 to give the compound of the present invention (45 mg) having the following properties.
TLC: Rf 0.50 (dichloromethane:methanol = 9:1);
NMR (DMSO-d₆) δ 6.99, 7.15, 7.22, 7.23, 7.30, 7.48, 7.52, 7.61, 7.68-7.74, 7.79, 8.25, 9.81, 13.13.

### Example 14

{3-[2-({(2E)-3-[4-(trifluoromethyl)phenyl]-2-propenoyl}amino)phenoxy]phenyl}acetic acid:

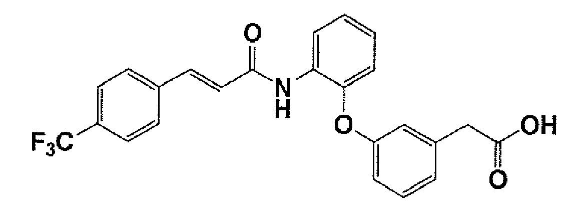

The same operation as Example 13 was carried out using methyl [3-(2-aminophenoxy)phenyl]acetate instead of methyl 3-(2-aminophenoxy)benzoate to give the compound of the present invention having the following properties.
TLC: Rf 0.55 (dichloromethane:methanol = 9:1);
NMR (DMSO-d₆) δ 3.57, 6.86, 6.91, 6.99, 7.02-7.18, 7.30, 7.33, 7.62, 7.78, 7.82, 8.25, 9.78, 12.34.

### Example 15

3-[2-({3-[4-(trifluoromethyl)phenyl]-2-propynoyl}amino)phenoxy]benzoic acid:

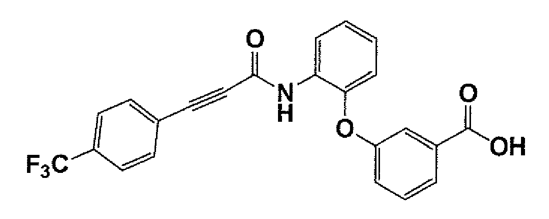

Oxalyl chloride (44 µL) and dimethylformamide (one drop) were dropped into a solution of 3-[4-(trifluoromethyl)phenyl]-2-propynoic acid (86 mg) in tetrahydrofuran (1 mL) under cooling with ice and stirred at room temperature for 2 hours. The reaction mixture was concentrated and the resulting residue was dissolved in methylene chloride (0.4 mL) (solution 1). The solution 1 was dropped into a solution of methyl 3-(2-aminophenxoy)benzoate (89 mg) and triethylamine (0.1 mL) in methylene chloride (0.4 mL) under cooling with ice. The reaction mixture was stirred at room temperature for 3 hours and, under cooling with ice, water was added thereto to stop the reaction. The reaction mixture was extracted with ethyl acetate. The organic layer was successively washed with 2N hydrochloric acid, water and a saturated aqueous solution of sodium hydrogen carbonate, dried over anhydrous magnesium sulfate and concentrated. The resulting residue was purified by a silica gel column chromatography (n-hexane : ethyl acetate = 9:1) to give methyl 3-[2-({(2Z)-3-chloro-3-[4-(trifluoromethyl)phenyl]-2-propenoyl}amino)phenoxy]benzoate. The same operation in Example 4 was carried out using the above-prepared methyl ester instead of the compound produced in Example 3 to give the compound of the present invention (65 mg) having the following properties.
TLC: Rf 0.43 (dichloromethane:methanol = 9:1);
NMR (DMSO-d₆) δ 6.97-7.02, 7.19-7.25, 7.28, 7.45-7.47, 7.51, 7.70, 7.78-7.86, 10.61, 13.10.

### Biological Examples

It was confirmed that compounds of the present invention represented by formula (I) have PPAR agonistic activities by the following experiments.

### Measurement of PPAR agonistic activities:

### (1) Preparation of materials in luciferase assay using human PPAR

The measurement of present invention is the method which has advancement of the measurement accuracy and improvement of the measurement sensitivity in order to evaluate the compounds of the present invention as follows.

That is, as a luciferase gene expression vector under the control of thymidine kinase (TK) promoter, luciferase structural gene was excised from PicaGene Basic Vector 2 (trade name, Toyo Ink Inc., catalogue No. 309-04821), to prepare luciferase gene expression vector pTK-Luc. under the control of TK promoter (-105/+51) as a minimum essential promoter activity from pTKβ having TK promoter (Chrontech Inc., catalogue No. 6179-1). In the upper stream of TK promoter, four times repeated UAS sequence was inserted, which is the response sequence of Gal4 protein, a basic transcription factor in yeast, to construct 4 X UAS-TK-Luc. as reporter gene. The following is the enhancer sequence used (SEQ ID NO:1).
SEQ ID NO:1: Enhancer sequence repeating Gal4 protein response sequence
5'-T(CGACGGAGTACTGTCCTCCG)×4 AGCT-3'

A vector was prepared as described hereafter which expresses chimeric receptor protein wherein in carboxyl terminus of yeast Gal4 protein DNA binding domain was fused to ligand binding domain of human PPAR α, γ or δ. That is to say, PicaGene Basic Vector 2 (trade name, Toyo Ink Inc., catalogue No. 309-04821) was used as a basic expression vector, the structural gene was exchanged for that of chimeric receptor protein, while promoter and enhancer domains were kept as they were.

DNA encoding ligand binding domain of human PPAR α, γ or δ fused to DNA encoding Gal4 protein DNA binding domain, the downstream of DNA encoding the 1st to 147th amino acid sequence for fitting their frames and inserted to the downstream of promotor/enhancer domains in PicaGene Basic Vector 2. Here, DNA sequence was aligned as follows, the amino terminus of human PPAR α, γ or δ ligand binding domain was sequenced nuclear translocation signal originated from SV-40 T-antigen, Ala Pro Lys Lys Lys Arg Lys Val Gly (SEQ ID NO:2), to make an expressed chimeric protein localizing intranuclearly. On the other hand, the carboxyl terminus of them was sequenced influenza hemagglutinin epitope, Tyr Pro Tyr Asp Val Pro Asp Tyr Ala (SEQ ID NO:3) and stop codon for translation in this order, to detect an expressed fused protein tagged epitope sequence.

According to the comparison of human PPAR structures described in the literatures by R. Mukherjee *et al*. (See *J*. *Steroid Biochem*. *Molec. Biol*., 51, 157 (1994)), M. E. *Green et al*., (See *Gene Expression.,* 4, 281 (1995)), A. Elbrecht *et al*. (See *Biochem Biophys. Res. Commun*., 224, 431 (1996)) or A. Schmidt *et al*. (See Mol. Endocrinology., 6, 1634 (1992)), the portion of structural gene used as ligand binding domain of human PPAR α, γ or δ was DNA encoding the following peptide:
human PPAR α ligand binding domain; Ser¹⁶⁷-Tyr⁴⁶⁸
human PPAR γ ligand binding domain: Ser¹⁷⁶-Tyr⁴⁷⁸
human PPAR δ ligand binding domain: Ser¹³⁹-Tyr⁴⁴¹
(each human PPAR γ1 ligand binding domain and human PPAR γ2 ligand binding domain is Ser²⁰⁴-Tyr⁵⁰⁶ which is identical sequence each other). In order to measure basal level of transcription, an expression vector containing DNA binding domain of Gal4 protein lacking in PPAR ligand binding domain, which is exclusively encoding the 1st to 147th amino acid sequence in Gal4 protein was also prepared.

### (2) Luciferase assay using human PPAR α, γ or δ

CV-1 cells used as host cells were cultured by a conventional technique. That is to say, Dulbecco's modified Eagle medium (DMEM) supplemented 10% bovine fetal serum (GIBCO BRL Inc., catalogue No. 26140-061) and 50 U/ ml of penicillin G and 50 µg/ ml of streptomycin sulfate were used to culture CV-1 cells under the atmosphere of 5% carbon dioxide gas at 37°C.

In case of the transfection for introducing DNA, both reporter gene and Gal4-PPAR expression vector, into host cells, 2×10⁶ cells were seeded in a 10 cm dish, and once washed with the medium without serum, followed by addition of the medium (10 ml) thereto. Reporter gene (10 µg), Gal4-PPAR expression vector (0.5 µg) and 50 µl of LipofectAMINE (GIBRO BRL Inc., catalogue No. 18324-012) were well mixed and added to the culture dishes. They were cultured at 37°C for 5 to 6 hours, and thereto was added 10 ml of medium containing 20% of dialyzed bovine fetal serum (GIBRO BRL Inc., catalogue No. 26300-061), and then cultured at 37°C overnight. The cells were dispersed by trypsin treatment, and they were again seeded in 96-well plates in a density of 8000 cells/100 µl of DMEM-10% dialyzed serum/well. Several hours after the cultivation, when cells were attached to the plastic ware, then 100 µl of DMEM-10% dialyzed serum containing the compounds of the present invention, whose concentration is twice as high as the final concentration of them, was added thereto. The culture was settled at 37°C for 42 hours and the cells were dissolved to measure luciferase activity according to manufacturer's instruction.

Carbacyclin activates PPAR δ, and when the data were expressed as relative values (Fold Increase)where the transcription activation degree at a final concentration of 30 µM was defined as 1. The PPAR δ transcription activation degree of the compound produced in Example 9 (47) are shown in Table 1.

**Table 1**

| Final concentration (µM) | Transcription activation degree |
|---|---|
| 0.1 | 0.73 |
| 0.3 | 1.09 |
| 1.0 | 1.02 |

As a result, the compound of the present invention shows excellent agoistic activity against PPAR δ.

### Biological Example:

Lowering action for cholesterol in blood and lipid in blood:

Male mice of seven weeks age (C57BL/6NCrj) was loaded with a high-cholesterol feed (CRF-1 Solid Feed in which 5.5% of peanut oil, 1.5% of cholesterol and 0.5% of cholic acid were mixed; Oriental Bio Service) for six days, then body weight of the rats was measured under fasting and the following various parameter concentrations were measured. Measured items were LDL, HDL, TG value, NEFA and TC value. Depending upon the TC concentrations, each five mice were assigned to one group and the grouping was carried out whereby no imbalance was resulted in mean values of other parameters. From the next day, a compound suspended in a medium (a 0.5% methyl cellulose solution) was compulsorily administered *per os* once daily for continuous six days and, at the same time, loading with the high cholesterol feed was continued as well. On the next day of the final administration (i.e., on the seventh day from the start of the administration), lipid levels in blood (TG, HDL, LDL, NEFA and TC values) were measured.

When the data were expressed as relative values where the value for the group to which a medium was administered was defined as 100%, a raising action for HDL and a lowering action for LDL of the compound produced in Example 9 (47) were as shown in Table 2.

**Table 2**

| Dose (mg/kg) | HDL-raising action | LDL-lowering action |
|---|---|---|
| 3 | 112.0 | 89.7 |
| 10 | 110.2 | 85.3 |
| 30 | 130.7 | 84.4 |

As a result, the compound of the present invention raised HDL and lowered LDL and, accordingly, it is useful as a treating agent for hyperlipemia.

### Preparation Example 1:

The following components were admixed in a conventional method, punched out to give 10000 tablets each containing 50 mg of active ingredient.

| | |
|---|---|
| {3-[2-({[bis(4-methylphenyl)methylene]ammo}oxy)ethoxy]phenyl}acetic acid | 500 g |
| carboxymethylcellulosecalcium (distintegrant) | 20 g |
| magnesium stearate (lubricant) | 10 g |
| microcrystalline cellulose | 470 g |

### Preparation Example 2:

After mixing the following components by a conventional method, the resulting solution was sterilized by a conventional method, 5 ml portions thereof were filled in ampuls, respectively, and freeze-dried by a conventional method to obtain 10000 ampuls of injection containing each 20 mg of the active ingredient.

| | |
|---|---|
| {3-[2-({[bis(4-methylphenyl)methylene]amino}oxy)ethoxy]phenyl} acetic acid | 200 g |
| mannitol | 2000 g |
| distilled water | 50 L |

### Industrial Applicability

The compound of the present invention represented by formula (I), a salt thereof, a solvate thereof or a prodrug thereof has a PPAR δ agonistic action and has, for example, a raising action for HDL cholesterol, an increasing action for LDL clearance, promoting actions for carrying-out and for reverse transfer of lipid (particularly, cholesterol) and an inhibitory action for biosynthesis of cholesterol so that it is useful as a preventive and/or treating agent for, for example, diseases where sugar and lipid metabolisms are abnormal [such as diabetes, hyperlipemia (such as hypercholesterolemia, hypo-HDL-emia, hyper-LDL-emia and hyper-TG-emia), arteriosclerosis, cardiovascular diseases, obesity and metabolic syndrome], hypertension, circulatory diseases and skin inflammation diseases.

## Claims

1. A compound represented by formula (I). 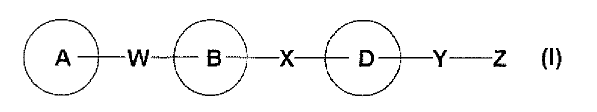 wherein ring A, ring B and ring D each independently represents a cyclic group which may have a substituent(s); W represents a spacer having I to 8 atom(s) in its main chain; X represents a spacer having 1 to 2 atom(s) in its main chain; Y represents a binding bond or a spacer having 1 to 8 atom(s) in its main chain; and Z represents an acidic group, or a salt thereof, a solvate thereof or a prodrug thereof

2. The compound according to claim 1, which is represented by formula (IC): 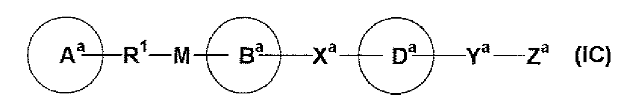 wherein ring A^{a}, ring B^{a} and ring D^{a} each independently represents a C5-10 monocyclic or bicyclic carbon ring which may have a substituent(s), or a 5- to 10-membered monocyclic or bicyclic heterocyclic ring containing 1 to 3 hetero atoms selected from an oxygen atom, a nitrogen atom and/or a sulfur atom; X^{a} represents -O-, -S-, -CO- or -CONR²-, in which R² represents a hydrogen atom, a hydrocarbon group which may have a substituent(s) or a cyclic group which may have a substituent(s); Y^{a} is a binding bond or methylene which may have a substituent(s); Z^{a} represents carboxyl which may be esterified; R¹ represents C1-6 alkylene, C2-6 alkenylene or C2-6 alkynylene; and M represents a spacer having 1 or 2 atoms in its main chain selected from an oxygen atom, carbonyl and a nitrogen atom which may have a substituent(s), or a salt thereof, a solvate thereof or a prodrug thereof.

3. The compound according to claim 1, wherein ring A is a benzene, a pyridine, oxazole or thiazole ring which may have a substituent(s); ring B is a benzene or pyridine ring which may have a substituent(s); ring D is a benzene or pyridine ring which may have a substituent(s); W is -R^{1a}-M^{a}-, in which R^{1a} is propylene, propenylene or prapynylene, and M^{a} is -O-, -NH-, -NHCO- or -CONH-; X represents -O- or -CONR²-, in which R² represents a hydrogen atom, a hydrocarbon group which may have a substituent(s) or a cyclic group which may have a substituent(s); Y is a binding bond or methylene; and Z is carboxyl which may be esterified, or a salt thereof, a solvate thereof or a prodrug thereof

4. The compound according to claim 2, which is represented by formula (ID): 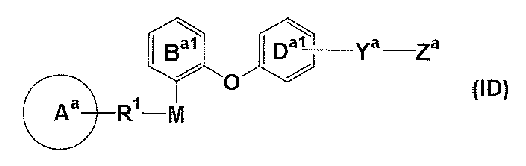 wherein ring B^{a1} and ring D^{a1} each independently represents a benzene ring which may have a substituent(s); and other symbols have the same meanings as described in claim 2, or a salt thereof, a solvate thereof or a prodrug thereof

5. The compound according to claim 2, which is represented by formula (IE): 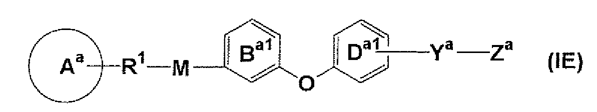 wherein all symbols have the same meanings as described in claim 2, or a salt thereof, a solvate thereof or a prodrug thereof.

6. The compound according to claim 2, which is represented by formula (IF): 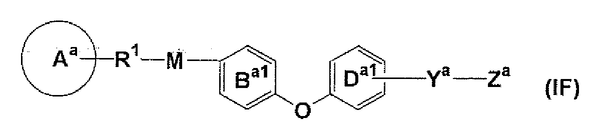 wherein all symbols have the same meanings as described in claim 2, or a salt thereof, a solvate thereof or a prodrug thereof

7. The compound according to claim 1, which is selected from the group consisting of:
(1) {4-methoxy-3-[3-({(2E)-3-[4-(trifluoromethyl)phenyl]-2-propen-1-yl}oxy)phenoxy]phenyl}acetic acid,
(2) {2-chloro-4-methyl-3-[3-({(2E)-3-[4-(trifluoromethyl)phenyl]-2-propen-1-yl}oxy)phenoxy]phenyl}acetic acid,
(3) {2-methyl-3-[2-({(2E)-3-[4-(trifluoromethyl)phenyl]-2-propenyl}oxy)phenoxy]phenyl}acetic acid,
(4) 2-chloro-4-methyl-3-[3-({(2E)-3-[4-(trifluoromethyl)phenyl]-2-propenyl}oxy)phenoxy]benzoic acid,
(5) 3 -methyl-5-[2-({(2E)-3 -[4-(trifluoromethyl)phenyl]-2-propenyl}oxy)phenoxy]benzoic acid,
(6) {3-methyl-5-[2-({(2E)-3-[4-(trifluoromethyl)phenyl]-2-propenyl}oxy)phenoxy]phenyl}acetic acid,
(7) {3-[4-methyl-2-({(2E)-3-[4-(trifluoromethyl)phenyl]-2-propenyl} oxy)phenoxy]phenyl}acetic acid,
(8) {3-[2-({3-[4-(trifluoromethyl)phenyl]-2-propynyl}oxy)phenoxy]phenyl}acetic acid,
(9) 3-methyl-5-[4-methyl-2-({3-[4-(trifluoromethyl)phenyl]-2-propynyl}oxy)phenoxy]benzoic acid,
(10) {3-methyl-5-[2-({3-[4-(trifluoromethyl)phenyl]-2-propynyl}oxy)phenoxy]phenyl}acetic acid,
(11) {3-methyl-5-[4-methyl-2-({3-[4-(trifluoromethyl)phenyl]-2-propynyl}oxy)phenoxy]phenyl}acetic acid,
(12) {3-methyl-5-[4-methyl-2-({(2E)-3-[4-(trifluoromethyl)phenyl]-2-propenyl }oxy)phenoxy]phenyl}acetic acid,
(13) [3-(2-{[3-(4-chlorophenyl)-2-propynyl]oxy}phenoxy)phenyl]acetic acid,
(14) {2-chloro-5-[2-({3-[4-(trifluoromethyl)phenyl]-2-propynyl}oxy)phenoxy]phenyl}acetic acid,
(15) {3-[2-chloro-6-({3-[4-(trifluoromethyl)phenyl]-2-propynyl}oxy)phenoxy]phenyl}acetic acid,
(16) 3-methyl-5-[4-methyl-2-({3-[6-(trifluoromethyl)-3-pyridinyl]-2-propynyl}oxy)phenoxy]benzoic acid,
(17) {3-[2-({(2E)-3-[4-(trifluoromethyl)phenyl]-2-propenyl}amino)phenoxy]phenyl}acetic acid,
(18) 3-(2-{[3-(4-chlorophenyl)-2-propynyl]oxy}phenoxy)-5-methylbenzoic acid,
(19) [3-(4-methyl-2-{[3-(4-methylphenyl)-2-propynyl]oxy}phenoxy)phenyl]acetic acid,
(20) [3-(2-{[3-(4-chlorophenyl)-2-propynyl]oxy}phenoxy)-5-methylphenyl]acetic acid,
(21) [3-methyl-5-(4-methyl-2-{[3-(4-methylphenyl)-2-propynyl]oxy}phenoxy)phenyl]acetic acid, and
(22) {3-methyl-5-[4-methyl-2-({3-[6-(trirluoromethyl)-3-pyridinyl]-2-propynyl}oxy)phenoxy]phenyl} acetic acid,
or a salt thereof a solvate thereof or a prodrug thereof

8. A pharmaceutical composition comprising the compound according to claim 1, or a salt thereof, a solvate thereof or a prodrug thereof.

9. The pharmaceutical composition according to claim 8, which is an agent for accelerating evacuation of lipid, an agent for reverse transport of lipid, an agent for inhibiting foam of macrophage, an agent for increasing HDL, an agent for decreasing LDL or an inhibitor of cholesterol biosynthesis.

10. The pharmaceutical composition according to claim 8, which is an agent for preventing and/or treating PPAR-mediated diseases.

11. The pharmaceutical composition according to claim 10, wherein PPAR is PPAR δ.

12. The pharmaceutical composition according to claim 11, wherein PPAR δ-mediated disease is hyperlipidemia or adiposity,

13. A medicament comprising the compound represented by formula (I) according to claim 1, a salt thereof, a solvate thereof or a prodrug thereof and one kind or more kinds selected from a MTP inhibitor, a HMG-CoA reductase inhibitor, a squalene synthase inhibitor, a fibrate drug, an ACAT inhibitor, a 5-lipoxygenase inhibitor, a cholesterol absorption inhibitor, a bile acid absorption inhibitor, an ideal Na⁺/bile acid transporter inhibitor, LDL receptor activator, LDL receptor expression enhancer, a pancreatic lipase inhibitor, a probucol formulation, a nicotine acid formulation and a cholesterol ester transporter protein inhibitor.

14. A method for accelerating evacuation of lipid in a mammal, which comprises administering to a mammal an effective amount of the compound represented by formula (I) according to claim 1, a salt thereof, a solvate thereof or a prodrug thereof

15. Use of the compound represented by formula (I) according to claim, a salt thereof, a solvate thereof or a prodrug thereof for the manufacture of a medicament for accelerating evacuation of lipid.

16. A method for preventing and/or treating PPAR δ-mediated diseases in a mammal, which comprises administering to a mammal an effective amount of the compound represented by formula (I) according to claim 1, a salt thereof, a solvate thereof or a prodrug thereof

17. Use of the compound represented by formula (I) according to claim, a salt thereof, a solvate thereof or a prodrug thereof for the manufacture of a medicament for preventing and/or treating PPAR δ-mediated diseases.
